# NEW EUROPEAN PATENT SPECIFICATION

(11) **EP 3 056 515 B2**
(45) Date of publication and mention of the opposition decision: **20.08.2025**
(45) Mention of the grant of the patent: 17.04.2019
(21) Application number: 16160476.4
(22) Date of filing: 15.01.2009
(51) Int. Cl.: A61K 39/00, C07K 16/28

(54) **METHODS FOR MANIPULATING PHAGOCYTOSIS MEDIATED BY CD47**
VERFAHREN ZUR BEHANDLUNG VON DURCH CD47 VERMITTELTER PHAGOZYTOSE
PROCÉDÉS PERMETTANT DE MANIPULER LA PHAGOCYTOSE MÉDIÉE PAR CD47

(30) Priority: 15.01.2008 US 11324 P; 22.08.2008 US 189786 P
(43) Date of publication of application: 17.08.2016
(62) Divisional of application: 09701993.9
(73) Proprietor: The Board of Trustees of the Leland Stanford Junior University, Stanford, CA 94305-2038 (US)
(72) Inventor: JAISWAL, Siddhartha, Boston MA 02114 (US); WEISSMAN, Irving L., Stanford, CA 97305 (US); JAMIESON, Catriona, Helen M., La Jolla, CA 92093-0820 (US); MAJETI, Ravindra, Stanford, CA 94304 (US)
(74) Representative: Carpmaels & Ransford LLP

(56) References cited:
- EP-A1- 1 693 385
- US-A1- 2007 111 238
- PARTHA MANNA ET AL: "CD47 mediates killing of breast tumor cells via Gi-dependent inhibition of protein kinase A.", CANCER RESEARCH, vol. 64, no. 3, 1 February 2004 (2004-02-01), pages 1026 - 1036, XP055030076, ISSN: 0008-5472
- Y. LIU ET AL: "Signal Regulatory Protein (SIRPalpha ), a Cellular Ligand for CD47, Regulates Neutrophil Transmigration", JOURNAL OF BIOLOGICAL CHEMISTRY, vol. 277, no. 12, 15 March 2002 (2002-03-15), pages 10028 - 10036, XP055030073, ISSN: 0021-9258, DOI: 10.1074/jbc.M109720200
- RAVINDRA MAJETI1 ET AL: "CD47 is an adverse prognostic factor and therapeutic antibody target on human acute myeloid leukemia stem cells", DEVELOPMENTAL CELL, CELL PRESS, CAMBRIDGE, MA, US, vol. 138, no. 2, 23 July 2009 (2009-07-23), pages 286 - 299, XP002632714, ISSN: 1097-4172, [retrieved on 20090723], DOI: 10.1016/J.CELL.2009.05.045
- S. B. WILLINGHAM ET AL: "The CD47-signal regulatory protein alpha (SIRPa) interaction is a therapeutic target for human solid tumors", PROCEEDINGS OF THE NATIONAL ACADEMY OF SCIENCES, vol. 109, no. 17, 26 March 2012 (2012-03-26), pages 6662 - 6667, XP055029983, ISSN: 0027-8424, DOI: 10.1073/pnas.1121623109
- MAJETI RAVINDRA ET AL: "CD47 Is An Independent Prognostic Factor and Therapeutic Antibody Target on Human Acute Myeloid Leukemia Stem Cells", BLOOD, AMERICAN SOCIETY OF HEMATOLOGY, US, vol. 112, no. 11, 1 November 2008 (2008-11-01), pages 284, XP009160228, ISSN: 0006-4971
- PETTERSEN R F ET AL: "CD47 SIGNALS T CELL DEATH", THE JOURNAL OF IMMUNOLOGY, THE AMERICAN ASSOCIATION OF IMMUNOLOGISTS, US, vol. 162, 1 January 1999 (1999-01-01), pages 7031 - 7040, XP002940659, ISSN: 0022-1767
- X. W. ZHAO ET AL: "Is targeting of CD47-SIRP enough for treating hematopoietic malignancy?", BLOOD, vol. 119, no. 18, 3 May 2012 (2012-05-03), pages 4333 - 4334, XP055116565, ISSN: 0006-4971, DOI: 10.1182/blood-2011-11-391367
- P. P. MANNA ET AL: "The Mechanism of CD47-Dependent Killing of T Cells: Heterotrimeric Gi-Dependent Inhibition of Protein Kinase A", THE JOURNAL OF IMMUNOLOGY, vol. 170, no. 7, 1 April 2003 (2003-04-01), pages 3544 - 3553, XP055116597, ISSN: 0022-1767, DOI: 10.4049/jimmunol.170.7.3544
- M. P. CHAO ET AL: "Therapeutic Antibody Targeting of CD47 Eliminates Human Acute Lymphoblastic Leukemia", CANCER RESEARCH, vol. 71, no. 4, 15 February 2011 (2011-02-15), pages 1374 - 1384, XP055029988, ISSN: 0008-5472, DOI: 10.1158/0008-5472.CAN-10-2238
- JAISWAL S ET AL: "Macrophages as mediators of tumor immunosurveillance", TRENDS IN IMMUNOLOGY, ELSEVIER LTD. * TRENDS JOURNALS, GB, vol. 31, no. 6, 1 June 2010 (2010-06-01), pages 212 - 219, XP027079216, ISSN: 1471-4906, [retrieved on 20100601], DOI: 10.1016/J.IT.2010.04.001
- X. W. ZHAO ET AL: "CD47-signal regulatory protein- (SIRP ) interactions form a barrier for antibody-mediated tumor cell destruction", PROCEEDINGS OF THE NATIONAL ACADEMY OF SCIENCES, vol. 108, no. 45, 8 November 2011 (2011-11-08), pages 18342 - 18347, XP055055636, ISSN: 0027-8424, DOI: 10.1073/pnas.1106550108

### SEQUENCE LISTING

<110> The Board of Trustees of the Leland Stanford Junior University
<120> Methods for manipulating phagocytosis mediated by CD47
<130> P067985EP
<150> EP 09701993.9
   <151> 2009-01-15
<150> US 61/011,324
   <151> 2008-01-15
<150> US 61/189,786
   <151> 2008-08-22
<150> PCT/US2009/000319
   <151> 2009-01-15
<160> 18
<170> FastSEQ for Windows Version 4.0
<210> 1
   <211> 142
   <212> PRT
   <213> Homo sapiens
<400> 1
   Met Trp Pro Leu Val Ala Ala Leu Leu Leu Gly Ser Ala Cys Cys Gly
    1               5                  10                  15
   Ser Ala Gln Leu Leu Phe Asn Lys Thr Lys Ser Val Glu Phe Thr Phe
               20                  25                  30
   Cys Asn Asp Thr Val Val Ile Pro Cys Phe Val Thr Asn Met Glu Ala
           35                  40                  45
   Gln Asn Thr Thr Glu Val Tyr Val Lys Trp Lys Phe Lys Gly Arg Asp
       50                  55                  60
   Ile Tyr Thr Phe Asp Gly Ala Leu Asn Lys Ser Thr Val Pro Thr Asp
   65                  70                  75                  80
   Phe Ser Ser Ala Lys Ile Glu Val Ser Gln Leu Leu Lys Gly Asp Ala
                   85                  90                  95
   Ser Leu Lys Met Asp Lys Ser Asp Ala Val Ser His Thr Gly Asn Tyr
               100                 105                 110
   Thr Cys Glu Val Thr Glu Leu Thr Arg Glu Gly Glu Thr Ile Ile Glu
           115                 120                 125
   Leu Lys Tyr Arg Val Val Ser Trp Phe Ser Pro Asn Glu Asn
       130                 135                 140
<210> 2
   <211> 5346
   <212> DNA
   <213> Homo sapiens
<400> 2
   ggggagcagg cgggggagcg ggcgggaagc agtgggagcg cgcgtgcgcg cggccgtgca 60
   gcctgggcag tgggtcctgc ctgtgacgcg cggcggcggt cggtcctgcc tgtaacggcg 120
   gcggcggctg ctgctccaga cacctgcggc ggcggcggcg accccgcggc gggcgcggag 180
   atgtggcccc tggtagcggc gctgttgctg ggctcggcgt gctgcggatc agctcagcta 240
   ctatttaata aaacaaaatc tgtagaattc acgttttgta atgacactgt cgtcattcca 300
   tgctttgtta ctaatatgga ggcacaaaac actactgaag tatacgtaaa gtggaaattt 360
   aaaggaagag atatttacac ctttgatgga gctctaaaca agtccactgt ccccactgac 420
   tttagtagtg caaaaattga agtctcacaa ttactaaaag gagatgcctc tttgaagatg 480
   gataagagtg atgctgtctc acacacagga aactacactt gtgaagtaac agaattaacc 540
   agagaaggtg aaacgatcat cgagctaaaa tatcgtgttg tttcatggtt ttctccaaat 600
   gaaaatattc ttattgttat tttcccaatt tttgctatac tcctgttctg gggacagttt 660
   ggtattaaaa cacttaaata tagatccggt ggtatggatg agaaaacaat tgctttactt 720
   gttgctggac tagtgatcac tgtcattgtc attgttggag ccattctttt cgtcccaggt 780
   gaatattcat taaagaatgc tactggcctt ggtttaattg tgacttctac agggatatta 840
   atattacttc actactatgt gtttagtaca gcgattggat taacctcctt cgtcattgcc 900
   atattggtta ttcaggtgat agcctatatc ctcgctgtgg ttggactgag tctctgtatt 960
   gcggcgtgta taccaatgca tggccctctt ctgatttcag gtttgagtat cttagctcta 1020
   gcacaattac ttggactagt ttatatgaaa tttgtggctt ccaatcagaa gactatacaa 1080
   cctcctagga aagctgtaga ggaacccctt aatgcattca aagaatcaaa aggaatgatg 1140
   aatgatgaat aactgaagtg aagtgatgga ctccgatttg gagagtagta agacgtgaaa 1200
   ggaatacact tgtgtttaag caccatggcc ttgatgattc actgttgggg agaagaaaca 1260
   agaaaagtaa ctggttgtca cctatgagac ccttacgtga ttgttagtta agtttttatt 1320
   caaagcagct gtaatttagt taataaaata attatgatct atgttgtttg cccaattgag 1380
   atccagtttt ttgttgttat ttttaatcaa ttaggggcaa tagtagaatg gacaatttcc 1440
   aagaatgatg cctttcaggt cctagggcct ctggcctcta ggtaaccagt ttaaattggt 1500
   tcagggtgat aactacttag cactgccctg gtgattaccc agagatatct atgaaaacca 1560
   gtggcttcca tcaaaccttt gccaactcag gttcacagca gctttgggca gttatggcag 1620
   tatggcatta gctgagaggt gtctgccact tctgggtcaa tggaataata aattaagtac 1680
   aggcaggaat ttggttggga gcatcttgta tgatctccgt atgatgtgat attgatggag 1740
   atagtggtcc tcattcttgg gggttgccat tcccacattc ccccttcaac aaacagtgta 1800
   acaggtcctt cccagattta gggtactttt attgatggat atgttttcct tttattcaca 1860
   taaccccttg aaaccctgtc ttgtcctcct gttacttgct tctgctgtac aagatgtagc 1920
   accttttctc ctctttgaac atggtctagt gacacggtag caccagttgc aggaaggagc 1980
   cagacttgtt ctcagagcac tgtgttcaca cttttcagca aaaatagcta tggttgtaac 2040
   atatgtattc ccttcctctg atttgaaggc aaaaatctac agtgtttctt cacttctttt 2100
   ctgatctggg gcatgaaaaa agcaagattg aaatttgaac tatgagtctc ctgcatggca 2160
   acaaaatgtg tgtcaccatc aggccaacag gccagccctt gaatggggat ttattactgt 2220
   tgtatctatg ttgcatgata aacattcatc accttcctcc tgtagtcctg cctcgtactc 2280
   cccttcccct atgattgaaa agtaaacaaa acccacattt cctatcctgg ttagaagaaa 2340
   attaatgttc tgacagttgt gatcgcctgg agtactttta gacttttagc attcgttttt 2400
   tacctgtttg tggatgtgtg tttgtatgtg catacgtatg agataggcac atgcatcttc 2460
   tgtatggaca aaggtggggt acctacagga gagcaaaggt taattttgtg cttttagtaa 2520
   aaacatttaa atacaaagtt ctttattggg tggaattata tttgatgcaa atatttgatc 2580
   acttaaaact tttaaaactt ctaggtaatt tgccacgctt tttgactgct caccaatacc 2640
   ctgtaaaaat acgtaattct tcctgtttgt gtaataagat attcatattt gtagttgcat 2700
   taataatagt tatttcttag tccatcagat gttcccgtgt gcctctttta tgccaaattg 2760
   attgtcatat ttcatgttgg gaccaagtag tttgcccatg gcaaacctaa atttatgacc 2820
   tgctgaggcc tctcagaaaa ctgagcatac tagcaagaca gctcttcttg aaaaaaaaaa 2880
   tatgtataca caaatatata cgtatatcta tatatacgta tgtatataca cacatgtata 2940
   ttcttccttg attgtgtagc tgtccaaaat aataacatat atagagggag ctgtattcct 3000
   ttatacaaat ctgatggctc ctgcagcact ttttccttct gaaaatattt acattttgct 3060
   aacctagttt gttactttaa aaatcagttt tgatgaaagg agggaaaagc agatggactt 3120
   gaaaaagatc caagctccta ttagaaaagg tatgaaaatc tttatagtaa aattttttat 3180
   aaactaaagt tgtacctttt aatatgtagt aaactctcat ttatttgggg ttcgctcttg 3240
   gatctcatcc atccattgtg ttctctttaa tgctgcctgc cttttgaggc attcactgcc 3300
   ctagacaatg ccaccagaga tagtggggga aatgccagat gaaaccaact cttgctctca 3360
   ctagttgtca gcttctctgg ataagtgacc acagaagcag gagtcctcct gcttgggcat 3420
   cattgggcca gttccttctc tttaaatcag atttgtaatg gctcccaaat tccatcacat 3480
   cacatttaaa ttgcagacag tgttttgcac atcatgtatc tgttttgtcc cataatatgc 3540
   tttttactcc ctgatcccag tttctgctgt tgactcttcc attcagtttt atttattgtg 3600
   tgttctcaca gtgacaccat ttgtcctttt ctgcaacaac ctttccagct acttttgcca 3660
   aattctattt gtcttctcct tcaaaacatt ctcctttgca gttcctcttc atctgtgtag 3720
   ctgctctttt gtctcttaac ttaccattcc tatagtactt tatgcatctc tgcttagttc 3780
   tattagtttt ttggccttgc tcttctcctt gattttaaaa ttccttctat agctagagct 3840
   tttctttctt tcattctctc ttcctgcagt gttttgcata catcagaagc taggtacata 3900
   agttaaatga ttgagagttg gctgtattta gatttatcac tttttaatag ggtgagcttg 3960
   agagttttct ttctttctgt tttttttttt tgtttttttt tttttttttt tttttttttt 4020
   ttttgactaa tttcacatgc tctaaaaacc ttcaaaggtg attatttttc tcctggaaac 4080
   tccaggtcca ttctgtttaa atccctaaga atgtcagaat taaaataaca gggctatccc 4140
   gtaattggaa atatttcttt tttcaggatg ctatagtcaa tttagtaagt gaccaccaaa 4200
   ttgttatttg cactaacaaa gctcaaaaca cgataagttt actcctccat ctcagtaata 4260
   aaaattaagc tgtaatcaac cttctaggtt tctcttgtct taaaatgggt attcaaaaat 4320
   ggggatctgt ggtgtatgta tggaaacaca tactccttaa tttacctgtt gttggaaact 4380
   ggagaaatga ttgtcgggca accgtttatt ttttattgta ttttatttgg ttgagggatt 4440
   tttttataaa cagttttact tgtgtcatat tttaaaatta ctaactgcca tcacctgctg 4500
   gggtcctttg ttaggtcatt ttcagtgact aatagggata atccaggtaa ctttgaagag 4560
   atgagcagtg agtgaccagg cagtttttct gcctttagct ttgacagttc ttaattaaga 4620
   tcattgaaga ccagctttct cataaatttc tctttttgaa aaaaagaaag catttgtact 4680
   aagctcctct gtaagacaac atcttaaatc ttaaaagtgt tgttatcatg actggtgaga 4740
   gaagaaaaca ttttgttttt attaaatgga gcattattta caaaaagcca ttgttgagaa 4800
   ttagatccca catcgtataa atatctatta accattctaa ataaagagaa ctccagtgtt 4860
   gctatgtgca agatcctctc ttggagcttt tttgcatagc aattaaaggt gtgctatttg 4920
   tcagtagcca tttttttgca gtgatttgaa gaccaaagtt gttttacagc tgtgttaccg 4980
   ttaaaggttt ttttttttat atgtattaaa tcaatttatc actgtttaaa gctttgaata 5040
   tctgcaatct ttgccaaggt acttttttat ttaaaaaaaa acataacttt gtaaatatta 5100
   ccctgtaata ttatatatac ttaataaaac attttaagct attttgttgg gctatttcta 5160
   ttgctgctac agcagaccac aagcacattt ctgaaaaatt taatttatta atgtattttt 5220
   aagttgctta tattctaggt aacaatgtaa agaatgattt aaaatattaa ttatgaattt 5280
   tttgagtata atacccaata agcttttaat tagagcagag ttttaattaa aagttttaaa 5340
   tcagtc                                                            5346
<210> 3
   <211> 5288
   <212> DNA
   <213> Homo sapiens
<400> 3
   ggggagcagg cgggggagcg ggcgggaagc agtgggagcg cgcgtgcgcg cggccgtgca 60
   gcctgggcag tgggtcctgc ctgtgacgcg cggcggcggt cggtcctgcc tgtaacggcg 120
   gcggcggctg ctgctccaga cacctgcggc ggcggcggcg accccgcggc gggcgcggag 180
   atgtggcccc tggtagcggc gctgttgctg ggctcggcgt gctgcggatc agctcagcta 240
   ctatttaata aaacaaaatc tgtagaattc acgttttgta atgacactgt cgtcattcca 300
   tgctttgtta ctaatatgga ggcacaaaac actactgaag tatacgtaaa gtggaaattt 360
   aaaggaagag atatttacac ctttgatgga gctctaaaca agtccactgt ccccactgac 420
   tttagtagtg caaaaattga agtctcacaa ttactaaaag gagatgcctc tttgaagatg 480
   gataagagtg atgctgtctc acacacagga aactacactt gtgaagtaac agaattaacc 540
   agagaaggtg aaacgatcat cgagctaaaa tatcgtgttg tttcatggtt ttctccaaat 600
   gaaaatattc ttattgttat tttcccaatt tttgctatac tcctgttctg gggacagttt 660
   ggtattaaaa cacttaaata tagatccggt ggtatggatg agaaaacaat tgctttactt 720
   gttgctggac tagtgatcac tgtcattgtc attgttggag ccattctttt cgtcccaggt 780
   gaatattcat taaagaatgc tactggcctt ggtttaattg tgacttctac agggatatta 840
   atattacttc actactatgt gtttagtaca gcgattggat taacctcctt cgtcattgcc 900
   atattggtta ttcaggtgat agcctatatc ctcgctgtgg ttggactgag tctctgtatt 960
   gcggcgtgta taccaatgca tggccctctt ctgatttcag gtttgagtat cttagctcta 1020
   gcacaattac ttggactagt ttatatgaaa tttgtggctt ccaatcagaa gactatacaa 1080
   cctcctagga ataactgaag tgaagtgatg gactccgatt tggagagtag taagacgtga 1140
   aaggaataca cttgtgttta agcaccatgg ccttgatgat tcactgttgg ggagaagaaa 1200
   caagaaaagt aactggttgt cacctatgag acccttacgt gattgttagt taagttttta 1260
   ttcaaagcag ctgtaattta gttaataaaa taattatgat ctatgttgtt tgcccaattg 1320
   agatccagtt ttttgttgtt atttttaatc aattaggggc aatagtagaa tggacaattt 1380
   ccaagaatga tgcctttcag gtcctagggc ctctggcctc taggtaacca gtttaaattg 1440
   gttcagggtg ataactactt agcactgccc tggtgattac ccagagatat ctatgaaaac 1500
   cagtggcttc catcaaacct ttgccaactc aggttcacag cagctttggg cagttatggc 1560
   agtatggcat tagctgagag gtgtctgcca cttctgggtc aatggaataa taaattaagt 1620
   acaggcagga atttggttgg gagcatcttg tatgatctcc gtatgatgtg atattgatgg 1680
   agatagtggt cctcattctt gggggttgcc attcccacat tcccccttca acaaacagtg 1740
   taacaggtcc ttcccagatt tagggtactt ttattgatgg atatgttttc cttttattca 1800
   cataacccct tgaaaccctg tcttgtcctc ctgttacttg cttctgctgt acaagatgta 1860
   gcaccttttc tcctctttga acatggtcta gtgacacggt agcaccagtt gcaggaagga 1920
   gccagacttg ttctcagagc actgtgttca cacttttcag caaaaatagc tatggttgta 1980
   acatatgtat tcccttcctc tgatttgaag gcaaaaatct acagtgtttc ttcacttctt 2040
   ttctgatctg ggcatgaaaa aaagcaagat tgaaatttga actatgagtc tcctgcatgg 2100
   caacaaaatg tgtgtcacca tcaggccaac aggccagccc ttgaatgggg atttattact 2160
   gttgtatcta tgttgcatga taaacattca tcaccttcct cctgtagtcc tgcctcgtac 2220
   tccccttccc ctatgattga aaagtaaaca aaacccacat ttcctatcct ggttagaaga 2280
   aaattaatgt tctgacagtt gtgatcgcct ggagtacttt tagactttta gcattcgttt 2340
   tttacctgtt tgtggatgtg tgtttgtatg tgcatacgta tgagataggc acatgcatct 2400
   tctgtatgga caaaggtggg gtacctacag gagagcaaag gttaattttg tgcttttagt 2460
   aaaaacattt aaatacaaag ttctttattg ggtggaatta tatttgatgc aaatatttga 2520
   tcacttaaaa cttttaaaac ttctaggtaa tttgccacgc tttttgactg ctcaccaata 2580
   ccctgtaaaa atacgtaatt cttcctgttt gtgtaataag atattcatat ttgtagttgc 2640
   attaataata gttatttctt agtccatcag atgttcccgt gtgcctcttt tatgccaaat 2700
   tgattgtcat atttcatgtt gggaccaagt agtttgccca tggcaaacct aaatttatga 2760
   cctgctgagg cctctcagaa aactgagcat actagcaaga cagctcttct tgaaaaaaaa 2820
   aatatgtata cacaaatata tacgtatatc tatatatacg tatgtatata cacacatgta 2880
   tattcttcct tgattgtgta gctgtccaaa ataataacat atatagaggg agctgtattc 2940
   ctttatacaa atctgatggc tcctgcagca ctttttcctt ctgaaaatat ttacattttg 3000
   ctaacctagt ttgttacttt aaaaatcagt tttgatgaaa ggagggaaaa gcagatggac 3060
   ttgaaaaaga tccaagctcc tattagaaaa ggtatgaaaa tctttatagt aaaatttttt 3120
   ataaactaaa gttgtacctt ttaatatgta gtaaactctc atttatttgg ggttcgctct 3180
   tggatctcat ccatccattg tgttctcttt aatgctgcct gccttttgag gcattcactg 3240
   ccctagacaa tgccaccaga gatagtgggg gaaatgccag atgaaaccaa ctcttgctct 3300
   cactagttgt cagcttctct ggataagtga ccacagaagc aggagtcctc ctgcttgggc 3360
   atcattgggc cagttccttc tctttaaatc agatttgtaa tggctcccaa attccatcac 3420
   atcacattta aattgcagac agtgttttgc acatcatgta tctgttttgt cccataatat 3480
   gctttttact ccctgatccc agtttctgct gttgactctt ccattcagtt ttatttattg 3540
   tgtgttctca cagtgacacc atttgtcctt ttctgcaaca acctttccag ctacttttgc 3600
   caaattctat ttgtcttctc cttcaaaaca ttctcctttg cagttcctct tcatctgtgt 3660
   agctgctctt ttgtctctta acttaccatt cctatagtac tttatgcatc tctgcttagt 3720
   tctattagtt ttttggcctt gctcttctcc ttgattttaa aattccttct atagctagag 3780
   cttttctttc tttcattctc tcttcctgca gtgttttgca tacatcagaa gctaggtaca 3840
   taagttaaat gattgagagt tggctgtatt tagatttatc actttttaat agggtgagct 3900
   tgagagtttt ctttctttct gttttttttt tttgtttttt tttttttttt tttttttttt 3960
   ttttttgact aatttcacat gctctaaaaa ccttcaaagg tgattatttt tctcctggaa 4020
   actccaggtc cattctgttt aaatccctaa gaatgtcaga attaaaataa cagggctatc 4080
   ccgtaattgg aaatatttct tttttcagga tgctatagtc aatttagtaa gtgaccacca 4140
   aattgttatt tgcactaaca aagctcaaaa cacgataagt ttactcctcc atctcagtaa 4200
   taaaaattaa gctgtaatca accttctagg tttctcttgt cttaaaatgg gtattcaaaa 4260
   atggggatct gtggtgtatg tatggaaaca catactcctt aatttacctg ttgttggaaa 4320
   ctggagaaat gattgtcggg caaccgttta ttttttattg tattttattt ggttgaggga 4380
   tttttttata aacagtttta cttgtgtcat attttaaaat tactaactgc catcacctgc 4440
   tggggtcctt tgttaggtca ttttcagtga ctaataggga taatccaggt aactttgaag 4500
   agatgagcag tgagtgacca ggcagttttt ctgcctttag ctttgacagt tcttaattaa 4560
   gatcattgaa gaccagcttt ctcataaatt tctctttttg aaaaaaagaa agcatttgta 4620
   ctaagctcct ctgtaagaca acatcttaaa tcttaaaagt gttgttatca tgactggtga 4680
   gagaagaaaa cattttgttt ttattaaatg gagcattatt tacaaaaagc cattgttgag 4740
   aattagatcc cacatcgtat aaatatctat taaccattct aaataaagag aactccagtg 4800
   ttgctatgtg caagatcctc tcttggagct tttttgcata gcaattaaag gtgtgctatt 4860
   tgtcagtagc catttttttg cagtgatttg aagaccaaag ttgttttaca gctgtgttac 4920
   cgttaaaggt tttttttttt atatgtatta aatcaattta tcactgttta aagctttgaa 4980
   tatctgcaat ctttgccaag gtactttttt atttaaaaaa aaacataact ttgtaaatat 5040
   taccctgtaa tattatatat acttaataaa acattttaag ctattttgtt gggctatttc 5100
   tattgctgct acagcagacc acaagcacat ttctgaaaaa tttaatttat taatgtattt 5160
   ttaagttgct tatattctag gtaacaatgt aaagaatgat ttaaaatatt aattatgaat 5220
   tttttgagta taatacccaa taagctttta attagagcag agttttaatt aaaagtttta 5280
   aatcagtc                                                          5288
<210> 4
   <211> 5313
   <212> DNA
   <213> Homo sapiens
<400> 4
   ggggagcagg cgggggagcg ggcgggaagc agtgggagcg cgcgtgcgcg cggccgtgca 60
   gcctgggcag tgggtcctgc ctgtgacgcg cggcggcggt cggtcctgcc tgtaacggcg 120
   gcggcggctg ctgctccaga cacctgcggc ggcggcggcg accccgcggc gggcgcggag 180
   atgtggcccc tggtagcggc gctgttgctg ggctcggcgt gctgcggatc agctcagcta 240
   ctatttaata aaacaaaatc tgtagaattc acgttttgta atgacactgt cgtcattcca 300
   tgctttgtta ctaatatgga ggcacaaaac actactgaag tatacgtaaa gtggaaattt 360
   aaaggaagag atatttacac ctttgatgga gctctaaaca agtccactgt ccccactgac 420
   tttagtagtg caaaaattga agtctcacaa ttactaaaag gagatgcctc tttgaagatg 480
   gataagagtg atgctgtctc acacacagga aactacactt gtgaagtaac agaattaacc 540
   agagaaggtg aaacgatcat cgagctaaaa tatcgtgttg tttcatggtt ttctccaaat 600
   gaaaatattc ttattgttat tttcccaatt tttgctatac tcctgttctg gggacagttt 660
   ggtattaaaa cacttaaata tagatccggt ggtatggatg agaaaacaat tgctttactt 720
   gttgctggac tagtgatcac tgtcattgtc attgttggag ccattctttt cgtcccaggt 780
   gaatattcat taaagaatgc tactggcctt ggtttaattg tgacttctac agggatatta 840
   atattacttc actactatgt gtttagtaca gcgattggat taacctcctt cgtcattgcc 900
   atattggtta ttcaggtgat agcctatatc ctcgctgtgg ttggactgag tctctgtatt 960
   gcggcgtgta taccaatgca tggccctctt ctgatttcag gtttgagtat cttagctcta 1020
   gcacaattac ttggactagt ttatatgaaa tttgtggctt ccaatcagaa gactatacaa 1080
   cctcctagga aagctgtaga ggaacccctt aatgaataac tgaagtgaag tgatggactc 1140
   cgatttggag agtagtaaga cgtgaaagga atacacttgt gtttaagcac catggccttg 1200
   atgattcact gttggggaga agaaacaaga aaagtaactg gttgtcacct atgagaccct 1260
   tacgtgattg ttagttaagt ttttattcaa agcagctgta atttagttaa taaaataatt 1320
   atgatctatg ttgtttgccc aattgagatc cagttttttg ttgttatttt taatcaatta 1380
   ggggcaatag tagaatggac aatttccaag aatgatgcct ttcaggtcct agggcctctg 1440
   gcctctaggt aaccagttta aattggttca gggtgataac tacttagcac tgccctggtg 1500
   attacccaga gatatctatg aaaaccagtg gcttccatca aacctttgcc aactcaggtt 1560
   cacagcagct ttgggcagtt atggcagtat ggcattagct gagaggtgtc tgccacttct 1620
   gggtcaatgg aataataaat taagtacagg caggaatttg gttgggagca tcttgtatga 1680
   tctccgtatg atgtgatatt gatggagata gtggtcctca ttcttggggg ttgccattcc 1740
   cacattcccc cttcaacaaa cagtgtaaca ggtccttccc agatttaggg tacttttatt 1800
   gatggatatg ttttcctttt attcacataa ccccttgaaa ccctgtcttg tcctcctgtt 1860
   acttgcttct gctgtacaag atgtagcacc ttttctcctc tttgaacatg gtctagtgac 1920
   acggtagcac cagttgcagg aaggagccag acttgttctc agagcactgt gttcacactt 1980
   ttcagcaaaa atagctatgg ttgtaacata tgtattccct tcctctgatt tgaaggcaaa 2040
   aatctacagt gtttcttcac ttcttttctg atctggggca tgaaaaaagc aagattgaaa 2100
   tttgaactat gagtctcctg catggcaaca aaatgtgtgt caccatcagg ccaacaggcc 2160
   agcccttgaa tggggattta ttactgttgt atctatgttg catgataaac attcatcacc 2220
   ttcctcctgt agtcctgcct cgtactcccc ttcccctatg attgaaaagt aaacaaaacc 2280
   cacatttcct atcctggtta gaagaaaatt aatgttctga cagttgtgat cgcctggagt 2340
   acttttagac ttttagcatt cgttttttac ctgtttgtgg atgtgtgttt gtatgtgcat 2400
   acgtatgaga taggcacatg catcttctgt atggacaaag gtggggtacc tacaggagag 2460
   caaaggttaa ttttgtgctt ttagtaaaaa catttaaata caaagttctt tattgggtgg 2520
   aattatattt gatgcaaata tttgatcact taaaactttt aaaacttcta ggtaatttgc 2580
   cacgcttttt gactgctcac caataccctg taaaaatacg taattcttcc tgtttgtgta 2640
   ataagatatt catatttgta gttgcattaa taatagttat ttcttagtcc atcagatgtt 2700
   cccgtgtgcc tcttttatgc caaattgatt gtcatatttc atgttgggac caagtagttt 2760
   gcccatggca aacctaaatt tatgacctgc tgaggcctct cagaaaactg agcatactag 2820
   caagacagct cttcttgaaa aaaaaaatat gtatacacaa atatatacgt atatctatat 2880
   atacgtatgt atatacacac atgtatattc ttccttgatt gtgtagctgt ccaaaataat 2940
   aacatatata gagggagctg tattccttta tacaaatctg atggctcctg cagcactttt 3000
   tccttctgaa aatatttaca ttttgctaac ctagtttgtt actttaaaaa tcagttttga 3060
   tgaaaggagg gaaaagcaga tggacttgaa aaagatccaa gctcctatta gaaaaggtat 3120
   gaaaatcttt atagtaaaat tttttataaa ctaaagttgt accttttaat atgtagtaaa 3180
   ctctcattta tttggggttc gctcttggat ctcatccatc cattgtgttc tctttaatgc 3240
   tgcctgcctt ttgaggcatt cactgcccta gacaatgcca ccagagatag tgggggaaat 3300
   gccagatgaa accaactctt gctctcacta gttgtcagct tctctggata agtgaccaca 3360
   gaagcaggag tcctcctgct tgggcatcat tgggccagtt ccttctcttt aaatcagatt 3420
   tgtaatggct cccaaattcc atcacatcac atttaaattg cagacagtgt tttgcacatc 3480
   atgtatctgt tttgtcccat aatatgcttt ttactccctg atcccagttt ctgctgttga 3540
   ctcttccatt cagttttatt tattgtgtgt tctcacagtg acaccatttg tccttttctg 3600
   caacaacctt tccagctact tttgccaaat tctatttgtc ttctccttca aaacattctc 3660
   ctttgcagtt cctcttcatc tgtgtagctg ctcttttgtc tcttaactta ccattcctat 3720
   agtactttat gcatctctgc ttagttctat tagttttttg gccttgctct tctccttgat 3780
   tttaaaattc cttctatagc tagagctttt ctttctttca ttctctcttc ctgcagtgtt 3840
   ttgcatacat cagaagctag gtacataagt taaatgattg agagttggct gtatttagat 3900
   ttatcacttt ttaatagggt gagcttgaga gttttctttc tttctgtttt ttttttttgt 3960
   tttttttttt tttttttttt tttttttttt tgactaattt cacatgctct aaaaaccttc 4020
   aaaggtgatt atttttctcc tggaaactcc aggtccattc tgtttaaatc cctaagaatg 4080
   tcagaattaa aataacaggg ctatcccgta attggaaata tttctttttt caggatgcta 4140
   tagtcaattt agtaagtgac caccaaattg ttatttgcac taacaaagct caaaacacga 4200
   taagtttact cctccatctc agtaataaaa attaagctgt aatcaacctt ctaggtttct 4260
   cttgtcttaa aatgggtatt caaaaatggg gatctgtggt gtatgtatgg aaacacatac 4320
   tccttaattt acctgttgtt ggaaactgga gaaatgattg tcgggcaacc gtttattttt 4380
   tattgtattt tatttggttg agggattttt ttataaacag ttttacttgt gtcatatttt 4440
   aaaattacta actgccatca cctgctgggg tcctttgtta ggtcattttc agtgactaat 4500
   agggataatc caggtaactt tgaagagatg agcagtgagt gaccaggcag tttttctgcc 4560
   tttagctttg acagttctta attaagatca ttgaagacca gctttctcat aaatttctct 4620
   ttttgaaaaa aagaaagcat ttgtactaag ctcctctgta agacaacatc ttaaatctta 4680
   aaagtgttgt tatcatgact ggtgagagaa gaaaacattt tgtttttatt aaatggagca 4740
   ttatttacaa aaagccattg ttgagaatta gatcccacat cgtataaata tctattaacc 4800
   attctaaata aagagaactc cagtgttgct atgtgcaaga tcctctcttg gagctttttt 4860
   gcatagcaat taaaggtgtg ctatttgtca gtagccattt ttttgcagtg atttgaagac 4920
   caaagttgtt ttacagctgt gttaccgtta aaggtttttt tttttatatg tattaaatca 4980
   atttatcact gtttaaagct ttgaatatct gcaatctttg ccaaggtact tttttattta 5040
   aaaaaaaaca taactttgta aatattaccc tgtaatatta tatatactta ataaaacatt 5100
   ttaagctatt ttgttgggct atttctattg ctgctacagc agaccacaag cacatttctg 5160
   aaaaatttaa tttattaatg tatttttaag ttgcttatat tctaggtaac aatgtaaaga 5220
   atgatttaaa atattaatta tgaatttttt gagtataata cccaataagc ttttaattag 5280
   agcagagttt taattaaaag ttttaaatca gtc                              5313
<210> 5
   <211> 323
   <212> PRT
   <213> Homo sapiens
<400> 5
   Met Trp Pro Leu Val Ala Ala Leu Leu Leu Gly Ser Ala Cys Cys Gly
    1               5                  10                  15
   Ser Ala Gln Leu Leu Phe Asn Lys Thr Lys Ser Val Glu Phe Thr Phe
               20                  25                  30
   Cys Asn Asp Thr Val Val Ile Pro Cys Phe Val Thr Asn Met Glu Ala
           35                  40                  45
   Gln Asn Thr Thr Glu Val Tyr Val Lys Trp Lys Phe Lys Gly Arg Asp
       50                  55                  60
   Ile Tyr Thr Phe Asp Gly Ala Leu Asn Lys Ser Thr Val Pro Thr Asp
   65                  70                  75                  80
   Phe Ser Ser Ala Lys Ile Glu Val Ser Gln Leu Leu Lys Gly Asp Ala
                   85                  90                  95
   Ser Leu Lys Met Asp Lys Ser Asp Ala Val Ser His Thr Gly Asn Tyr
               100                 105                 110
   Thr Cys Glu Val Thr Glu Leu Thr Arg Glu Gly Glu Thr Ile Ile Glu
           115                 120                 125
   Leu Lys Tyr Arg Val Val Ser Trp Phe Ser Pro Asn Glu Asn Ile Leu
       130                 135                 140
   Ile Val Ile Phe Pro Ile Phe Ala Ile Leu Leu Phe Trp Gly Gln Phe
   145                 150                 155                 160
   Gly Ile Lys Thr Leu Lys Tyr Arg Ser Gly Gly Met Asp Glu Lys Thr
                   165                 170                 175
   Ile Ala Leu Leu Val Ala Gly Leu Val Ile Thr Val Ile Val Ile Val
               180                 185                 190
   Gly Ala Ile Leu Phe Val Pro Gly Glu Tyr Ser Leu Lys Asn Ala Thr
           195                 200                 205
   Gly Leu Gly Leu Ile Val Thr Ser Thr Gly Ile Leu Ile Leu Leu His
       210                 215                 220
   Tyr Tyr Val Phe Ser Thr Ala Ile Gly Leu Thr Ser Phe Val Ile Ala
   225                 230                 235                 240
   Ile Leu Val Ile Gln Val Ile Ala Tyr Ile Leu Ala Val Val Gly Leu
                   245                 250                 255
   Ser Leu Cys Ile Ala Ala Cys Ile Pro Met His Gly Pro Leu Leu Ile
               260                 265                 270
   Ser Gly Leu Ser Ile Leu Ala Leu Ala Gln Leu Leu Gly Leu Val Tyr
           275                 280                 285
   Met Lys Phe Val Ala Ser Asn Gln Lys Thr Ile Gln Pro Pro Arg Lys
       290                 295                 300
   Ala Val Glu Glu Pro Leu Asn Ala Phe Lys Glu Ser Lys Gly Met Met
   305                 310                 315                 320
   Asn Asp Glu
<210> 6
   <211> 305
   <212> PRT
   <213> Homo sapiens
<400> 6
   Met Trp Pro Leu Val Ala Ala Leu Leu Leu Gly Ser Ala Cys Cys Gly
    1               5                  10                  15
   Ser Ala Gln Leu Leu Phe Asn Lys Thr Lys Ser Val Glu Phe Thr Phe
               20                  25                  30
   Cys Asn Asp Thr Val Val Ile Pro Cys Phe Val Thr Asn Met Glu Ala
           35                  40                  45
   Gln Asn Thr Thr Glu Val Tyr Val Lys Trp Lys Phe Lys Gly Arg Asp
       50                  55                  60
   Ile Tyr Thr Phe Asp Gly Ala Leu Asn Lys Ser Thr Val Pro Thr Asp
   65                  70                  75                  80
   Phe Ser Ser Ala Lys Ile Glu Val Ser Gln Leu Leu Lys Gly Asp Ala
                   85                  90                  95
   Ser Leu Lys Met Asp Lys Ser Asp Ala Val Ser His Thr Gly Asn Tyr
               100                 105                 110
   Thr Cys Glu Val Thr Glu Leu Thr Arg Glu Gly Glu Thr Ile Ile Glu
           115                 120                 125
   Leu Lys Tyr Arg Val Val Ser Trp Phe Ser Pro Asn Glu Asn Ile Leu
       130                 135                 140
   Ile Val Ile Phe Pro Ile Phe Ala Ile Leu Leu Phe Trp Gly Gln Phe
   145                 150                 155                 160
   Gly Ile Lys Thr Leu Lys Tyr Arg Ser Gly Gly Met Asp Glu Lys Thr
                   165                 170                 175
   Ile Ala Leu Leu Val Ala Gly Leu Val Ile Thr Val Ile Val Ile Val
               180                 185                 190
   Gly Ala Ile Leu Phe Val Pro Gly Glu Tyr Ser Leu Lys Asn Ala Thr
           195                 200                 205
   Gly Leu Gly Leu Ile Val Thr Ser Thr Gly Ile Leu Ile Leu Leu His
       210                 215                 220
   Tyr Tyr Val Phe Ser Thr Ala Ile Gly Leu Thr Ser Phe Val Ile Ala
   225                 230                 235                 240
   Ile Leu Val Ile Gln Val Ile Ala Tyr Ile Leu Ala Val Val Gly Leu
                   245                 250                 255
   Ser Leu Cys Ile Ala Ala Cys Ile Pro Met His Gly Pro Leu Leu Ile
               260                 265                 270
   Ser Gly Leu Ser Ile Leu Ala Leu Ala Gln Leu Leu Gly Leu Val Tyr
           275                 280                 285
   Met Lys Phe Val Ala Ser Asn Gln Lys Thr Ile Gln Pro Pro Arg Asn
       290                 295                 300
   Asn
   305
<210> 7
   <211> 312
   <212> PRT
   <213> Homo sapiens
<400> 7
   Met Trp Pro Leu Val Ala Ala Leu Leu Leu Gly Ser Ala Cys Cys Gly
    1               5                  10                  15
   Ser Ala Gln Leu Leu Phe Asn Lys Thr Lys Ser Val Glu Phe Thr Phe
   20 25 30
   Cys Asn Asp Thr Val Val Ile Pro Cys Phe Val Thr Asn Met Glu Ala
   35 40 45
   Gln Asn Thr Thr Glu Val Tyr Val Lys Trp Lys Phe Lys Gly Arg Asp
   50 55 60
   Ile Tyr Thr Phe Asp Gly Ala Leu Asn Lys Ser Thr Val Pro Thr Asp
   65 70 75 80
   Phe Ser Ser Ala Lys Ile Glu Val Ser Gln Leu Leu Lys Gly Asp Ala
   85 90 95
   Ser Leu Lys Met Asp Lys Ser Asp Ala Val Ser His Thr Gly Asn Tyr
   100 105 110
   Thr Cys Glu Val Thr Glu Leu Thr Arg Glu Gly Glu Thr Ile Ile Glu
   115 120 125
   Leu Lys Tyr Arg Val Val Ser Trp Phe Ser Pro Asn Glu Asn Ile Leu
   130 135 140
   Ile Val Ile Phe Pro Ile Phe Ala Ile Leu Leu Phe Trp Gly Gln Phe
   145 150 155 160
   Gly Ile Lys Thr Leu Lys Tyr Arg Ser Gly Gly Met Asp Glu Lys Thr
   165 170 175
   Ile Ala Leu Leu Val Ala Gly Leu Val Ile Thr Val Ile Val Ile Val
   180 185 190
   Gly Ala Ile Leu Phe Val Pro Gly Glu Tyr Ser Leu Lys Asn Ala Thr
   195 200 205
   Gly Leu Gly Leu Ile Val Thr Ser Thr Gly Ile Leu Ile Leu Leu His
   210 215 220
   Tyr Tyr Val Phe Ser Thr Ala Ile Gly Leu Thr Ser Phe Val Ile Ala
   225 230 235 240
   Ile Leu Val Ile Gln Val Ile Ala Tyr Ile Leu Ala Val Val Gly Leu
   245 250 255
   Ser Leu Cys Ile Ala Ala Cys Ile Pro Met His Gly Pro Leu Leu Ile
   260 265 270
   Ser Gly Leu Ser Ile Leu Ala Leu Ala Gln Leu Leu Gly Leu Val Tyr
   275 280 285
   Met Lys Phe Val Ala Ser Asn Gln Lys Thr Ile Gln Pro Pro Arg Lys
   290 295 300
   Ala Val Glu Glu Pro Leu Asn Glu
   305 310
<210> 8
   <211> 21
   <212> DNA
   <213> Artificial Sequence
<220>
   <223> 18S RNA forward primer
<400> 8
   ttgacggaag ggcaccacca g 21
<210> 9
   <211> 21
   <212> DNA
   <213> Artificial Sequence
<220>
   <223> 18S RNA reverse primer
<400> 9
   gcaccaccac ccacggaatc g 21
<210> 10
   <211> 20
   <212> DNA
   <213> Artificial Sequence
<220>
   <223> beta-actin forward primer
<400> 10
   ttccttcttg ggtatggaat 20
<210> 11
   <211> 20
   <212> DNA
   <213> Artificial Sequence
<220>
   <223> beta-actin reverse primer
<400> 11
   gagcaatgat cttgatcctc 20
<210> 12
   <211> 21
   <212> DNA
   <213> Artificial Sequence
<220>
   <223> CD47 forward primer
<400> 12
   aggccaagtc cagaagcatt c 21
<210> 13
   <211> 21
   <212> DNA
   <213> Artificial Sequence
<220>
   <223> CD47 reverse primer
<400> 13
   aatcattctg ctgctcgttg c 21
<210> 14
   <211> 34
   <212> DNA
   <213> Artificial Sequence
<220>
   <223> 3' Neo PCR primer
<400> 14
   gcatcgcatt gtctgagtag gtgtcattct attc 34
<210> 15
   <211> 28
   <212> DNA
   <213> Artificial Sequence
<220>
   <223> 5' IAP PCR primer
<400> 15
   tcaccttgtt gttcctgtac tacaagca 28
<210> 16
   <211> 23
   <212> DNA
   <213> Artificial Sequence
<220>
   <223> 3' IAP PCR Primer
<400> 16
   tgtcacttcg caagtgtagt tcc 23
<210> 17
   <211> 23
   <212> DNA
   <213> Artificial Sequence
<220>
   <223> FLT3-ITD PCR Forward Primer 11F
<400> 17
   gcaatttagg tatgaaagcc agc 23
<210> 18
   <211> 23
   <212> DNA
   <213> Artificial Sequence
<220>
   <223> FLT3-ITD PCR Reverse Primer 12R
<400> 18
   ctttcagcat tttgacggca acc 23

## Description

### BACKGROUND

The reticuloendothelial system (RES) is a part of the immune system. The RES consists of the phagocytic cells located in reticular connective tissue, primarily monocytes and macrophages. The RES consists of 1) circulating monocytes; 2) resident macrophages in the liver, spleen, lymph nodes, thymus, submucosal tissues of the respiratory and alimentary tracts, bone marrow, and connective tissues; and 3) macrophage-like cells including dendritic cells in lymph nodes, Langerhans cells in skin, and microglial cells in the central nervous system. These cells accumulate in lymph nodes and the spleen. The RES functions to clear pathogens, particulate matter in circulation, and aged or damaged hematopoietic cells.

To eliminate foreign cells or particles in the innate immune response, macrophage-mediated phagocytosis is induced when the phosphatidylserine receptor (PSR) reacts to phosphatidylserine (PS), which can be externalized from the membranes of dead cells, such as apoptotic and necrotic cells. In turn, the interaction between PS and PSR plays a crucial role in the clearance of apoptotic cells by macrophages. Once phagocytosis has been performed by macrophages, the inflammatory response is downregulated by an increase in factors such as IL-10, TGF-β, and prostaglandin E2 (PGE2). The strict balance between the inflammatory and anti-inflammatory responses in both innate and adaptive immunity plays a critical role in maintaining cellular homeostasis and protecting a host from extrinsic invasion.

EP1693385describes a humanized anti-CD47 antibody. Manna et al. (2004) Cancer Research 64(3):1026-1036 describes that CD47 mediates killing of breast tumor cells via Gi-dependent inhibition of protein kinase A. US 2007/111238 describes methods for diagnosing and evaluating treatment of blood disorders. Liu et al. (2002) Journal of Biological Chemistry 277(12):10028-10036 describes that Signal Regulatory Protein (SIRPalpha), a cellular ligand for CD47, regulates neutrophil transmigration. Majeti *et al.* (2008) describes that CD47 is an independent prognostic factor and therapeutic antibody target on human acute myeloid leukemia stem cells. Pettersen et al. (1999) describes that CD47 signals T cell death. Manna et al. (2003) The Journal of Immunology 170(7):3544-3553 describes a mechanism of CD47-dependent killing of T cells: heterodimeric Gi-dependent inhibition of protein kinase A.

The causal relationship between inflammation and the neoplastic progression is a concept widely accepted. Data now support the concept of cancer immunosurveillance - that one of the physiologic functions of the immune system is to recognize and destroy transformed cells. However, some tumor cells are capable of evading recognition and destruction by the immune system. Once tumor cells have escaped, the immune system may participate in their growth, for example by promoting the vascularization of tumors.

Both adaptive and innate immune cells participate in the surveillance and the elimination of tumor cells, but monocytes/macrophages may be the first line of defense in tumors, as they colonize rapidly and secrete cytokines that attract and activate dendritic cells (DC) and NK cells, which in turn can initiate the adaptive immune response against transformed cells.

Tumors that escape from the immune machinery can be a consequence of alterations occurring during the immunosurveillance phase. As an example, some tumor cells develop deficiencies in antigen processing and presentation pathways, which facilitate evasion from an adaptive immune response, such as the absence or abnormal functions of components of the IFN-y receptor signaling pathway. Other tumors suppress the induction of proinflammatory danger signals, leading, for example, to impaired DC maturation. Finally, the inhibition of the protective functions of the immune system may also facilitate tumor escape, such as the overproduction of the anti-inflammatory cytokines IL-10 and TGF-β, which can be produced by many tumor cells themselves but also by macrophages or T regulatory cells.

A tumor can be viewed as an aberrant organ initiated by a tumorigenic cancer cell that acquired the capacity for indefinite proliferation through accumulated mutations. In this view of a tumor as an abnormal organ, the principles of normal stem cell biology can be applied to better understand how tumors develop. Many observations suggest that analogies between normal stem cells and tumorigenic cells are appropriate. Both normal stem cells and tumorigenic cells have extensive proliferative potential and the ability to give rise to new (normal or abnormal) tissues. Both tumors and normal tissues are composed of heterogeneous combinations of cells, with different phenotypic characteristics and different proliferative potentials.

Stem cells are defined as cells that have the ability to perpetuate themselves through self-renewal and to generate mature cells of a particular tissue through differentiation. In most tissues, stem cells are rare. As a result, stem cells must be identified prospectively and purified carefully in order to study their properties. Perhaps the most important and useful property of stem cells is that of self-renewal. Through this property, striking parallels can be found between stem cells and cancer cells: tumors may often originate from the transformation of normal stem cells, similar signaling pathways may regulate self-renewal in stem cells and cancer cells, and cancers may comprise rare cells with indefinite potential for self-renewal that drive tumorigenesis.

Study of cell surface markers specific to or specifically upregulated in cancer cells is pivotal in providing targets for reducing growth of or for depleting cancer cells. Provided herein is a marker for myeloid leukemia, especially a marker for Acute Myeloid Leukemia (AML). Our studies have revealed a role of this marker in helping AML stem cells avoid clearance by phagocytosis. Methods are provided for using this marker to increase phagocytosis of AML stem cells (AML SCs), as well as to improve transplantation of hematopoietic and progenitor stem cells.

Interestingly, certain markers are shown to be shared by leukemia stem cells and hematopoietic stem cells (HSCs). During normal development, HSCs migrate to ectopic niches in fetal and adult life via the blood stream. Once in the blood stream, HSCs must navigate the vascular beds of the spleen and liver before settling in a niche. At these vascular beds, macrophages function to remove damaged cells and foreign particles from the blood stream. Furthermore, during inflammatory states, macrophages become more phagocytically active. The newly arriving stem cells thus face the possibility of being phagocytosed while en route, unless additional protection can be generated. Exploration of mechanisms by which the endogenous HSC avoid being cleared by phagocytosis can provide insight into ways for improving transplantation success of hematopoietic and progenitor stem cells. The present invention satisfies these, and other, needs.

The invention provides a composition comprising a monoclonal antibody that specifically binds CD47 on the surface of cancer cells and inhibits its interaction with SIRPα receptor on the surface of phagocytic cells, for use in treating an ovarian tumor by increasing phagocytosis of cancer cells of the ovarian tumor in a human subject, wherein said antibody up-regulates phagocytosis of said cancer cells by inhibiting the binding of CD47 on the surface of the cancer cells to SIRPα receptor on the surface of phagocytic cells.

Disclosed herein are methods to manipulate phagocytosis of hematopoietic cells, including circulating hematopoietic cells, e.g. bone marrow cells. In some instances, the circulating cells are hematopoietic stem cells, or hematopoietic progenitor cells, particularly in a transplantation context, where protection from phagocytosis is desirable. In other instances the circulating cells are leukemia cells, particularly acute myeloid leukemia (AML), where increased phagocytosis is desirable. In certain instances, the disclosed methods manipulate macrophage phagocytosis of circulating hematopoietic cells. In yet other instances, the disclosed methods manipulate phagocytosis of solid tumors.

### SUMMARY OF THE INVENTION

In some embodiments, hematopoietic stem or progenitor cells are protected from phagocytosis in circulation by providing a host animal with a CD47 mimetic molecule, which interacts with SIRPα on phagocytic cells, such as, macrophages, and decreases phagocytosis. The CD47 mimetic may be soluble CD47; CD47 coated on the surface of the cells to be protected, a CD47 mimetic that binds to SIRPα at the CD47 binding site, and the like. In some embodiments, CD47 is provided as a fusion protein, for example soluble CD47 fused to an Fc fragment, *e.g*., IgG1 Fc, IgG2 Fc, Ig A Fc *etc*.

In other instances, tumor cells, e.g. solid tumor cells, leukemia cells, etc. are targeted for phagocytosis by blocking CD47 on the cell surface. It is shown that leukemia cells, particularly AML cells, evade macrophage surveillance by upregulation of CD47 expression. In some instances, administration of agents that mask the CD47 protein, e.g. antibodies that bind to CD47 and prevent interaction between CD47 and SIRPα are administered to a patient, which increases the clearance of AML cells via phagocytosis. In other aspects of the disclosure, an agent that masks CD47 is combined with monoclonal antibodies directed against one or more additional AMLSC markers, e.g. CD96, and the like, which compositions can be synergistic in enhancing phagocytosis and elimination of AMLSC as compared to the use of single agents. In other instances, cells of solid tumors are targeted for phagocytosis by blocking CD47 present on the cell surface.

### BRIEF DESCRIPTION OF THE DRAWINGS

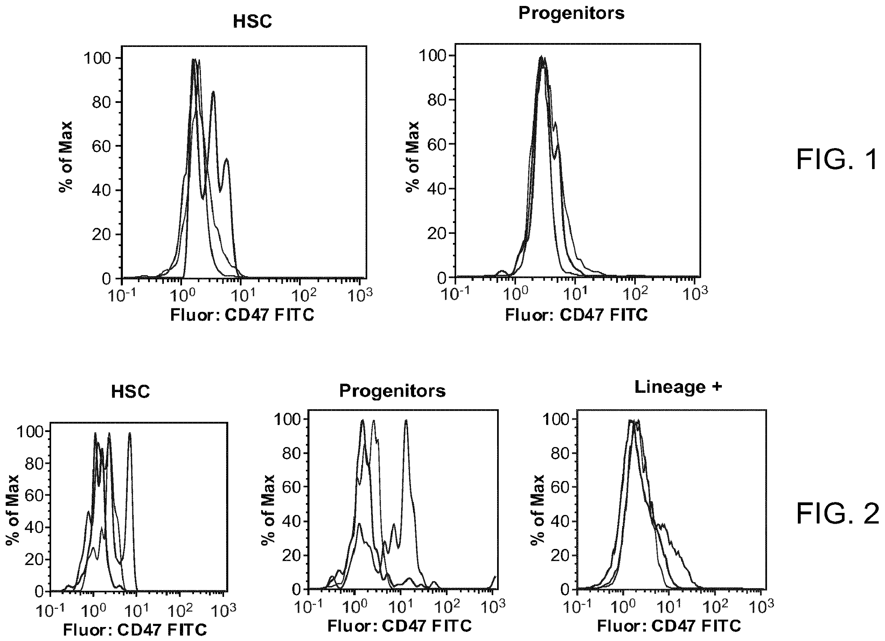

Figure 1. FACS analysis of human HSC and progenitor CD47 expression from Myelodysplastic syndrome (MDS, blue), Chronic Myelogenous Leukemia, Accelerated Phase (CML AP, green) and normal bone marrow (red).
Figure 2. ET vs. PV. FACS analysis of CD47 expression by human myeloproliferative disorders such as essential thrombocythemia (ET, blue) and polycythemia vera (PV, green) HSC, progenitor and lineage positive cells compared with human normal bone marrow (red).

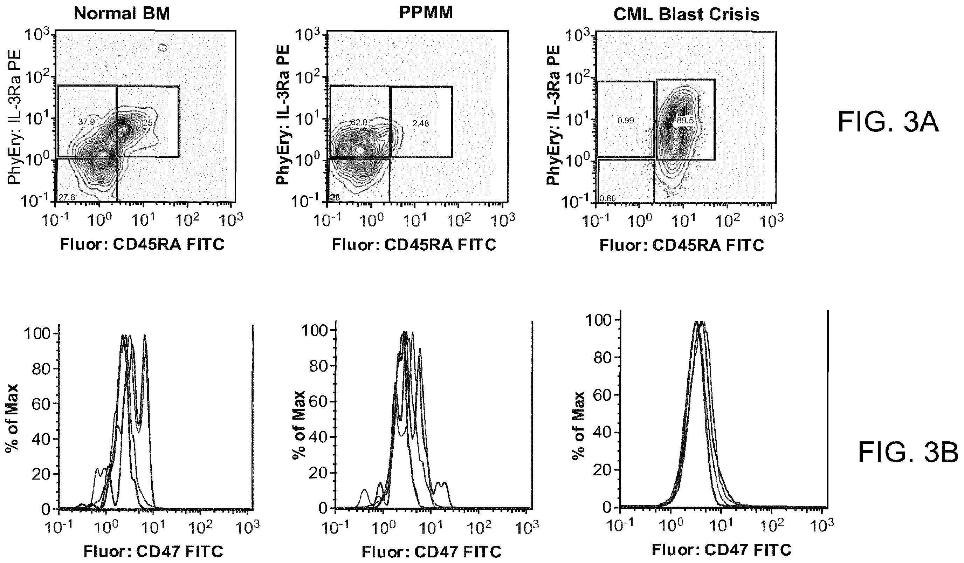

Figure 3A. Progenitor Profiles of Normal Bone Marrow (left), post-polycythemic myelofibrosis with myeloid metaplasia (PPMM) and CML Blast Crisis. Figure 3B. FACS analysis of human normal bone marrow (red) versus UMPD (green) versus PV (blue = ML) versus atypical CML (orange), HSC, progenitor and lineage positive cell CD47 expression.

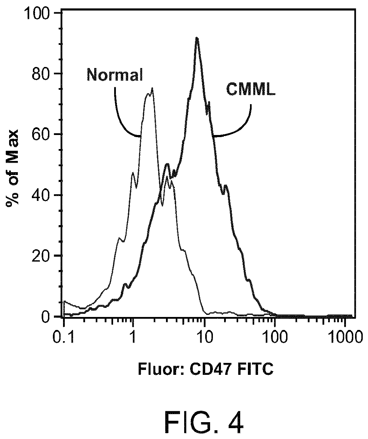

Figure 4. Increased CD47 Expression by CMML Progenitors (blue) compared with normal bone marrow (red) with disease progression.

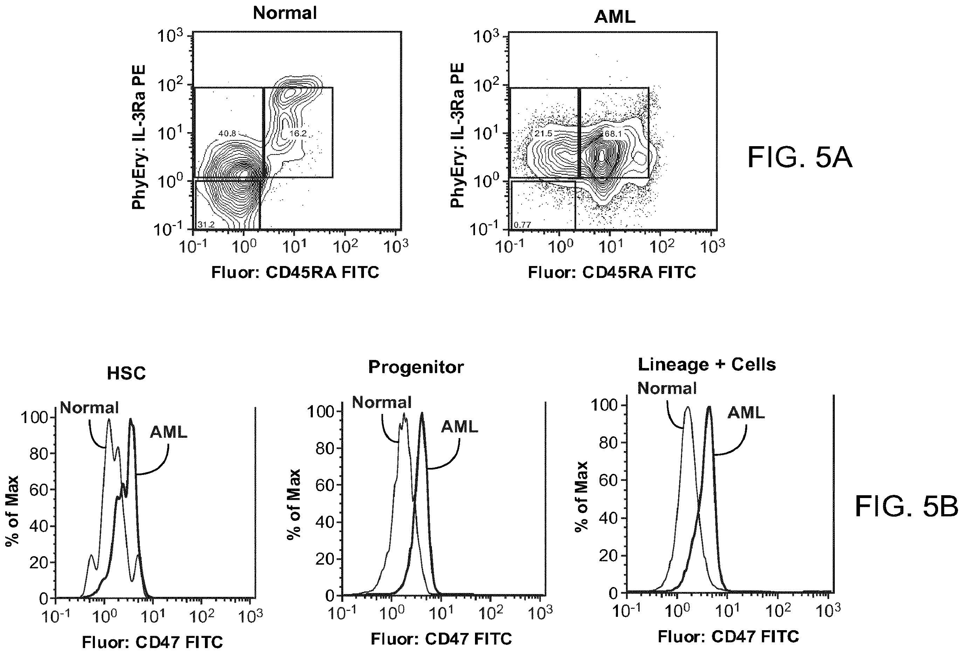

Figures 5A-5B. (A) Progenitor Profiles of Normal bone marrow (left) versus AML (right). (B) FACS analysis of human normal bone marrow (red) versus AML (blue) HSC, progenitor and lineage positive cell (blast) CD47 expression.

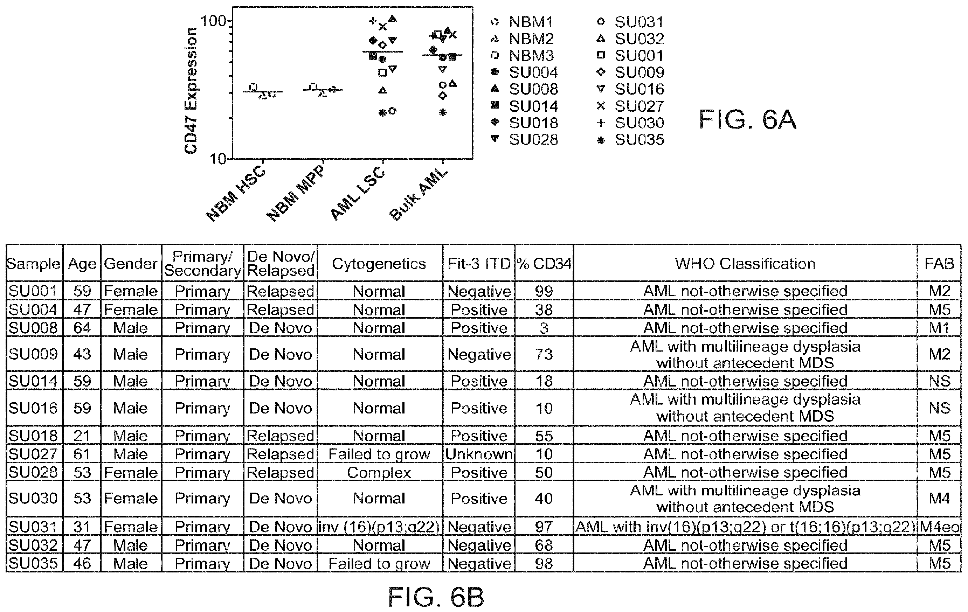

Figure 6. CD47 is More Highly Expressed on AML LSC Compared to Their Normal Counterparts. A. Relative CD47 expression on normal bone marrow HSC (Lin-CD34+CD38-CD90+) and MPP (Lin-CD34+CD38-CD90-CD45RA-), as well as LSC (Lin-CD34+CD38-CD90-) and bulk leukemia cells from human AML samples was determined by flow cytometry. Mean fluorescence intensity was normalized for cell size and against lineage positive cells to account for analysis on different days. The same sample of normal bone marrow (red, n=3) or AML (blue, n=13) is indicated by the same symbol in the different populations. The differences between the mean expression of HSC with LSC (p=0.003), HSC with bulk leukemia (p=0.001), MPP with LSC (p=0.004), and MPP with bulk leukemia (p=0.002) were statistically significant using a 2-sided Student's t-test. The difference between the mean expression of AML LSC compared to bulk AML was not statistically significant with p=0.50 using a paired 2-sided Student's t-test. B. Clinical and molecular characteristics of primary human AML samples manipulated in vitro and/or in vivo.

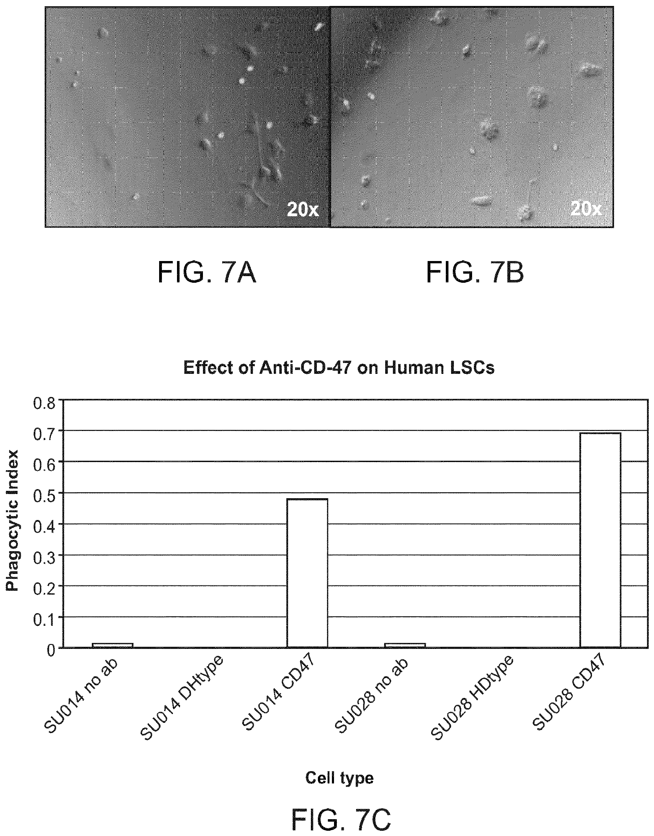

Figure 7. Anti-CD47 Antibody Stimulates *In Vitro* Macrophage Phagocytosis of Primary Human AML LSC. AML LSC were purified by FACS from two primary human AML samples, labeled with the fluorescent dye CFSE, and incubated with mouse bone marrow-derived macrophages either in the presence of an isotype control (A) or anti-CD47 antibody (B). These cells were assessed by immunofluorescence microscopy for the presence of fluorescently labeled LSC within the macrophages. (C) The phagocytic index was determined for each condition by calculating the number of ingested cells per 100 macrophages.

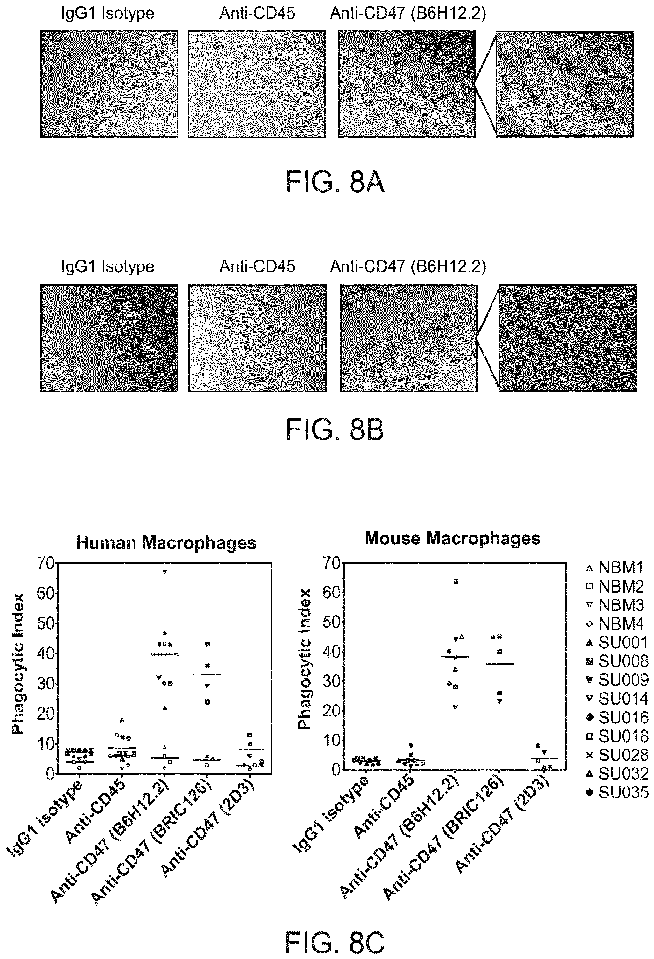

Figure 8A-C. Monoclonal Antibodies Directed Against Human CD47 Preferentially Enable Phagocytosis of Human AML LSC by Human and Mouse Macrophages. A,B. CFSE-labeled AML LSC were incubated with human peripheral blood-derived macrophages (A) or mouse bone marrow-derived macrophages (B) in the presence of IgG1 isotype control, anti-CD45 IgG1, or anti-CD47 (B6H12.2) IgG1 antibody. These cells were assessed by immunofluorescence microscopy for the presence of fluorescently labeled LSC within the macrophages (indicated by arrows). C. CFSE-labeled AML LSC or normal bone marrow CD34+ cells were incubated with human (left) or mouse (right) macrophages in the presence of the indicated antibodies and then assessed for phagocytosis by immunofluorescence microscopy. The phagocytic index was determined for each condition by calculating the number of ingested cells per 100 macrophages. For AML LSC, the differences between isotype or anti-CD45 antibody with blocking anti-CD47 antibody treatment (B6H12.2 and BRIC126) were statistically significant with p<0.001 for all pairwise comparisons with human and mouse macrophages. For human macrophages, the differences between AML LSC and normal CD34⁺ cells were statistically significant for B6H12.2 (p<0.001) and BRIC126 (p=0.002).

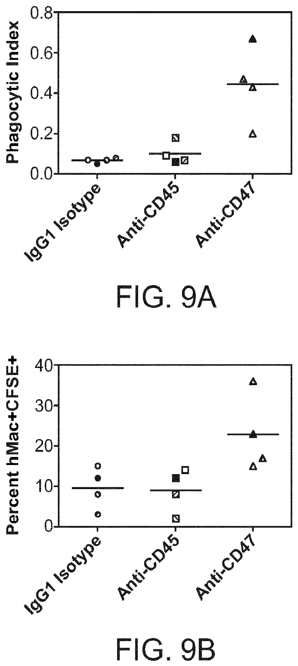

Figure 9. Anti-CD47 Antibody stimulates *in vitro* macrophage phagocytosis of primary human AML LSC. AML LSC were purified by FACS from four primary human AML samples, labeled with the fluorescent dye CFSE, and incubated with human peripheral blood macrophages either in the presence of an isotype control, isotype matched anti-CD45, or anti-CD47 antibody. (A) These cells were assessed by immunofluorescence microscopy for the presence of fluorescently-labeled LSC within the macrophages. The phagocytic index was determined for each condition by calculating the number of ingested cells per 100 macrophages. (B) The macrophages were harvested, stained with a fluorescently labeled anti-human macrophage antibody, and analyzed by flow cytometry. hMac+CFSE+ double positive events identify macrophages that have phagocytosed CFSE-labeled LSC. Each sample is represented by a different color.

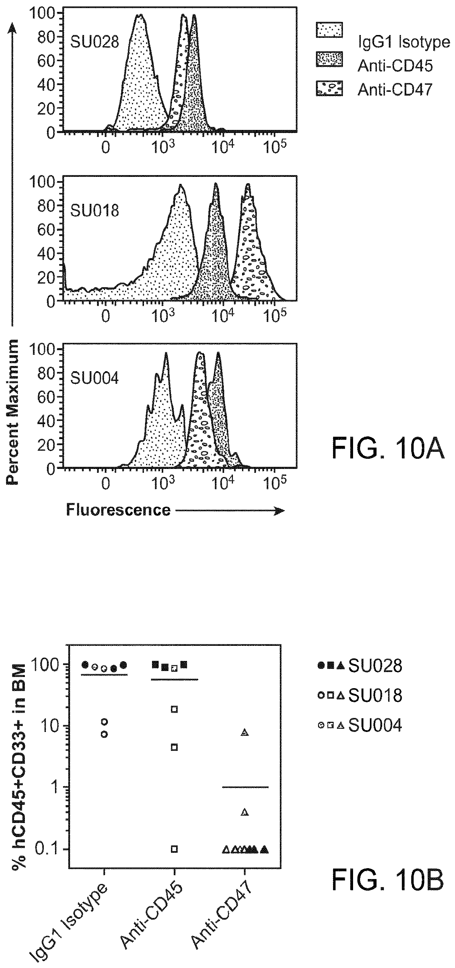

Figure 10A-B: *A Monoclonal Antibody Directed Against Human CD47 Inhibits AML LSC Engraftment In Vivo.* Three primary human AML samples were incubated with IgG1 isotype control, anti-CD45 IgG1, or anti-CD47 IgG1 antibody (B6H12.2) prior to transplantation into newborn NOG mice. A portion of the cells was analyzed for coating by staining with a secondary anti-mouse IgG antibody and analyzed by flow cytometry (A). 13 weeks later, mice were sacrificed and the bone marrow was analyzed for the percentage of human CD45+CD33+ myeloid leukemia cells by flow cytometry (B). The difference in mean engraftment between anti-CD47-coated cells and both isotype (p<0.001) and anti-CD45 (p=0.003) coated cells was statistically significant.

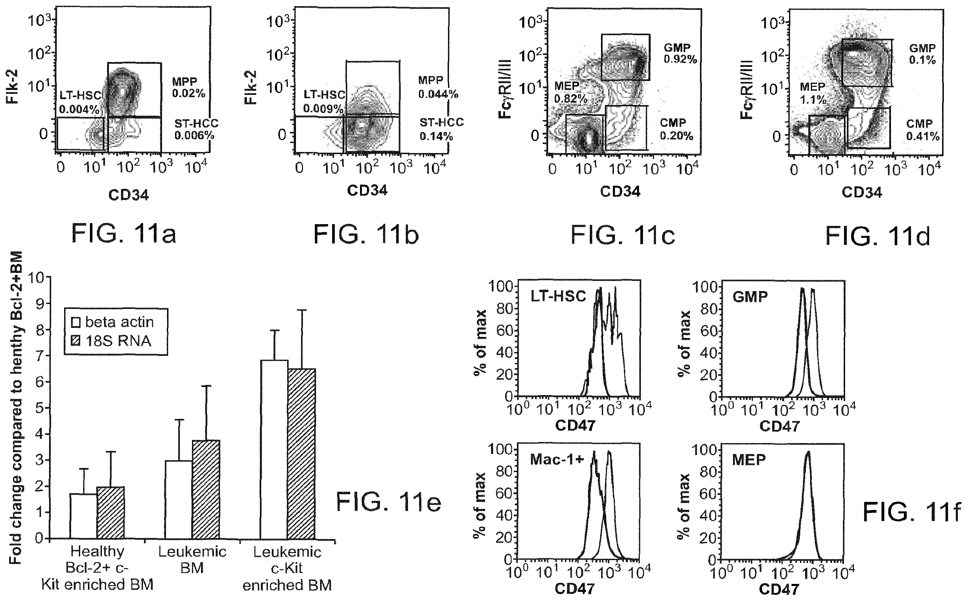

Figure 11. CD47 is upregulated in murine acute myeloid leukemia. Typical stem and progenitor plots are shown for leukemic hMRP8*bcrabl* x hMRP8*bcl2* cells compared to control non-leukemic animals. Lin- c-Kit+ Sca-1+ gated cells from control bone marrow **(a)** and leukemic spleen **(b)** and Lin- c-Kit+ Sca-1- gated cells from control bone marrow **(c)** and leukemic spleen **(d)** demonstrate perturberances in normal hematopoiesis in leukemic mice. Frequency is shown as a percentage of entire marrow or spleen mononuclear fraction. **(e)** Quantitative RT-PCR shows that CD47 is upregulated in leukemic BM cells. Data are shown from 3 sets of mice transplanted with either leukemic or control hRMP8*bcrabl* x hMRP8*bcl2* BM cells and then sacrificed 2-6 weeks later. Results were normalized to beta-actin and 18S rRNA expression. Fold change relative to control transplanted whole Bcl-2+ BM cells was determined. Error bars represent 1 s.d. **(f)** Histograms show expression of CD47 on gated populations for leukemic (gray) and control (black) mice.

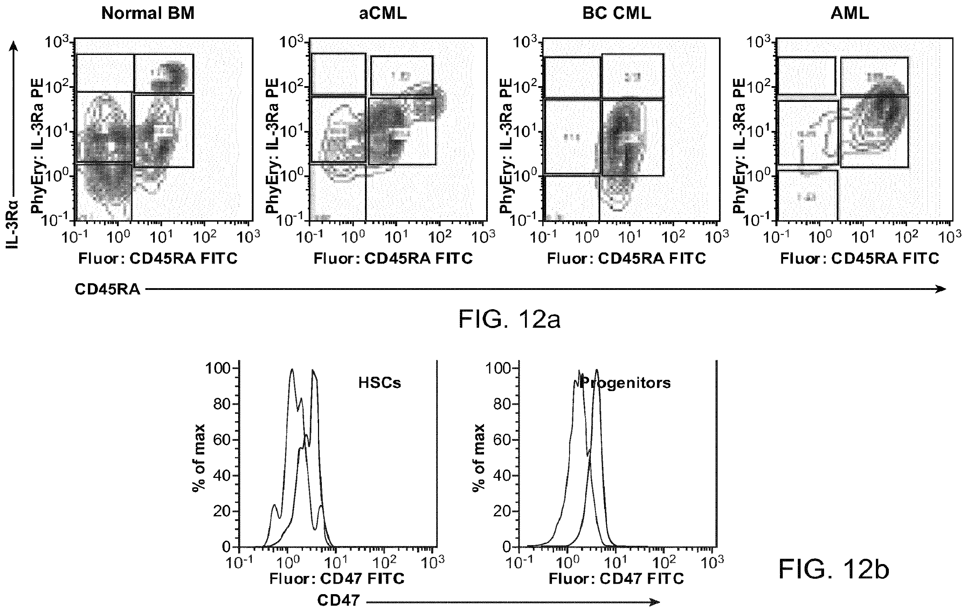

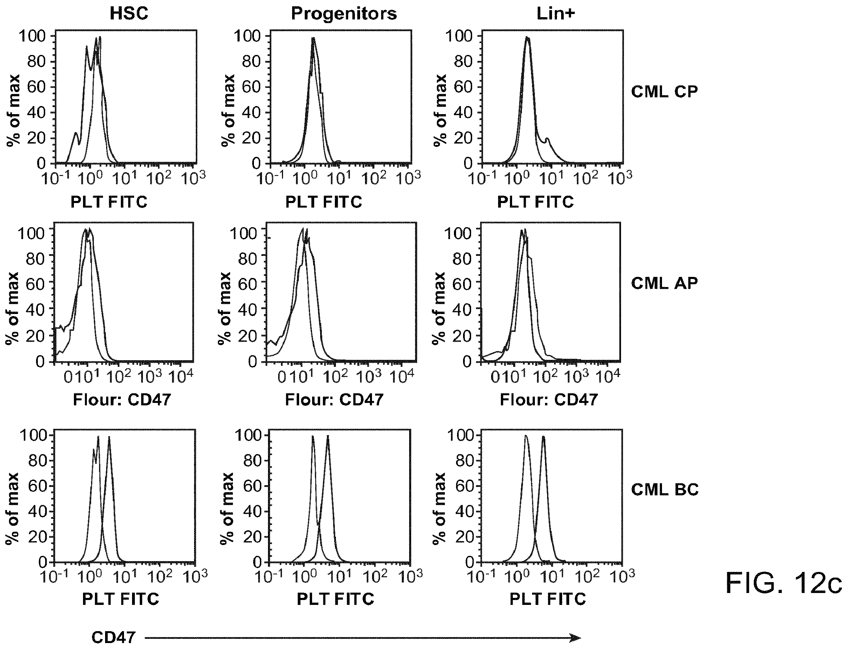

Figure 12. GMP expansion and CD47 upregulation in human myeloid leukemia. **a)** Representative FACS plots of myeloid progenitors (CD34+CD38+Lin-) including common myeloid progenitors (CMP), megakaryocyte-erythroid progenitors (MEP) and granulocyte-macrophage progenitors (GMP) in normal bone marrow (BM) versus aCML, BC CML and AML. b) Comparative FACS histograms of CD47 expression by normal (red; n=6) and acute myelogenous leukemic (AML, blue; n=6) hematopoietic stem cells (HSC; CD34+CD38-CD90+Lin-) and progenitors (CD34+CD38+Lin-). **c)** Comparative FACS histograms of CD47 expression by normal (red) and chronic myelogenous leukemia hematopoietic stem cells (HSC; CD34+CD38-CD90+Lin) and committed progenitors (CD34+CD38+Lin-). Upper panel: Normal (n=7) versus chronic phase CML (n=4) HSC, progenitors and lineage positive cells. Middle panel: Normal (n=7) versus accelerated phase CML (n=7) HSC, progenitors and lineage positive cells. Lower panel: Normal (n=7) versus blast crisis CML (n=4) HSC, progenitors and lineage positive cells.

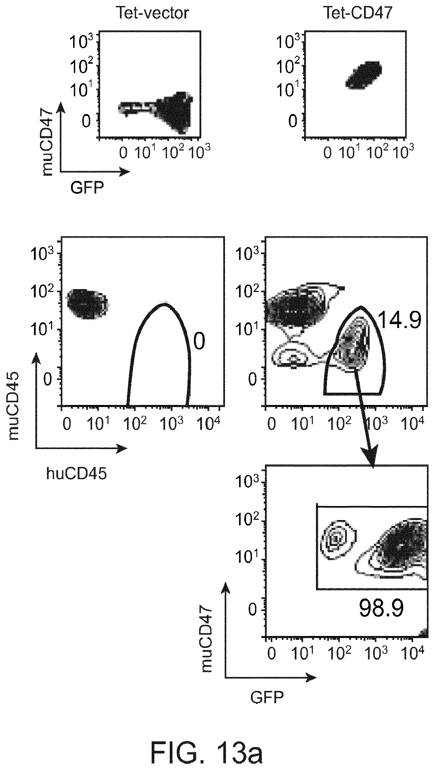

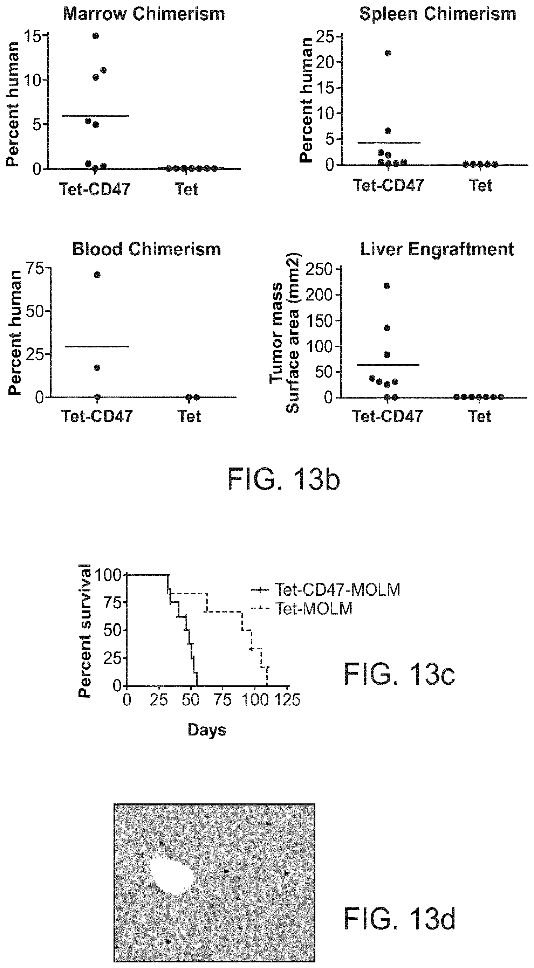

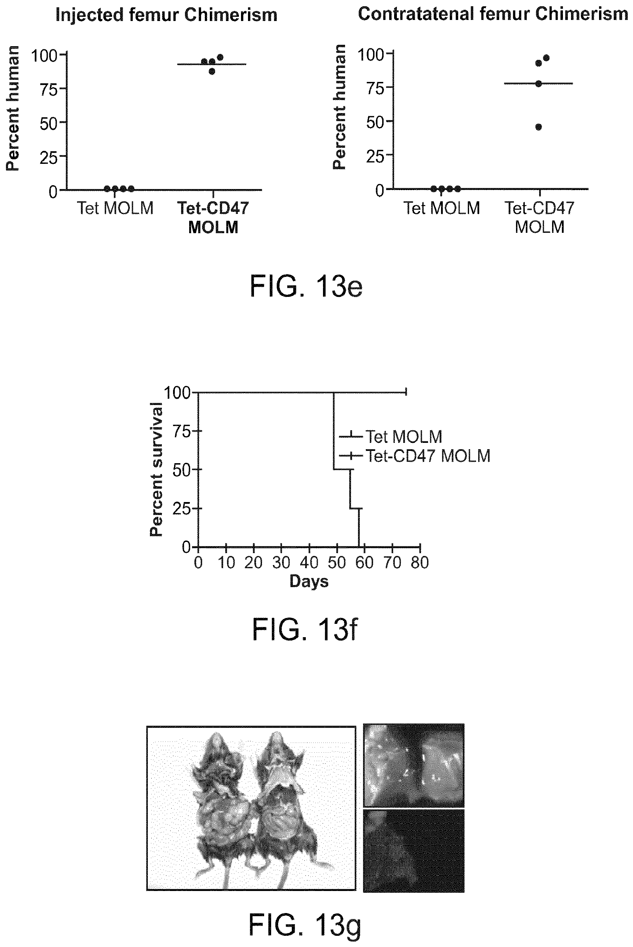

Figure 13. Over-expression of murine CD47 increases tumorigenicity of MOLM-13 cells. **a)** MOLM-13 cells were transduced with either control virus or virus expressing murine CD47 cDNA form 2. The resulting cell lines, termed Tet or Tet-CD47, were transplanted competitively into RAG/common gamma chain deficient mice with untransduced MOLM-13 cells (5x10⁵ Tet (n=6) or Tet-47 (n=8) cells with 5x10⁵ MOLM-13). Mice were analyzed for GFP and human CD45 chimerism when moribund. **b)** MOLM-13 chimerism in hematopoietic tissues was determined by human CD45 chimerism and measurement of tumor lesion size. **c)** Survival of mice competitively transplanted with MOLM-13 plus Tet or Tet-CD47 MOLM-13 cells was plotted. Control mice died of large tumor burden at the site of injection but had no engraftment in hematopoietic tissues. **d)** Hematoxylin and eosin sections of Tet-CD47 MOLM-13 transplanted liver (200x). Periportal (arrow) and sinusoidal (arrowhead) tumor infiltration is evident. **e)** 1x10⁶ Tet (n=5) or Tet-CD47 MOLM-13 (n=4) cells were injected into the right femur of RAG2-/-, Gc-/- mice and the tissues were analyzed 50-75 days later and chimerism of MOLM-13 cells in bone marrow was determined. **f)** Survival curve of mice transplanted intrafemorally with Tet or Tet-CD47 MOLM-13 cells. **g)** Examples of liver tumor formation and hepatomegaly in Tet-CD47 MOLM-13 transplanted mice versus control transplanted mice. GFP fluorescence demonstrates tumor nodule formation as well diffuse infiltration.

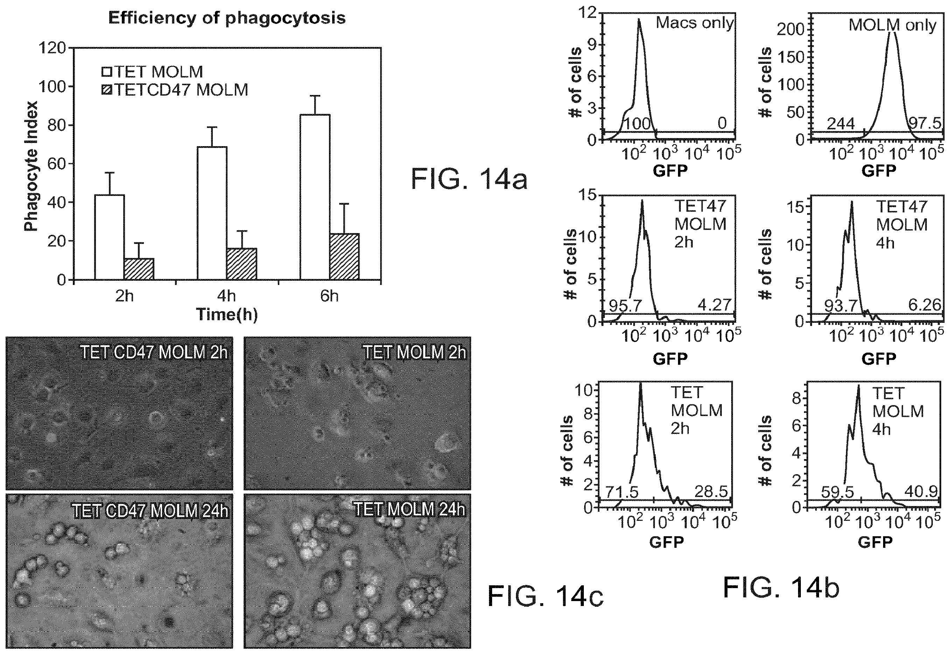

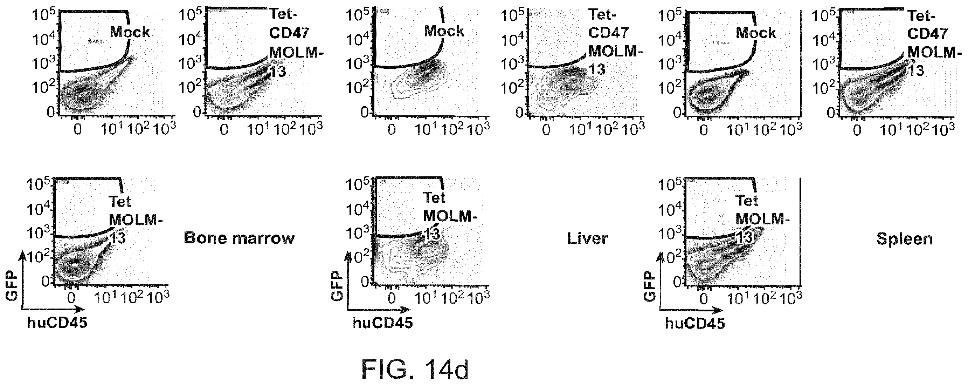

Figure 14. CD47 over-expression prevents phagocytosis of live unopsonized MOLM-13 cells. **a)** Tet or Tet-CD47 MOLM-13 cells were incubated with bone marrow derived macrophages (BMDM) for 2, 4, or 6 hours and phagocytic index was determined. Error bars represent 1 s.d. (n=6 for each time point). **b)** FACS analysis of BMDMs incubated with either Tet or Tet-CD47 cells. **c)** Photomicrographs of BMDMs incubated with Tet or Tet-CD47 MOLM-13 cells at 2 and 24 hours (400X). **d)** Tet or Tet-CD47 MOLM-13 cells were transplanted into RAG2-/-, Gc-/- mice and marrow, spleen, and liver macrophages were analyzed 2 hours later. GFP+ fraction of macrophages are gated. Results are representative of 3 experiments.

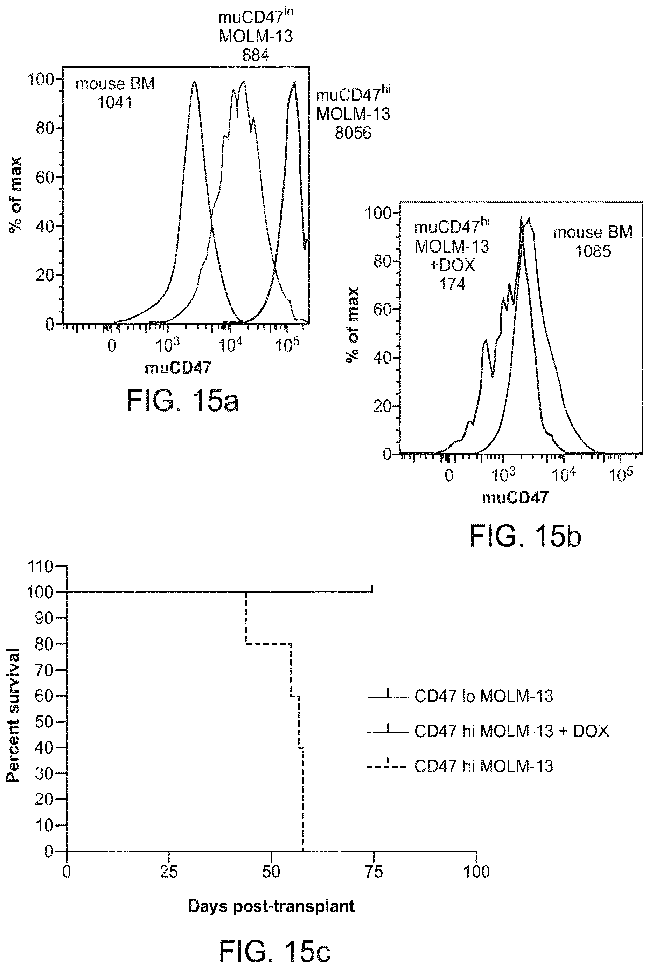

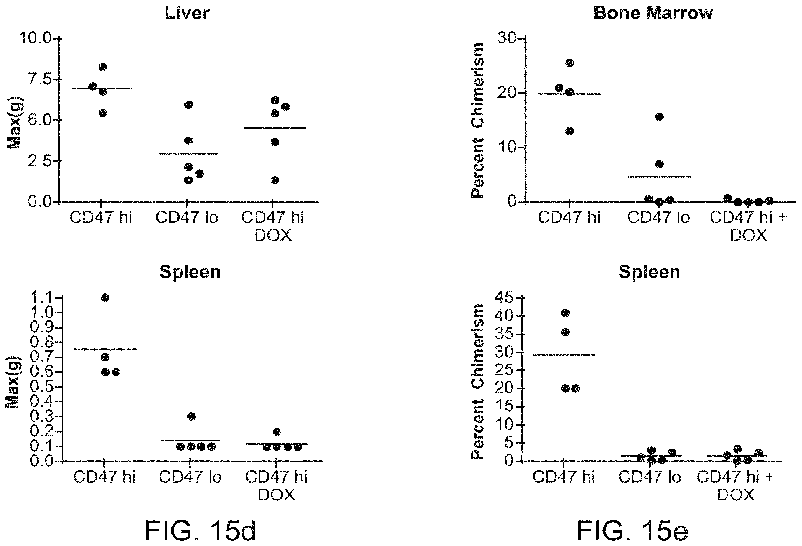

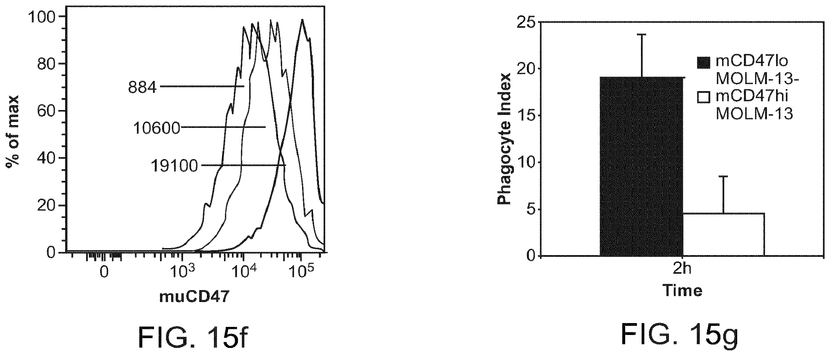

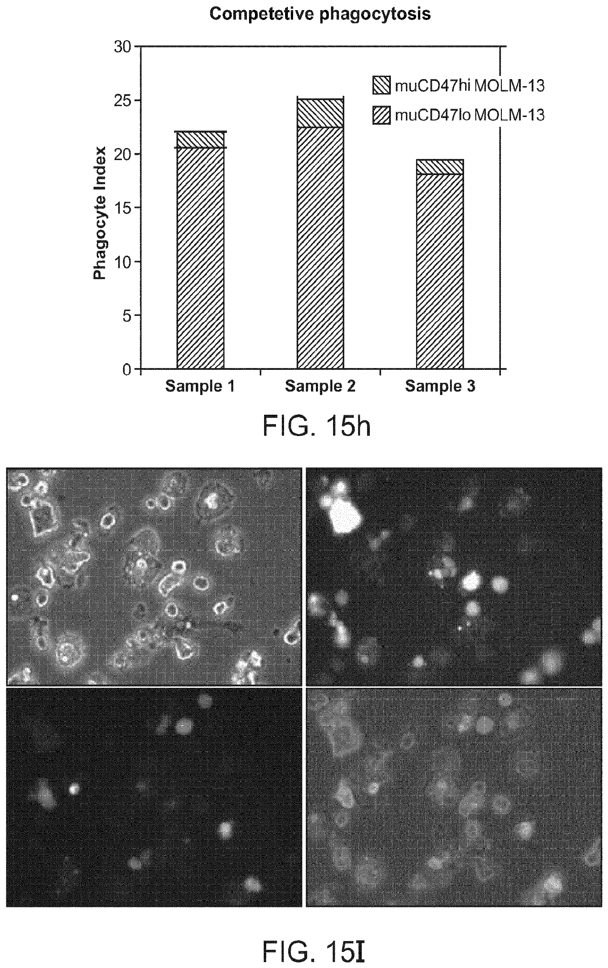

Figure 15. Higher expression of CD47 on MOLM-13 cells correlates with tumorigenic potential and evasion of phagocytosis. **a)** Tet-CD47 MOLM-13 cells were divided into high and low expressing clones as described. Histograms show CD47 expression in MOLM-13 high (black), MOLM-13 low (gray), and mouse bone marrow (shaded) cells. Value obtained for MFI/FSC² (x10⁹) are shown. **b)** Mice transplanted with CD47hi MOLM-13 cells were given doxycycline for 2 weeks. The histograms show level of CD47 expression in untreated (shaded) and treated (shaded) mice, with the values of MFI/FSC² (x10⁹) indicated. **c)** Survival of RAG2-/-,Gc-/- mice transplanted with 1 x 10⁶ CD47^{hi}, CD47^{lo} MOLM-13 cells, or CD47^{hi} MOLM-13 cells with doxycycline administration after 2 weeks post-transplant. **d)** Liver and spleen size of mice at necropsy or 75 days after transplant with 1 x 10⁶ CD47^{hi}, CD47^{lo} MOLM-13 cells, or CD47^{hi} MOLM-13 cells with doxycycline administration after 2 weeks post-transplant. **e)** Bone marrow and spleen chimerism of human cells in mice at necropsy or 75 days after transplant with 1 x 10⁶ CD47^{hi}, CD47^{lo} MOLM-13 cells, or CD47^{loi} MOLM-13 cells with doxycycline administration after 2 weeks post-transplant. **f)** Murine CD47 expression on CD47¹⁰ MOLM-13 cells engrafting in bone marrow (open) compared with original cell line (shaded). The values of MFI/FSC² (x10⁹) are indicated. **g)** 2.5 x 10⁵ CD47^{hi} or CD47^{lo} MOLM-13 cells were incubated with 5 x 10⁴ BMDMs for 2 hours. Phagocytic index is shown. **h)** 2.5 x 10⁵ CD47^{hi} RFP and CD47¹⁰ MOLM-13 GFP cells were incubated with 5 x 10⁴ BMDMs for 2 hours. Phagocytic index is shown for three separate samples for CD47^{hi} RFP (red) and CD47¹⁰ MOLM-13 GFP (green) cells. i) 2.5 x 10⁵ CD47^{hi} RFP and CD47^{lo} MOLM-13 GFP cells were incubated with 5 x 10⁴ BMDMs for 24 hours. Photomicrographs show brightfield (top left), RFP (top right), GFP (bottom left), and merged (bottom right) images.

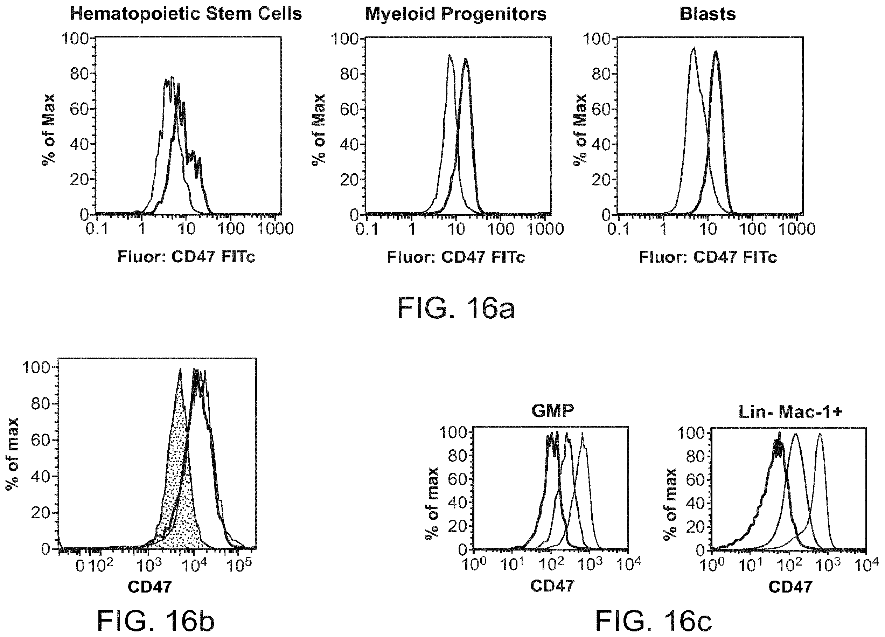

Figure 16. **a)** FACS analysis of CD47 expression of non-leukemic Fas Ipr/lpr hMRP8bcl-2 (blue) and leukemic Fas Ipr/lpr hMRP8bcl-2 (green) bone marrow hematopoietic stem cells (c-kit+Sca+Lin-), myeloid progenitors (c-kit+Sca-Lin-) or blasts (c-kit lo Sca-Lin-). **b)** Mouse bone marrow was transduced with retrovirus containing p210 bcr/abl as previously described²⁴. Mice were sacrificed when moribund and the spleens were analyzed. Expression of CD47 in c-Kit+ Mac-1+ cells in the spleens of two leukemic mice (unshaded histograms) and bone marrow from a wild-type mouse (shaded histogram) are shown. **c)** Histograms show expression of CD47 on gated populations for leukemic hMRP8bcrabl x hMRP8bcl2 mice (red), hMRP8bcl2 non-leukemic (blue) and wild-type (green) mice. CD47 was stained using FITC conjugated anti-mouse CD47 (Pharmingen).

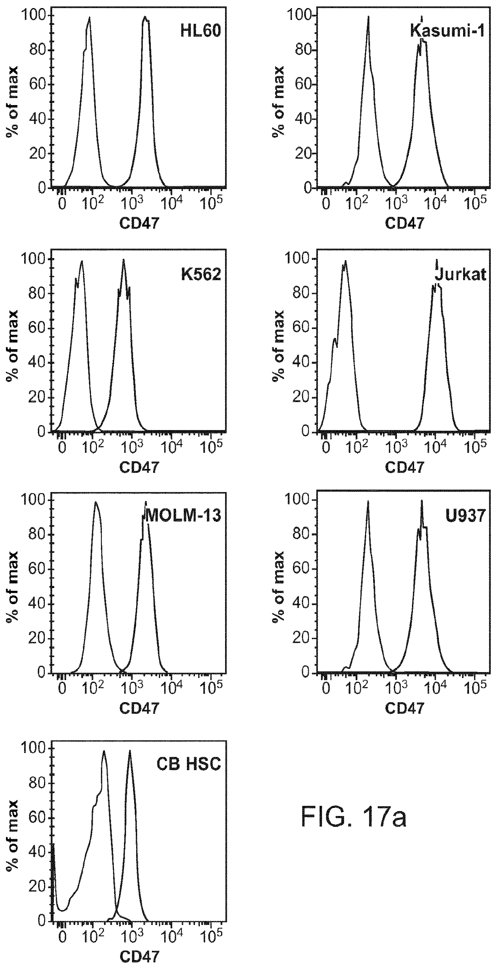

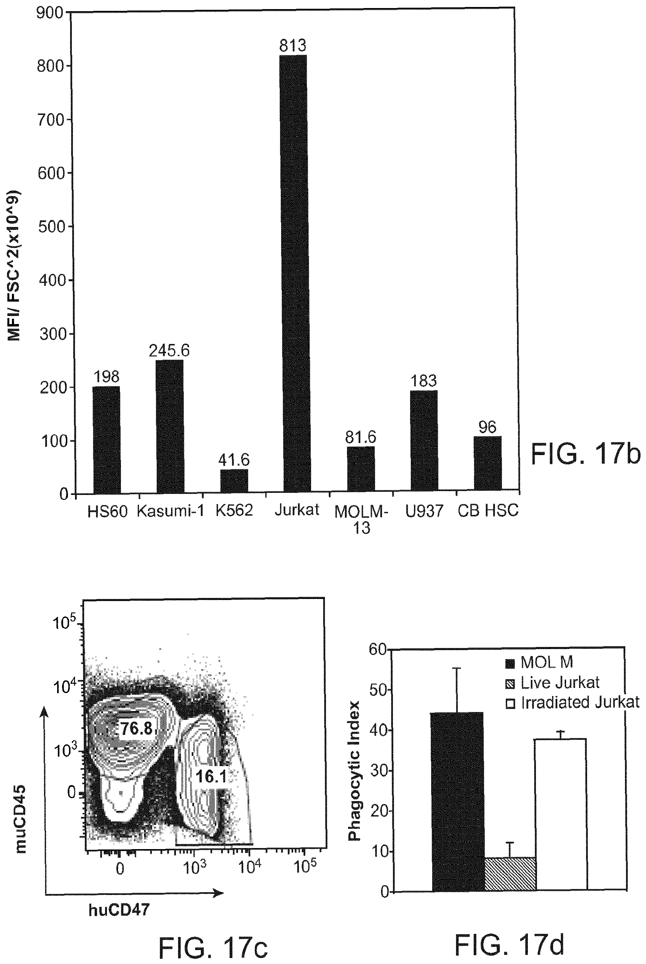

Figure 17. **a)** Expression of human CD47 (black histograms) on human leukemia cell lines and cord blood HSCs is shown. Isotype control staining is shown in gray. **b)** CD47 MFI over background was normalized to cell size by dividing by FSC². The value obtained for each cell type is shown above the bar. **c)** HL-60 cells engraft mouse bone marrow. 5 x 10⁵ cells were injected intravenously into RAG2-/-, Gc-/- animals and mice were analyzed 4 weeks later. **d)** Cells were stained with CFSE and co-cultured with BMDM. Phagocytic events were counted after 2h. For irradiation, Jurkat cells were given a dose of 2 Gray and incubated for 16h prior to the phagocytosis assay.

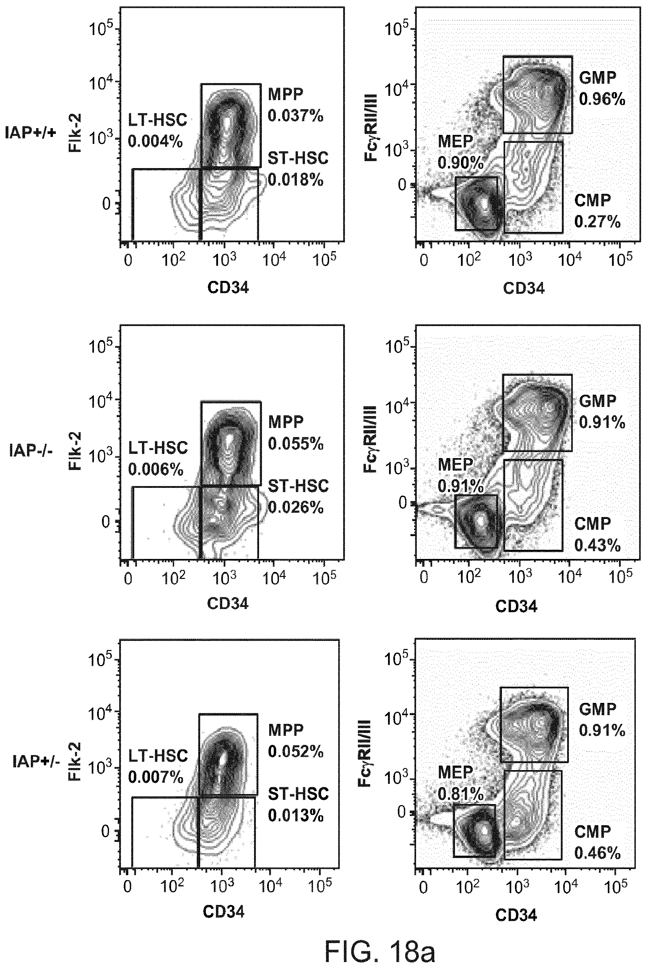

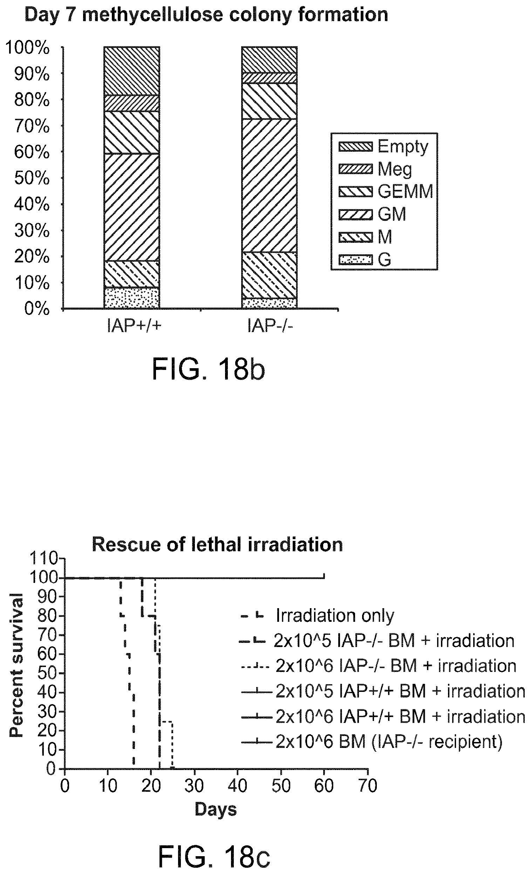

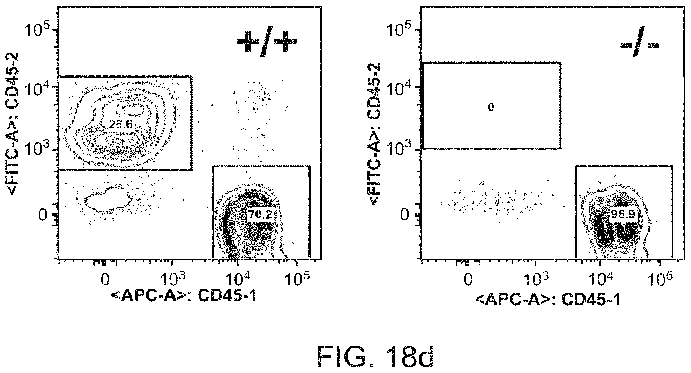

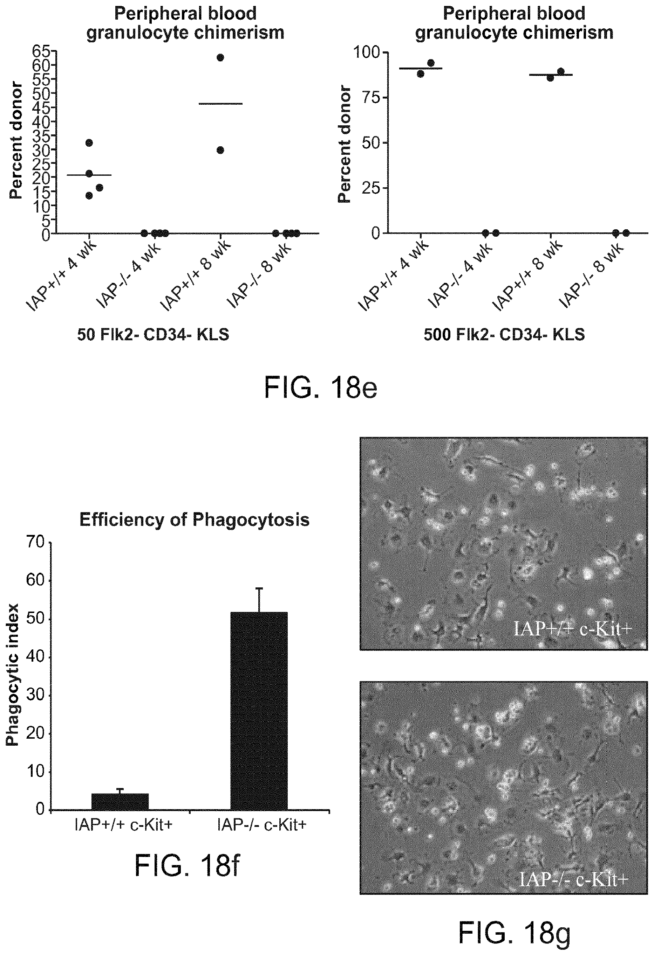

Figure 18. (a) Analysis of stem and progenitor cells from bone marrow of IAP+/+, IAP+/-, and IAP-/- mice. Stem cells (left) are gated on lineage- c-Kit+ Sca-1+ cells. Myeloid progenitors (right) are gated on lineage- c-Kit+ Sca-1+ cells. Frequency in whole bone marrow is shown adjacent to each gated population. (b) Colony output on day 7 of individually sorted LT-HSC. G-granulocyte, M-macrophage, GM-granulocyte and macrophage, GEMM-granulocyte, macrophage, erythroid, and megakaryocyte, Meg-megakaryocyte. (c) Survival curve of recipient mice given a radiation dose of 9.5 Gray and transplanted with the cells shown. Radiation control mice all died within 12-15 days. n=5 for each group. (d) Examples of CD45.1/CD45.2 chimerism plots at 4 weeks post-transplant. CD45.1 mice were transplanted with 50 LT-HSC (CD45.2) and 2 x 10⁵ CD45.1 helper marrow. Cells are gated on B220- CD3- Mac-1 + side scatter mid/hi cells. IAP-/- cells fail to engraft. (e) Summary of chimerism analysis of mice transplanted with either 50 or 500 IAP+/+ or IAP-/- cells. (f) IAP+/+ or IAP-/- c-Kit enriched cells were incubated with wild-type BMDM. Results indicate mean phagocytic index calculated from three separate samples. Error bars represent 1 s.d. (g) Photomicrographs of phagocytosis assays taken after 2 hours. Genotype of the -Kit enriched cells is shown.

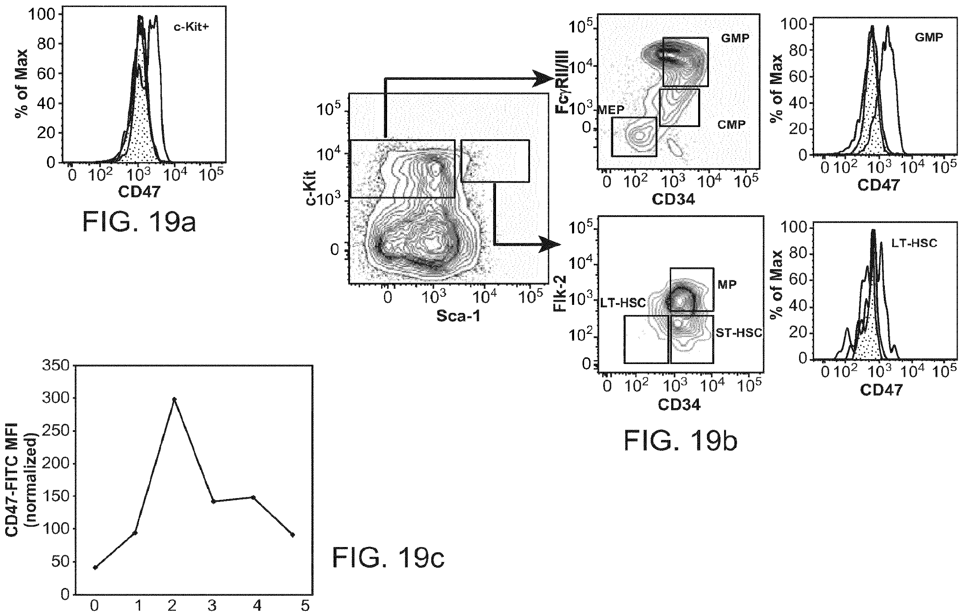

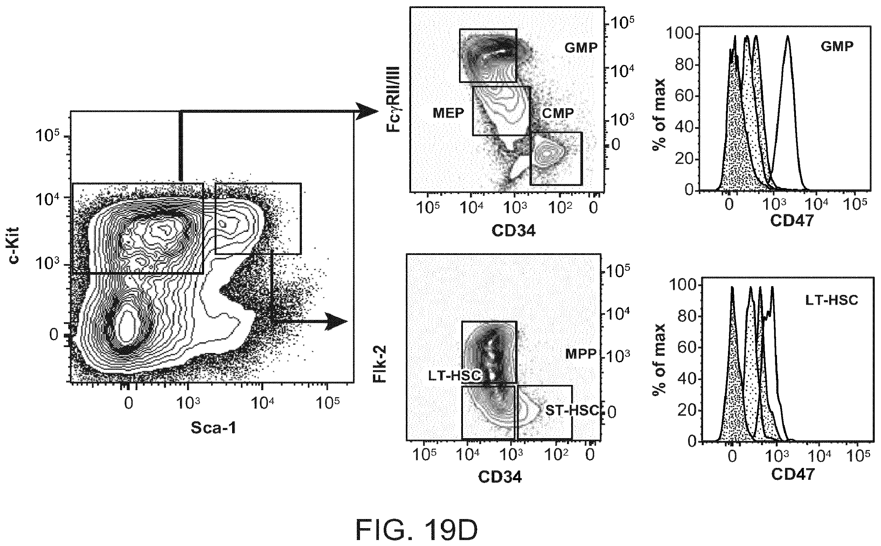

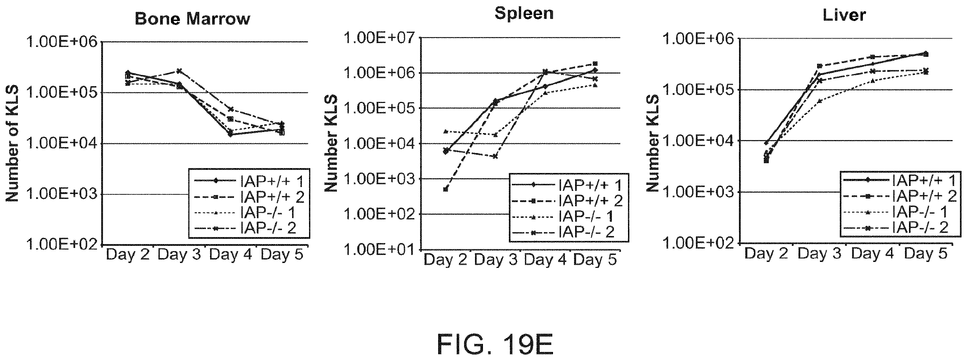

Figure 19. (a) Mice were mobilized with Cy/G and bone marrow was analyzed on day 2. Expression level of CD47 on c-Kit+ cells is shown. (b) Myeloid progenitor and stem cell gates are shown for day 2 mobilized bone marrow. Histograms on left show level of CD47 expression in marrow LT-HSC and GMP for steady-state (shaded histogram), day 2 mobilized (black line), and day 5 mobilized (gray line). (c) Relative MFI of CD47 for GMP on days 0-5 of Cy/G mobilization. Results were normalized so that steady state GMP were equal to 100. (d) Myeloid progenitor and stem cell gates are shown for day 2 bone marrow post-LPS treatment. Histograms show level of CD47 expression on day 2 post-LPS (black line), day 5 post-LPS (dark gray shaded histogram), steady state (light gray shaded histogram), and IAP-/- (black shaded histogram) LT-HSC and GMP. (e) Evaluation of KLS cells in the hematopoietic organs of IAP+/+ and IAP-/- mice mobilized on days 2 through 5. Two mice are analyzed per genotype per day.

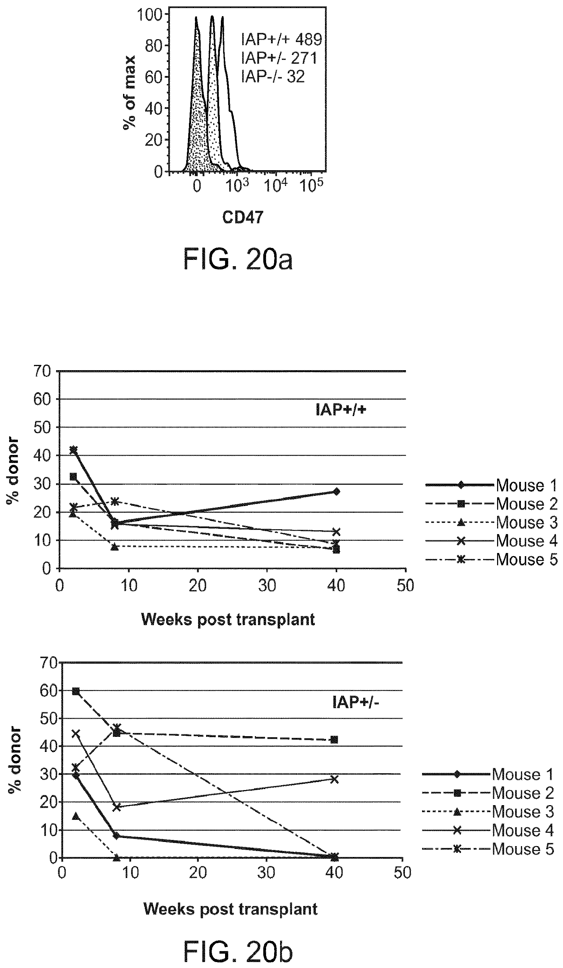

Figure 20. (a) CD47 expression level of IAP+/+, IAP+/-, and IAP-/- LT-HSC. The numbers shown are the MFI for each group. (b) Donor chimerism analysis for transplants of IAP+/+ (top) or IAP+/- (bottom) mice. Mice were bled at 2, 8, and 40 weeks post transplant. 2 x 10⁶ donor cells were transplanted into sub-lethally irradiated congenic recipients.

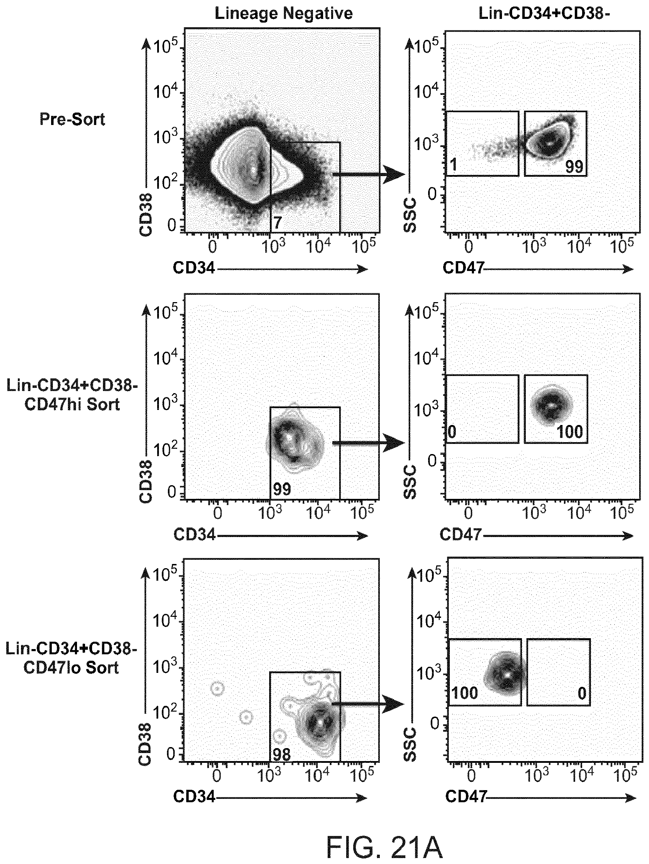

Figure 21A-D: Identification and Separation of Normal and Leukemic Progenitors From the Same Patient Based On Differential CD47 Expression. A. CD47 expression on the Lin-CD34+CD38- LSC-enriched fraction of specimen SU008 was determined by flow cytometry. CD47hi- and CD47lo-expressing cells were identified and purified using FACS. The left panels are gated on lineage negative cells, while the right panels are gated on Lin-CD34+CD38- cells. B. Lin-CD34+CD38-CD47lo and Lin-CD34+CD38-CD47hi cells were plated into complete methylcellulose, capable of supporting the growth of all myeloid colonies. 14 days later, myeloid colony formation was determined by morphologic assessment. Representative CFU-G/M (left) and BFU-E (right) are presented. C. Lin-CD34+CD38-CD47lo cells were transplanted into 2 newborn NOG mice. 12 weeks later, the mice were sacrificed and the bone marrow was analyzed for the presence of human CD45+CD33+ myeloid cells and human CD45+CD19+ lymphoid cells by flow cytometry. D. Normal bone marrow HSC, bulk SU008 leukemia cells, Lin-CD34+CD38-CD47hi cells, Lin-CD34+CD38-CD47lo cells, or human CD45+ cells purified from the bone marrow of mice engrafted with Lin-CD34+CD38-CD47lo cells were assessed for the presence of the FLT3-ITD mutation by PCR. The wild type FLT3 and the FLT3-ITD products are indicated.

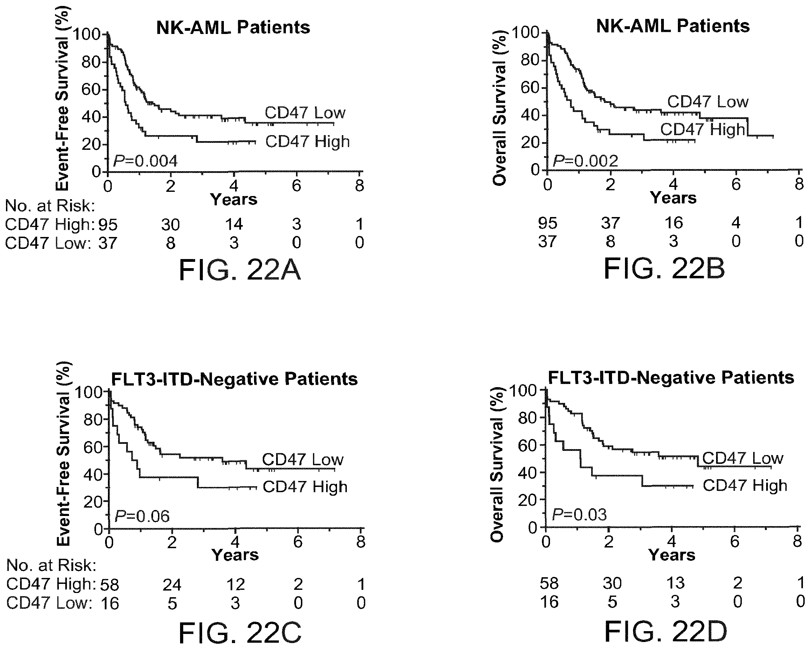

Figure 22: Increased CD47 Expression in Human AML is Associated with Poor Clinical Outcomes. Event-free (A,C) and overall (B,D) survival of 132 AML patients with normal cytogenetics (A,B) and the subset of 74 patients without the FLT3-ITD mutation (C,D). Patients were stratified into low CD47 and high CD47 expression groups based on an optimal threshold (28% high, 72% low) determined by microarray analysis from an independent training data set. The significance measures are based on log-likelihood estimates of the p-value, when treating the model with CD47 expression as a binary classification.

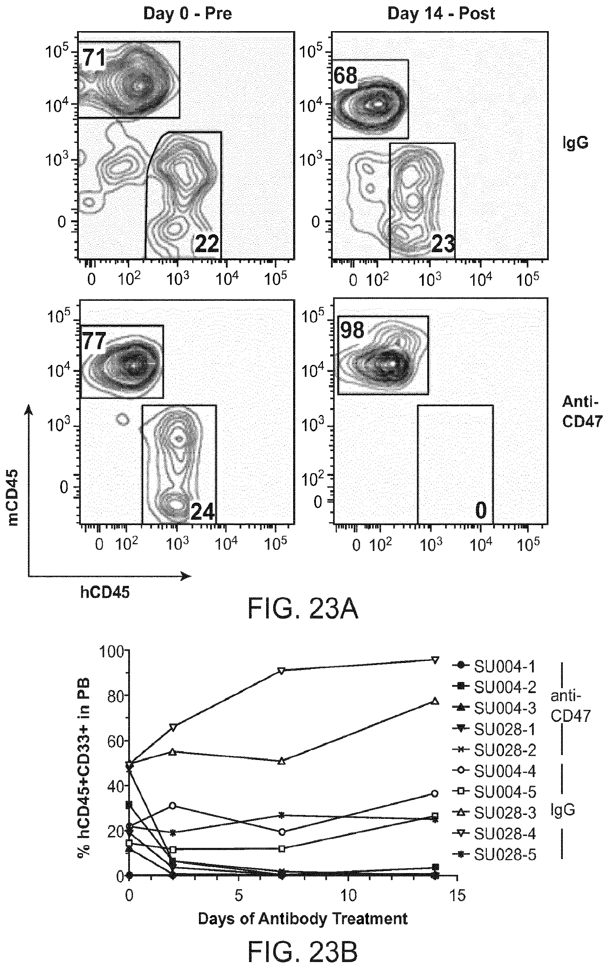

Figure 23A-E: A Monoclonal Antibody Directed Against Human CD47 Eliminates AML In Vivo. Newborn NOG mice were transplanted with AML LSC, and 8-12 weeks later, peripheral blood (A,B) and bone marrow (C-E) were analyzed for baseline engraftment prior to treatment with anti-CD47 (B6H12.2) or control IgG antibody (Day 0). Mice were treated with daily 100 microgram intraperitoneal injections for 14 days, at the end of which, they were sacrificed and peripheral blood and bone marrow were analyzed for the percentage of human CD45+CD33+ leukemia. A. Pre- and post-treatment human leukemic chimerism in the peripheral blood from representative anti-CD47 antibody and control IgG-treated mice as determined by flow cytometry. B. Summary of human leukemic chimerism in the peripheral blood assessed on multiple days during the course of treatment demonstrated elimination of leukemia in anti-CD47 antibody treated mice compared to control IgG treatment (p=0.007). C. Pre- and post-treatment human leukemic chimerism in the bone marrow from representative anti-CD47 antibody or control IgG-treated mice as determined by flow cytometry. D. Summary of human leukemic chimerism in the bone marrow on day 14 relative to day 0 demonstrated a dramatic reduction in leukemic burden in anti-CD47 antibody treated mice compared to control IgG treatment (p<0.001). E. H&E sections of representative mouse bone marrow cavities from mice engrafted with SU004 post-treatment with either control IgG (panels 1,2) or anti-CD47 antibody (panels 4,5). IgG-treated marrows were packed with monomorphic leukemic blasts, while anti-CD47-treated marrows were hypocellular, demonstrating elimination of the human leukemia. In some anti-CD47 antibody-treated mice that contained residual leukemia, macrophages were detected containing phagocytosed pyknotic cells, capturing the elimination of human leukemia (panels 3,6 arrows).

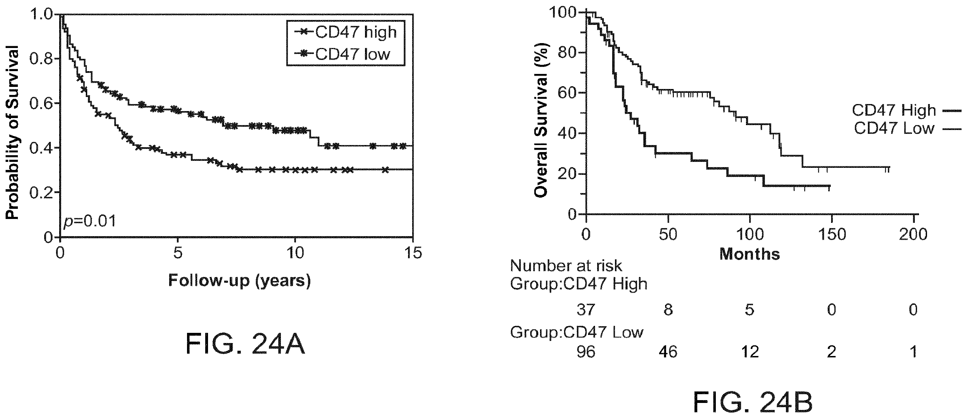

Figure 24. Increased CD47 expression predicts worse overall survival in DLBCL and ovarian cancer. (A) A cohort of 230 patients with diffuse large B-cell lymphoma (p=0.01). (B) A cohort of 133 patients with advanced stage (III/IV) ovarian carcinoma (p=0.04).

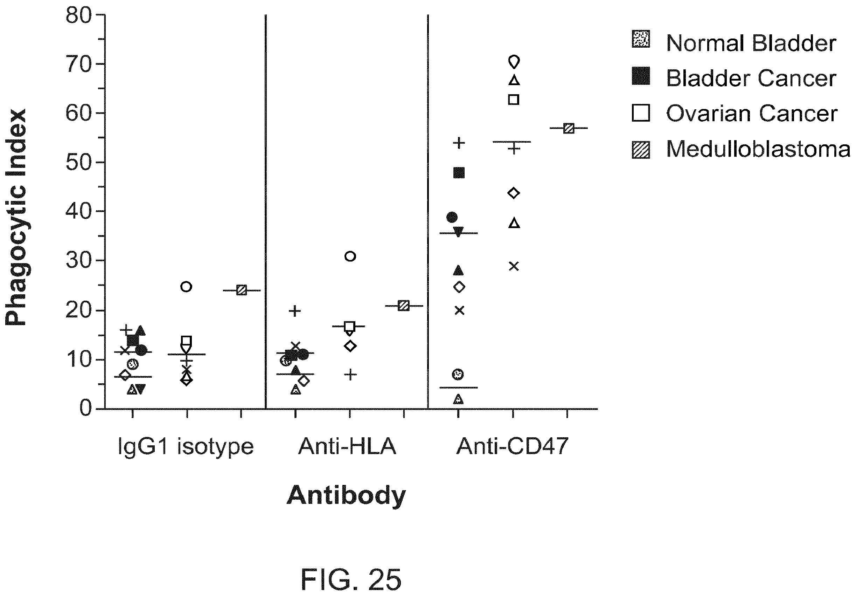

Figure 25: Anti-CD47 antibody enables the phagocytosis of solid tumor stem cells in vitro. The indicated cells were incubated with human macrophages in the presence of IgG1 isotype, anti-HLA, or anti-CD47 antibodies and the phagocytic index was determined by immunofluorescence microscopy. Statistics: Bladder cancer cells IgG1 isotype compared to anti-HLA (p=0.93) and anti-CD47 (p=0.01); normal bladder urothelium IgG1 isotype compared to anti-HLA (p=0.50) and anti-CD47 (p=0.13); ovarian cancer cells IgG1 isotype compared to anti-HLA (p=0.11) and anti-CD47 (p<0.001). Each individual data point represents a distinct tumor or normal tissue sample.

### DETAILED DESCRIPTION OF THE EMBODIMENTS

Disclosed herein are methods to manipulate hematopoietic cells, including circulating hematopoietic cells. In some instances, hematopoietic stem or progenitor cells are protected from phagocytosis in circulation by providing a host animal with a CD47 mimetic molecule, which interacts with SIRPα on phagocytic cells, such as, macrophages, and decreases phagocytosis. In other instances leukemia cells are targeted for phagocytosis by blocking CD47 on the cell surface. In other embodiments, cells of solid tumors are targeted for phagocytosis by blocking CD47 on the cell surface. In another instance, methods are provided for targeting or depleting AML cancer stem cells, the method comprising contacting reagent blood cells with an antibody that specifically binds CD47 in order to target or deplete AMLSC. In another instance, methods are provided for targeting cancer cells of a solid tumor in a human subject by administering an antibody against CD47 to the subject.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Methods recited herein may be carried out in any order of the recited events which is logically possible, as well as the recited order of events.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

CD47 polypeptides. The three transcript variants of human CD 47 (variant 1, NM 001777; variant 2, NM 198793; and variant 3, NM 001025079) encode three isoforms of CD47 polypeptide. CD47 isoform 1 (NP 001768), the longest of the three isoforms, is 323 amino acids long. CD47 isoform 2 (NP 942088) is 305 amino acid long. CD47 isoform 3 is 312 amino acids long. The three isoforms are identical in sequence in the first 303 amino acids. Amino acids 1-8 comprise the signal sequence, amino acids 9-142 comprise the CD47 immunoglobulin like domain, which is the soluble fragment, and amino acids 143-300 is the transmembrane domain.

"CD47 mimetics" include molecules that function similarly to CD47 by binding and activating SIRPα receptor. Molecules useful as CD47 mimetics include derivatives, variants, and biologically active fragments of naturally occurring CD47. A "variant" polypeptide means a biologically active polypeptide as defined below having less than 100% sequence identity with a native sequence polypeptide. Such variants include polypeptides wherein one or more amino acid residues are added at the N- or C-terminus of, or within, the native sequence; from about one to forty amino acid residues are deleted, and optionally substituted by one or more amino acid residues; and derivatives of the above polypeptides, wherein an amino acid residue has been covalently modified so that the resulting product has a non-naturally occurring amino acid. Ordinarily, a biologically active variant will have an amino acid sequence having at least about 90% amino acid sequence identity with a native sequence polypeptide, preferably at least about 95%, more preferably at least about 99%. The variant polypeptides can be naturally or non-naturally glycosylated, i.e., the polypeptide has a glycosylation pattern that differs from the glycosylation pattern found in the corresponding naturally occurring protein. The variant polypeptides can have post-translational modifications not found on the natural CD47 protein.

Fragments of the soluble CD47, particularly biologically active fragments and/or fragments corresponding to functional domains, are of interest. Fragments of interest will typically be at least about 10 aa to at least about 15 aa in length, usually at least about 50 aa in length, but will usually not exceed about 142 aa in length, where the fragment will have a stretch of amino acids that is identical to CD47. A fragment "at least 20 aa in length," for example, is intended to include 20 or more contiguous amino acids from, for example, the polypeptide encoded by a cDNA for CD47. In this context "about" includes the particularly recited value or a value larger or smaller by several (5, 4, 3, 2, or 1) amino acids. The protein variants described herein are encoded by polynucleotides that are within the scope of the disclosure. The genetic code can be used to select the appropriate codons to construct the corresponding variants. The polynucleotides may be used to produce polypeptides, and these polypeptides may be used to produce antibodies by known methods.

A "fusion" polypeptide is a polypeptide comprising a polypeptide or portion (e.g., one or more domains) thereof fused or bonded to heterologous polypeptide. A fusion soluble CD47 protein, for example, will share at least one biological property in common with a native sequence soluble CD47 polypeptide. Examples of fusion polypeptides include immunoadhesins, as described above, which combine a portion of the CD47 polypeptide with an immunoglobulin sequence, and epitope tagged polypeptides, which comprise a soluble CD47 polypeptide or portion thereof fused to a "tag polypeptide". The tag polypeptide has enough residues to provide an epitope against which an antibody can be made, yet is short enough such that it does not interfere with biological activity of the CD47 polypeptide. Suitable tag polypeptides generally have at least six amino acid residues and usually between about 6-60 amino acid residues.

A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. The term "derivative" encompasses both amino acid sequence variants of polypeptide and covalent modifications thereof. Derivatives and fusion of soluble CD47 find use as CD47 mimetic molecules.

The first 142 amino acids of CD47 polypeptide comprise the extracellular region of CD47 (SEQ ID NO: 1). The three isoforms have identical amino acid sequence in the extracellular region, and thus any of the isoforms are can be used to generate soluble CD47. "Soluble CD47" is a CD47 protein that lacks the transmembrane domain. Soluble CD47 is secreted out of the cell expressing it instead of being localized at the cell surface. Soluble CD47 may be fused to another polypeptide to provide for added functionality, e.g. to increase the in vivo stability. Generally such fusion partners are a stable plasma protein that is capable of extending the *in vivo* plasma half-life of soluble CD47 protein when present as a fusion, in particular wherein such a stable plasma protein is an immunoglobulin constant domain. In most cases where the stable plasma protein is normally found in a multimeric form, e.g., immunoglobulins or lipoproteins, in which the same or different polypeptide chains are normally disulfide and/or noncovalently bound to form an assembled multichain polypeptide. Soluble CD47 fused to human Ig G1 has been described (Motegi S. et al. EMBO J. 22(11): 2634-2644).

Stable plasma proteins are proteins typically having about from 30 to 2,000 residues, which exhibit in their native environment an extended half-life in the circulation, i.e. greater than about 20 hours. Examples of suitable stable plasma proteins are immunoglobulins, albumin, lipoproteins, apolipoproteins and transferrin. The extracellular region of CD47 is typically fused to the plasma protein at the N-terminus of the plasma protein or fragment thereof which is capable of conferring an extended half-life upon the soluble CD47. Increases of greater than about 100% on the plasma half-life of the soluble CD47 are satisfactory.

Ordinarily, the soluble CD47 is fused C-terminally to the N-terminus of the constant region of immunoglobulins in place of the variable region(s) thereof, however N-terminal fusions may also find use. Typically, such fusions retain at least functionally active hinge, CH2 and CH3 domains of the constant region of an immunoglobulin heavy chain, which heavy chains may include IgG1, IgG2a, IgG2b, IgG3, IgG4, IgA, IgM, IgE, and IgD, usually one or a combination of proteins in the IgG class. Fusions are also made to the C-terminus of the Fc portion of a constant domain, or immediately N-terminal to the CH1 of the heavy chain or the corresponding region of the light chain. This ordinarily is accomplished by constructing the appropriate DNA sequence and expressing it in recombinant cell culture. Alternatively, the polypeptides may be synthesized according to known methods.

The precise site at which the fusion is made is not critical; particular sites may be selected in order to optimize the biological activity, secretion or binding characteristics of CD47. The optimal site will be determined by routine experimentation.

In some embodiments the hybrid immunoglobulins are assembled as monomers, or hetero- or homo-multimers, and particularly as dimers or tetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of basic four-chain units held together by disulfide bonds. IgA immunoglobulin, and occasionally IgG immunoglobulin, may also exist in a multimeric form in serum. In the case of multimers, each four chain unit may be the same or different.

Suitable CD47 mimetics and/or fusion proteins may be identified by compound screening by detecting the ability of an agent to mimic the biological activity of CD47. One biological activity of CD47 is the activation of SIRPα receptor on macrophages. *In vitro* assays may be conducted as a first screen for efficacy of a candidate agent, and usually an *in vivo* assay will be performed to confirm the biological assay. Desirable agents are effective in temporarily blocking SIRP α receptor activation. Desirable agents are temporary in nature, *e.g.* due to biological degradation.

I*n vitro* assays for CD47 biological activity include, *e.g.* inhibition of phagocytosis of porcine cells by human macrophages, binding to SIRP α receptor, SIRP α□ tyrosine phosphorylation, *etc.* An exemplary assay for CD47 biological activity contacts a human macrophage composition in the presence of a candidate agent. The cells are incubated with the candidate agent for about 30 minutes and lysed. The cell lysate is mixed with anti-human SIRP α□ antibodies to immunoprecipitate SIRP α. Precipitated proteins are resolved by SDS PAGE, then transferred to nitrocellulose and probed with antibodies specific for phosphotyrosine. A candidate agent useful as CD47mimetic increases SIRP α tyrosine phosphorylation by at least 10%, or up to 20%, or 50%, or 70% or 80% or up to about 90% compared to the level of phosphorylation observed in the absence of candidate agent. Another exemplary assay for CD47 biological activity measures phagocytosis of hematopoietic cells by human macrophages. A candidate agent useful as a CD47 mimetic results in the down regulation of phagocytosis by at least about 10%, at least about 20%, at least about 50%, at least about 70%, at least about 80%, or up to about 90% compared to level of phagocytosis observed in absence of candidate agent.

Polynucleotide encoding soluble CD47 or soluble CD47-Fc can be introduced into a suitable expression vector. The expression vector is introduced into a suitable cell. Expression vectors generally have convenient restriction sites located near the promoter sequence to provide for the insertion of polynucleotide sequences. Transcription cassettes may be prepared comprising a transcription initiation region, CD47 gene or fragment thereof, and a transcriptional termination region. The transcription cassettes may be introduced into a variety of vectors, e.g. plasmid; retrovirus, e.g. lentivirus; adenovirus; and the like, where the vectors are able to transiently or stably be maintained in the cells, usually for a period of at least about one day, more usually for a period of at least about several days to several weeks.

The various manipulations may be carried out *in vitro* or may be performed in an appropriate host, e.g. E. coli. After each manipulation, the resulting construct may be cloned, the vector isolated, and the DNA screened or sequenced to ensure the correctness of the construct. The sequence may be screened by restriction analysis, sequencing, or the like.

Soluble CD47 can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, protein G affinity chromatography, for example, hydroxylapatite chromatography and lectin chromatography. Most preferably, high performance liquid chromatography ("HPLC") is employed for purification.

Soluble CD47 can also be recovered from: products of purified cells, whether directly isolated or cultured; products of chemical synthetic procedures; and products produced by recombinant techniques from a prokaryotic or eukaryotic host, including, for example, bacterial, yeast higher plant, insect, and mammalian cells.

A plurality of assays may be run in parallel with different concentrations to obtain a differential response to the various concentrations. As known in the art, determining the effective concentration of an agent typically uses a range of concentrations resulting from 1:10, or other log scale, dilutions. The concentrations may be further refined with a second series of dilutions, if necessary. Typically, one of these concentrations serves as a negative control, *i.e*. at zero concentration or below the level of detection of the agent or at or below the concentration of agent that does not give a detectable change in binding.

Compounds of interest for screening include biologically active agents of numerous chemical classes, primarily organic molecules, although including in some instances inorganic molecules, organometallic molecules, immunoglobulins, chimeric CD47 proteins, CD47 related proteins, genetic sequences, *etc.* Also of interest are small organic molecules, which comprise functional groups necessary for structural interaction with proteins, particularly hydrogen bonding, and typically include at least an amine, carbonyl, hydroxyl or carboxyl group, frequently at least two of the functional chemical groups. The candidate agents often comprise cyclical carbon or heterocyclic structures and/or aromatic or polyaromatic structures substituted with one or more of the above functional groups. Candidate agents are also found among biomolecules, including peptides, polynucleotides, saccharides, fatty acids, steroids, purines, pyrimidines, derivatives, structural analogs or combinations thereof.

Compounds are obtained from a wide variety of sources including libraries of synthetic or natural compounds. For example, numerous means are available for random and directed synthesis of a wide variety of organic compounds, including biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, *etc.* to produce structural analogs.

By "manipulating phagocytosis" is meant an up-regulation or a down-regulation in phagocytosis by at least about 10%, or up to 20%, or 50%, or 70% or 80% or up to about 90% compared to level of phagocytosis observed in absence of intervention. Thus in the context of decreasing phagocytosis of circulating hematopoietic cells, particularly in a transplantation context, manipulating phagocytosis means a down-regulation in phagocytosis by at least about 10%, or up to 20%, or 50%, or 70% or 80% or up to about 90% compared to level of phagocytosis observed in absence of intervention.

CD47 inhibitors. Agents of interest as CD47 inhibitors include specific binding members that prevent the binding of CD47 with SIRP α receptor. The term "specific binding member" or "binding member" as used herein refers to a member of a specific binding pair, i.e. two molecules, usually two different molecules, where one of the molecules (i.e., first specific binding member) through chemical or physical means specifically binds to the other molecule (i.e., second specific binding member). CD47 inhibitors useful in the methods of the disclosure include analogs, derivatives and fragments of the original specific binding member.

In a preferred embodiment, the specific binding member is an antibody. The term "antibody" or "antibody moiety" is intended to include any polypeptide chain-containing molecular structure with a specific shape that fits to and recognizes an epitope, where one or more non-covalent binding interactions stabilize the complex between the molecular structure and the epitope. Antibodies utilized in the present invention are monoclonal antibodies. Monoclonal antibodies may be reproduced by cell culture or recombinantly, and can be modified to reduce their antigenicity.

Polyclonal antibodies can be raised by a standard protocol by injecting a production animal with an antigenic composition. See, e.g., Harlow and Lane, Antibodies: A Laboratory Manual, Cold Spring Harbor Laboratory, 1988. When utilizing an entire protein, or a larger section of the protein, antibodies may be raised by immunizing the production animal with the protein and a suitable adjuvant (e.g., Freund's, Freund's complete, oil-in-water emulsions, etc.) When a smaller peptide is utilized, it is advantageous to conjugate the peptide with a larger molecule to make an immunostimulatory conjugate. Commonly utilized conjugate proteins that are commercially available for such use include bovine serum albumin (BSA) and keyhole limpet hemocyanin (KLH). In order to raise antibodies to particular epitopes, peptides derived from the full sequence may be utilized. Alternatively, in order to generate antibodies to relatively short peptide portions of the protein target, a superior immune response may be elicited if the polypeptide is joined to a carrier protein, such as ovalbumin, BSA or KLH. Alternatively, for monoclonal antibodies, hybridomas may be formed by isolating the stimulated immune cells, such as those from the spleen of the inoculated animal. These cells are then fused to immortalized cells, such as myeloma cells or transformed cells, which are capable of replicating indefinitely in cell culture, thereby producing an immortal, immunoglobulin-secreting cell line. In addition, the antibodies or antigen binding fragments may be produced by genetic engineering. Humanized, chimeric, or xenogeneic human antibodies, which produce less of an immune response when administered to humans, are preferred for use in the present invention.

In addition to entire immunoglobulins (or their recombinant counterparts), immunoglobulin fragments comprising the epitope binding site (e.g., Fab', F(ab')2, or other fragments) are useful as antibody moieties in the present invention. Such antibody fragments may be generated from whole immunoglobulins by ricin, pepsin, papain, or other protease cleavage. "Fragment," or minimal immunoglobulins may be designed utilizing recombinant immunoglobulin techniques. For instance "Fv" immunoglobulins for use in the present invention may be produced by linking a variable light chain region to a variable heavy chain region via a peptide linker (e.g., poly-glycine or another sequence which does not form an alpha helix or beta sheet motif).

The efficacy of a CD47 inhibitor is assessed by assaying CD47 activity. The above-mentioned assays or modified versions thereof are used. In an exemplary assay, AML SCs are incubated with bone marrow derived macrophages, in the presence or absence of the candidate agent. An inhibitor of the cell surface CD47 will up-regulate phagocytosis by at least about 10%, or up to 20%, or 50%, or 70% or 80% or up to about 90% compared to the phagocytosis in absence of the candidate agent. Similarly, an *in vitro* assay for levels of tyrosine phosphorylation of SIRPα will show a decrease in phosphorylation by at least about 10%, or up to 20%, or 50%, or 70% or 80% or up to about 90% compared to phosphorylation observed in absence of the candidate agent.

In one embodiment of the invention, the agent, or a pharmaceutical composition comprising the agent, is provided in an amount effective to detectably inhibit the binding of CD47 to SIRPα receptor present on the surface of phagocytic cells. The effective amount is determined via empirical testing routine in the art. The effective amount may vary depending on the number of cells being transplanted, site of transplantation and factors specific to the transplant recipient.

The terms "phagocytic cells" and "phagocytes" are used interchangeably herein to refer to a cell that is capable of phagocytosis. There are three main categories of phagocytes: macrophages, mononuclear cells (histiocytes and monocytes); polymorphonuclear leukocytes (neutrophils) and dendritic cells.

The term "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

Hematopoietic stem cells (HSC), as used herein, refers to a population of cells having the ability to self-renew, and to give rise to all hematopoietic lineages. Such cell populations have been described in detail in the art. Hematopoietic progenitor cells include the myeloid committed progenitors (CMP), the lymphoid committed progenitors (CLP), megakaryocyte progenitors, and multipotent progenitors. The earliest known lymphoid-restricted cell in adult mouse bone marrow is the common lymphocyte progenitor (CLP), and the earliest known myeloid-restricted cell is the common myeloid progenitor (CMP). Importantly, these cell populations possess an extremely high level of lineage fidelity in *in vitro* and *in vivo* developmental assays. A complete description of these cell subsets may be found in Akashi et al. (2000) Nature 404(6774):193, U.S. Patent no. 6,465,247; and published application USSN 09/956,279 (common myeloid progenitor); Kondo et al. (1997) Cell 91(5):661-7, and International application WO99/10478 (common lymphoid progenitor); and is reviewed by Kondo et al. (2003) Annu Rev Immunol. 21:759-806. The composition may be frozen at liquid nitrogen temperatures and stored for long periods of time, being capable of use on thawing. For such a composition, the cells will usually be stored in a 10% DMSO, 50% FCS, 40% RPMI 1640 medium.

Populations of interest for use in the methods of the disclosure include substantially pure compositions, e.g. at least about 50% HSC, at least about 75% HSC, at least about 85% HSC, at least about 95% HSC or more; or may be combinations of one or more stem and progenitor cells populations, e.g. white cells obtained from apheresis, *etc.* Where purified cell populations are desired, the target population may be purified in accordance with known techniques. For example, a population containing white blood cells, particularly including blood or bone marrow samples, are stained with reagents specific for markers present of hematopoietic stem and progenitor cells, which markers are sufficient to distinguish the major stem and progenitor groups. The reagents, *e.g.* antibodies, may be detectably labeled, or may be indirectly labeled in the staining procedure.

Any combination of markers may be used that are sufficient to select for the stem/progenitor cells of interest. A marker combination of interest may include CD34 and CD38, which distinguishes hematopoietic stem cells, (CD34⁺, CD38⁻) from progenitor cells, which are CD34⁺, CD38⁺). HSC are lineage marker negative, and positive for expression of CD90.

In the myeloid lineage are three cell populations, termed CMPs, GMPs, and MEPs. These cells are CD34⁺ CD38⁺, they are negative for multiple mature lineage markers including early lymphoid markers such as CD7, CD10, and IL-7R, and they are further distinguished by the markers CD45RA, an isoform of CD45 that can negatively regulate at least some classes of cytokine receptor signaling, and IL-3R. These characteristics are CD45RA⁻ IL-3Rα^{lo} (CMPs), CD45RA⁺IL-3Rα^{lo} (GMPs), and CD45RA⁻ IL-3Rα⁻ (MEPs). CD45RA⁻ IL-3Rα^{lo} cells give rise to GMPs and MEPs and at least one third generate both GM and MegE colonies on a single-cell level. All three of the myeloid lineage progenitors stain negatively for the markers Thy-1 (CD90), IL-7Rα (CD127); and with a panel of lineage markers, which lineage markers may include CD2; CD3; CD4; CD7; CD8; CD10; CD11b; CD14; CD19; CD20; CD56; and glycophorin A (GPA) in humans and CD2; CD3; CD4; CD8; CD19; IgM; Ter110; Gr-1 in mice. With the exception of the mouse MEP subset, all of the progenitor cells are CD34 positive. In the mouse all of the progenitor subsets may be further characterized as Sca-1 negative, (Ly-6E and Ly-6A), and c-kit high. In the human, all three of the subsets are CD38⁺.

Common lymphoid progenitors, CLP, express low levels of *c-kit* (CD117) on their cell surface. Antibodies that specifically bind *c-kit* in humans, mice, rats, *etc.* are known in the art. Alternatively, the *c-kit* ligand, steel factor (Slf) may be used to identify cells expressing *c-kit.* The CLP cells express high levels of the IL-7 receptor alpha chain (CDw127). Antibodies that bind to human or to mouse CDw127 are known in the art. Alternatively, the cells are identified by binding of the ligand to the receptor, IL-7. Human CLPs express low levels of CD34. Antibodies specific for human CD34 are commercially available and well known in the art. See, for example, Chen et al. (1997) Immunol Rev 157:41-51. Human CLP cells are also characterized as CD38 positive and CD10 positive. The CLP subset also has the phenotype of lacking expression of lineage specific markers, exemplified by B220, CD4, CD8, CD3, Gr-1 and Mac-1. The CLP cells are characterized as lacking expression of Thy-1, a marker that is characteristic of hematopoietic stem cells. The phenotype of the CLP may be further characterized as Mel-14⁻, CD43^{lo}, HSA^{lo}, CD45⁺ and common cytokine receptor γ chain positive.

Megakaryocyte progenitor cells (MKP) cells are positive for CD34 expression, and tetraspanin CD9 antigen. The CD9 antigen is a 227-amino acid molecule with 4 hydrophobic domains and 1 N-glycosylation site. The antigen is widely expressed, but is not present on certain progenitor cells in the hematopoietic lineages. The MKP cells express CD41, also referred to as the glycoprotein IIb/IIIa integrin, which is the platelet receptor for fibrinogen and several other extracellular matrix molecules., for which antibodies are commercially available, for example from BD Biosciences, Pharmingen, San Diego, CA., catalog number 340929, 555466. The MKP cells are positive for expression of CD117, which recognizes the receptor tyrosine kinase c-Kit. Antibodies are commercially available, for example from BD Biosciences, Pharmingen, San Diego, CA, Cat. No. 340529. MKP cells are also lineage negative, and negative for expression of Thy-1 (CD90).

The phrase "solid tumor" as used herein refers to an abnormal mass of tissue that usually does not contain cysts or liquid areas. Solid tumors may be benign or malignant. Different types of solid tumors are named for the type of cells that form them. Examples of solid tumors are sarcomas, carcinomas, lymphomas etc.

Anti-CD47 antibodies. Certain antibodies that bind CD47 prevent its interaction with SIRPα receptor. Antibodies include free antibodies and antigen binding fragments derived therefrom, and conjugates, e.g. pegylated antibodies, drug, radioisotope, or toxin conjugates, and the like.

Monoclonal antibodies directed against a specific epitope, or combination of epitopes, will allow for the targeting and/or depletion of cellular populations expressing the marker. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (See, e.g., U.S. Pat. No. 5,985,660; and Morrison et al. Cell, 96:737-49 (1999)). These techniques allow for the screening of particular populations of cells; in immunohistochemistry of biopsy samples; in detecting the presence of markers shed by cancer cells into the blood and other biologic fluids, and the like.

Humanized versions of such antibodies are also within the scope of this invention. Humanized antibodies are especially useful for in vivo applications in humans due to their low antigenicity.

The phrase "bispecific antibody" refers to a synthetic or recombinant antibody that recognizes more than one protein. Examples include bispecific antibodies 2B1, 520C9xH22, mDX-H210, and MDX447. Bispecific antibodies directed against a combination of epitopes, will allow for the targeting and/or depletion of cellular populations expressing the combination of epitopes. Exemplary bi-specific antibodies include those targeting a combination of CD47 and a cancer cell marker, such as, CD96, CD97, CD99, PTHR2, HAVCR2 etc. Generation of bi-specific antibody is described in the literature, for example, in USPN 5989830, USPN 5798229.

The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

The terms "recipient", "individual", "subject", "host", and "patient", used interchangeably herein and refer to any mammalian subject for whom diagnosis, treatment, or therapy is desired, particularly humans.

A "host cell", as used herein, refers to a microorganism or a eukaryotic cell or cell line cultured as a unicellular entity which can be, or has been, used as a recipient for a recombinant vector or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

The terms "cancer", "neoplasm", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection or treatment in the present application include precancerous (e.g., benign), malignant, pre-metastatic, metastatic, and non-metastatic cells. Detection of cancerous cells is of particular interest. The term "normal" as used in the context of "normal cell," is meant to refer to a cell of an untransformed phenotype or exhibiting a morphology of a non-transformed cell of the tissue type being examined. "Cancerous phenotype" generally refers to any of a variety of biological phenomena that are characteristic of a cancerous cell, which phenomena can vary with the type of cancer. The cancerous phenotype is generally identified by abnormalities in, for example, cell growth or proliferation (e.g., uncontrolled growth or proliferation), regulation of the cell cycle, cell mobility, cell-cell interaction, or metastasis, etc.

"Therapeutic target" refers to a gene or gene product that, upon modulation of its activity (e.g., by modulation of expression, biological activity, and the like), can provide for modulation of the cancerous phenotype. As used throughout, "modulation" is meant to refer to an increase or a decrease in the indicated phenomenon (e.g., modulation of a biological activity refers to an increase in a biological activity or a decrease in a biological activity).

### METHODS FOR TRANSPLANTATION

Disclosed herein are methods to manipulate phagocytosis of circulating hematopoietic cells. In some instances, the circulating cells are hematopoietic stem cells, or hematopoietic progenitor cells, particularly in a transplantation context, where protection from phagocytosis is desirable. In other instances the circulating cells are leukemia cells, particularly acute myeloid leukemia (AML), where increased phagocytosis is desirable.

In some embodiments, hematopoietic stem or progenitor cells are protected from phagocytosis in circulation by providing a host animal with a CD47 mimetic molecule, which interacts with SIRPα on macrophages and decreases macrophage phagocytosis. The CD47 mimetic may be soluble CD47; CD47 coated on the surface of the cells to be protected, a CD47 mimetic that binds to SIRPα at the CD47 binding site, and the like. In some embodiments, CD47 is provided as a fusion protein, for example soluble CD47 fused to an Fc fragment, *e.g*., IgG1 Fc, IgG2 Fc, Ig A Fc *etc.*

Methods for generating proteins lacking the transmembrane region are well known in the art. For example, a soluble CD47 can be generated by introducing a stop codon immediately before the polynucleotide sequence encoding the transmembrane region. Alternatively, the polynucleotide sequence encoding the transmembrane region can be replaced by a polynucleotide sequence encoding a fusion protein such as IgG1 Fc. Sequence for Fc fragments from different sources are available via publicly accessible database including Entrez, Embl, etc. For example, mRNA encoding human IgG1 Fc fragment is provided by accession number X70421.

Herein disclosed are methods for transplanting hematopoietic stem or progenitor cells into a mammalian recipient. A need for transplantation may be caused by genetic or environmental conditions, *e.g.* chemotherapy, exposure to radiation, *etc.* The cells for transplantation may be mixtures of cells, e.g. buffy coat lymphocytes from a donor, or may be partially or substantially pure. The cells may be autologous cells, particularly if removed prior to cytoreductive or other therapy, or allogeneic cells, and may be used for hematopoietic stem or progenitor cell isolation and subsequent transplantation.

The cells may be combined with the soluble CD47 mimetic prior to administration. For example, the cells may be combined with the mimetic at a concentration of from about 10 µg/ml, about 100 µg/ml, about 1 mg/ml, about 10 mg/ml, etc., at a temperature of from about 4°, about 10°, about 25° about 37°, for a period of time sufficient to coat the cells, where in some embodiments the cells are maintained on ice. In other embodiments the cells are contacted with the CD47 mimetic immediately prior to introduction into the recipient, where the concentrations of mimetic are as described above.

The composition comprising hematopoietic stem or progenitor cells and a CD47 mimetic is administered in any physiologically acceptable medium, normally intravascularly, although they may also be introduced into bone or other convenient site, where the cells may find an appropriate site for regeneration and differentiation. Usually, at least 1x10⁵ cells will be administered, preferably 1x10⁶ or more. The composition may be introduced by injection, catheter, or the like.

### MYELOPROLIFERATIVE DISORDERS, LEUKEMIAS, AND MYELODYSPLASTIC SYNDROME

Acute leukemias are rapidly progressing leukemia characterized by replacement of normal bone marrow by blast cells of a clone arising from malignant transformation of a hematopoietic cell. The acute leukemias include acute lymphoblastic leukemia (ALL) and acute myelogenous leukemia (AML). ALL often involves the CNS, whereas acute monoblastic leukemia involves the gums, and AML involves localized collections in any site (granulocytic sarcomas or chloromas).

The presenting symptoms are usually nonspecific (e.g., fatigue, fever, malaise, weight loss) and reflect the failure of normal hematopoiesis. Anemia and thrombocytopenia are very common (75 to 90%). The WBC count may be decreased, normal, or increased. Blast cells are usually found in the blood smear unless the WBC count is markedly decreased. The blasts of ALL can be distinguished from those of AML by histochemical studies, cytogenetics, immunophenotyping, and molecular biology studies. In addition to smears with the usual stains, terminal transferase, myeloperoxidase, Sudan black B, and specific and nonspecific esterase.

ALL is the most common malignancy in children, with a peak incidence from ages 3 to 5 yr. It also occurs in adolescents and has a second, lower peak in adults. Typical treatment emphasizes early introduction of an intensive multidrug regimen, which may include prednisone, vincristine, anthracycline or asparaginase. Other drugs and combinations are cytarabine and etoposide, and cyclophosphamide. Relapse usually occurs in the bone marrow but may also occur in the CNS or testes, alone or concurrent with bone marrow. Although second remissions can be induced in many children, subsequent remissions tend to be brief.

The incidence of AML increases with age; it is the more common acute leukemia in adults. AML may be associated with chemotherapy or irradiation (secondary AML). Remission induction rates are lower than with ALL, and long-term disease-free survival reportedly occurs in only 20 to 40% of patients. Treatment differs most from ALL in that AML responds to fewer drugs. The basic induction regimen includes cytarabine; along with daunorubicin or idarubicin. Some regimens include 6-thioguanine, etoposide, vincristine, and prednisone.

Polycythemia vera (PV) is an idiopathic chronic myeloproliferative disorder characterized by an increase in Hb concentration and RBC mass (erythrocytosis). PV occurs in about 2.3/100,000 people per year; more often in males (about 1.4:1). The mean age at diagnosis is 60 yr (range, 15 to 90 yr; rarely in childhood); 5% of patients are < 40 yr at onset. The bone marrow sometimes appears normal but usually is hypercellular; hyperplasia involves all marrow elements and replaces marrow fat. There is increased production and turnover of RBCs, neutrophils, and platelets. Increased megakaryocytes may be present in clumps. Marrow iron is absent in > 90% of patients, even when phlebotomy has not been performed.

Studies of women with PV who are heterozygous at the X-chromosome-linked locus for G6PD have shown that RBCs, neutrophils, and platelets have the same G6PD isoenzyme, supporting a clonal origin of this disorder at a pluripotent stem cell level.

Eventually, about 25% of patients have reduced RBC survival and fail to adequately increase erythropoiesis; anemia and myelofibrosis develop. Extramedullary hemopoiesis occurs in the spleen, liver, and other sites with the potential for blood cell formation.

Without treatment, 50% of symptomatic patients die within 18 mo of diagnosis. With treatment, median survival is 7 to 15 yr. Thrombosis is the most common cause of death, followed by complications of myeloid metaplasia, hemorrhage, and development of leukemia.

The incidence of transformation into an acute leukemia is greater in patients treated with radioactive phosphate (³²P) or alkylating agents than in those treated with phlebotomy alone. PV that transforms into acute leukemia is more resistant to induction chemotherapy than de novo leukemia.

Because PV is the only form of erythrocytosis for which myelosuppressive therapy may be indicated, accurate diagnosis is critical. Therapy must be individualized according to age, sex, medical status, clinical manifestations, and hematologic findings.

Myelodysplastic syndrome (MDS) is a group of syndromes (preleukemia, refractory anemias, Ph-negative chronic myelocytic leukemia, chronic myelomonocytic leukemia, myeloid metaplasia) commonly seen in older patients. Exposure to carcinogens may by be implicated. MDS is characterized by clonal proliferation of hematopoietic cells, including erythroid, myeloid, and megakaryocytic forms. The bone marrow is normal or hypercellular, and ineffective hematopoiesis causes variable cytopenias, the most frequent being anemia. The disordered cell production is also associated with morphologic cellular abnormalities in marrow and blood. Extramedullary hematopoiesis may occur, leading to hepatomegaly and splenomegaly. Myelofibrosis is occasionally present at diagnosis or may develop during the course of MDS. The MDS clone is unstable and tends to progress to AML.

Anemia is the most common clinical feature, associated usually with macrocytosis and anisocytosis. Some degree of thrombocytopenia is usual; on blood smear, the platelets vary in size, and some appear hypogranular. The WBC count may be normal, increased, or decreased. Neutrophil cytoplasmic granularity is abnormal, with anisocytosis and variable numbers of granules. Eosinophils also may have abnormal granularity. A monocytosis is characteristic of the chronic myelomonocytic leukemia subgroup, and immature myeloid cells may occur in the less well differentiated subgroups. The prognosis is highly dependent on classification and on any associated disease. Response of MDS to AML chemotherapy is similar to that of AML, after age and karyotype are considered.

### TREATMENT OF CANCER

Herein disclosed are methods for reducing growth of cancer cells by increasing their clearance by phagocytosis, through the introduction of a CD47 blocking agent, e.g. an anti-CD47 antibody. In the invention, the cancer cells are ovarian tumour cells. By blocking the activity of CD47, the downregulation of phagocytosis that is found with ovarian tumor cells is prevented.

In addition to CD47, we have discovered a number of markers specific to AML SC. These include CD96, CD97, CD99, PTHR2, HAVCR2 etc. These markers have been disclosed in U.S. Patent Application No. 61/011,324, filed on January 15, 2008.

"Reducing growth of cancer cells" includes, but is not limited to, reducing proliferation of cancer cells, and reducing the incidence of a non-cancerous cell becoming a cancerous cell. Whether a reduction in cancer cell growth has been achieved can be readily determined using any known assay, including, but not limited to, [³H]-thymidine incorporation; counting cell number over a period of time; detecting and/or measuring a marker associated with AML, etc.

Whether a substance, or a specific amount of the substance, is effective in treating cancer can be assessed using any of a variety of known diagnostic assays for cancer, including, but not limited to biopsy, contrast radiographic studies, CAT scan, and detection of a tumor marker associated with cancer in the blood of the individual. The substance can be administered systemically or locally, usually systemically.

In certain embodiments, a bi-specific antibody may be used. For example a bi-specific antibody in which one antigen binding domain is directed against CD47 and the other antigen binding domain is directed against a cancer cell marker, such as, CD96 CD97, CD99, PTHR2, HAVCR2 etc. may be used.

Depletion of AMLSC is useful in the treatment of AML. Depletion can be achieved by several methods. Depletion is defined as a reduction in the target population by up to about 30%, or up to about 40%, or up to about 50%, or up to about 75% or more. An effective depletion is usually determined by the sensitivity of the particular disease condition to the levels of the target population. Thus in the treatment of certain conditions a depletion of even about 20% could be beneficial.

A CD47 specific agent that specifically depletes the targeted AMLSC is used to contact the patient blood *in vitro* or *in vivo,* wherein after the contacting step, there is a reduction in the number of viable AMLSC in the targeted population. An effective dose of antibodies for such a purpose is sufficient to decrease the targeted population to the desired level, for example as described above. Antibodies for such purposes may have low antigenicity in humans or may be humanized antibodies.

### EXAMPLE 1

### CD47 IS A MARKER OF MYELOID LEUKEMIAS

### Materials and Methods

*Immunohistochemistry.* Cytospins of double sorted myeloid progenitor populations (CMP, GMP), IL-3Rα high CD45 RA+ cells and CD14+c-kit+lin- cells were performed using a Shandon cytospin apparatus. Cytospins were stained with Giemsa diluted 1/5 with H20 for 10 min followed by staining with May-Grunwald for 20 minutes. Cytospins were analyzed with the aid of a Zeiss microscope.

*Human Bone Marrow and Peripheral Blood Samples.* Normal bone marrow samples were obtained with informed consent from 20 - 25 year old paid donors who were hepatitis A, B, C and HIV negative by serology (All Cells). CMML bone marrow samples were obtained with informed consent, from previously untreated patients, at Stanford University Medical Center.

*Human Bone Marrow HSC and Myeloid Progenitor Flow-Cytometric Analysis and Cell Sorting.* Mononuclear fractions were extracted following Ficoll density centrifugation according to standard methods and analyzed fresh or subsequent to rapid thawing of samples previously frozen in 90% FCS and 10% DMSO in liquid nitrogen. In some cases, CD34+ cells were enriched from mononuclear fractions with the aid of immunomagnetic beads (CD34+ Progenitor Isolation Kit, Miltenyi Biotec, Bergisch-Gladbach, Germany). Prior to FACS analysis and sorting, myeloid progenitors were stained with lineage marker specific phycoerythrin (PE)-Cy5-conjugated antibodies including CD2 RPA-2.10; CD11b, ICRF44; CD20, 2H7; CD56, B159; GPA, GA-R2 (Becton Dickinson - PharMingen, San Diego), CD3,S4.1;CD4, S3.5; CD7, CD7-6B7; CD8, 3B5; CD10, 5-1B4, CD14, TUK4; CD19, SJ25-C1 (Caltag, South San Francisco, CA) and APC-conjugated anti-CD34, HPCA-2 (Becton Dickinson-PharMingen), biotinylated anti-CD38, HIT2 (Caltag) in addition to PE-conjugated anti-IL-3Rα, 9F5 (Becton Dickinson- ParMingen) and FITC-conjugated anti-CD45RA, MEM56 (Caltag) followed by staining with Streptavidin -Texas Red to visualize CD38-BIO stained cells and resuspension in propidium iodide to exclude dead cells. Unstained samples and isotype controls were included to assess background fluorescence.

Following staining, cells were analyzed and sorted using a modified FACS Vantage (Becton Dickinson Immunocytometry Systems, Mountain View, CA) equipped with a 599 nm dye laser and a 488 nm argon laser. Double sorted progenitor cells (HSC) were identified as CD34+ CD38+ and lineage negative. Common myeloid progenitors (CMP) were identified based on CD34+ CD38+ IL-3Rα+ CD45RA- lin- staining and their progeny including granulocyte/macrophage progenitors (GMP) were CD34+CD38+IL-3Rα+ CD45RA+ while megakaryocyte/erythrocyte progenitors (MEP) were identified based on CD34+ CD38+ IL-3Rα - CD45RA- lin- staining (Manz, PNAS 11872).

### CD47 Expression by Normal versus Myeloproliferative and AML Progenitors

Peripheral blood and bone marrow samples were obtained with informed consent from patients with myeloproliferative disorders and acute myelogenous leukemia at Stanford University Medical Center according to Stanford IRB and HIPAA regulations. Peripheral blood or bone marrow mononuclear cells (1 - 5x10⁶ cells) were stained with lineage cocktail as above but excluding CD7, CD11b and CD14. Subsequently, samples were stained with CD14 PE (1/25), CD47 FITC (1/25), CD38 Bio (Bio) and c-kit APC (1/25) or CD34 APC or FITC (1/50) for 45 min followed by washing and staining with Streptavidin Texas Red (1/25) for 45 min and finally resuspension in propidium iodide.

### Discussion

Here we show that CD47 overexpression is characteristic of progression of human myeloproliferative disorders to AML (see Figures 1 - 5B). CD47 controls integrin function but also the ability of macrophages to phagocytose cells, depending on the level of CD47 expression. Thus, aberrant CD47 expression may allow LSC to evade both innate and adaptive host immunity.

Human CD47 expression analysis was performed via FACS on human normal, pre-leukemic myeloproliferative disorder (MPD) or AML HSC, progenitors and lineage positive cells derived from marrow or peripheral blood. MPD samples (n=63) included polycythemia vera (PV; n=15), post-polycythemic myeloid metaplasia/myelofibrosis (PPMM/MF; n=5), essential thrombocythemia (ET; n=8), atypical chronic myelogenous leukemia (aCML; n=2), CML (n=7), chronic eosinophilic leukemia (CEL; n=1), chronic myelomonocytic leukemia (CMML; n= 13) and acute myelogenous leukemia (AML; n=12). As we have observed with the transgenic leukemic mouse models, progression of human myeloproliferative disorders to AML (n=12) was associated with an expansion of the GMP pool (70.6%+/- S.D. 2.15) compared with normal bone marrow (14.7%+/- S.D. 2.3). Furthermore, FACS analysis revealed that CD47 expression first increased 1.7 fold in AML compared with normal HSC and then increased to 2.2 fold greater than normal with commitment of AML progenitors to the myeloid lineage. CD47 was over-expressed by AML primitive progenitors and their progeny but not by the majority of MPD (MFI 2.3+/-S.D. 0.43) compared with normal bone marrow (MFI 1.9 +/-S.D. 0.07). Thus, increased CD47 expression is a useful diagnostic marker for progression to AML and in addition represents a novel therapeutic target.

### Example 2

### Human and mouse leukemias upregulate CD47 to evade macrophage killing

*CD47 Facilitates Engraftment, Inhibits Phagocytosis, and is More Highly Expressed on AML LSC.* We determined expression of CD47 on human AML LSC and normal HSC by flow cytometry. HSC (Lin-CD34+CD38-CD90+) from three samples of normal human mobilized peripheral blood and AML LSC (Lin-CD34+CD38-CD90-) from seven samples of human AML were analyzed for surface expression of CD47 (Figure 6). CD47 was expressed at low levels on the surface of normal HSC; however, on average, it was approximately 5-fold more highly expressed on AML LSC, as well as bulk leukemic blasts.

*Anti-Human CD47 Monoclonal Antibody Stimulates Phagocytosis and Inhibits Engraftment of AML LSC.* In order to test the model that CD47 overexpression on AML LSC prevents phagocytosis of these cells through its interaction with SIRPα on effector cells, we have utilized a monoclonal antibody directed against CD47 known to disrupt the CD47-SIRPα interaction. The hybridoma producing a mouse-anti-human CD47 monoclonal antibody, termed B6H12, was obtained from ATCC and used to produce purified antibody. First, we conducted *in vitro* phagocytosis assays. Primary human AML LSC were purified by FACS from two samples of human AML, and then loaded with the fluorescent dye CFSE. These cells were incubated with mouse bone marrow-derived macrophages and monitored using immunofluorescence microscopy (Figure 7) and flow cytometry (Figure 9) to identify phagocytosed cells. In both cases, no phagocytosis was observed in the presence of an isotype control antibody; however, significant phagocytosis was detected with the addition of the anti-CD47 antibody (Figure 9). Thus, blockage of human CD47 with a monoclonal antibody is capable of stimulating the phagocytosis of these cells by mouse macrophages.

We next investigated the ability of the anti-CD47 antibody to inhibit AML LSC engraftment *in vivo.* Two primary human AML samples were either untreated or coated with the anti-CD47 antibody prior to transplantation into NOG newborn mice. 13 weeks later, the mice were sacrificed and analyzed for human leukemia bone marrow engraftment by flow cytometry (Figure 10). The control mice demonstrated leukemic engraftment while mice transplanted with the anti-CD47-coated cells showed little to no engraftment. These data indicate that blockade of human CD47 with a monoclonal antibody is able to inhibit AML LSC engraftment.

*CD96 is a Human Acute Myeloid Leukemia Stem Cell-Specific Cell Surface Molecule.* CD96, originally termed Tactile, was first identified as a T cell surface molecule that is highly upregulated upon T cell activation. CD96 is expressed at low levels on resting T and NK cells and is strongly upregulated upon stimulation in both cell types. It is not expressed on other hematopoietic cells, and examination of its expression pattern showed that it is only otherwise present on some intestinal epithelia. The cytoplasmic domain of CD96 contains a putative ITIM motif, but it is not know if this functions in signal transduction. CD96 promotes adhesion of NK cells to target cells expressing CD155, resulting in stimulation of cytotoxicity of activated NK cells.

*Preferential Cell Surface Expression of Molecules Identified from Gene Expression Analysis.* Beyond CD47 and CD96, several molecules described in U.S. Patent Application No. 61/011,324 are known to be expressed on AML LSC, including: CD123, CD44, CD99 and CD33.

Tumor progression is characterized by several hallmarks, including growth signal independence, inhibition of apoptosis, and evasion of the immune system, among others. We show here that expression of CD47, a ligand for the macrophage inhibitory signal regulatory protein alpha (SIRPα) receptor, is increased in human and mouse myeloid leukaemia and allows cells to evade phagocytosis and increase their tumorigenic potential. CD47, also known as integrin associated protein (IAP), is an immunoglobulin-like transmembrane pentaspanin that is broadly expressed in mammalian tissues. We provide evidence that CD47 is upregulated in mouse and human myeloid leukaemia stem and progenitor cells, as well as leukemic blasts. Consistent with a biological role for CD47 in myeloid leukaemia development and maintenance, we demonstrate that ectopic over-expression of CD47 allows a myeloid leukaemia cell line to grow in mice that are T, B, and NK-cell deficient, whereas it is otherwise cleared rapidly when transplanted into these recipients. The leukemogenic potential of CD47 is also shown to be dose-dependent, as higher expressing clones have greater tumor forming potential than lower expressing clones. We also show that CD47 functions in promoting leukemogenesis by inhibiting phagocytosis of the leukemic cells by macrophages.

CD47 is significantly upregulated in leukemic Fas^{lpr/lpr} x hMRP8bcl2 transgenic bone marrow, and in leukemic hMRP8bcr/abl x hMRP8bcl2 mice. Transcripts for CD47 are increased in leukemic hMRP8bcr/abl x hMRP8bcl2 bone marrow 3-4 fold by quantitative RT-PCR and 6-7 fold in c-Kit enriched leukemic marrow relative to healthy hMRP8bcl2+ bone marrow (Figure 11e). Leukemic spleen had an expansion of the granulocyte macrophage progenitor (GMP) population as well as c-Kit+ Sca-1+ Lin-stem and progenitor subsets relative to control mice, which were of the same genotype as leukemic mice but failed to develop disease (Figure 11a-d). Expression levels for CD47 protein were found to begin increasing in leukemic mice relative to control mice at the stage of the Flk2- CD34- c-Kit+ Sca-1+ Lin- long-term hematopoietic stem cell (LT-HSC) (Figure 11f). This increased level of expression was maintained in GMP and Mac-1+ blasts, but not megakaryocyte/erythroid restricted progenitors (MEP) (Figure 11f). The increase in CD47 between leukemic and normal cells was between 3 to 20 fold. All mice that developed leukaemia that we have examined from hMRP8bcr/abl x hMRP8bcl2 primary (n=3) and secondary transplanted mice (n=3), Fas ^{lpr/lpr} x hMRP8bcl2 primary (n=14) and secondary (n=19) mice, and hMRP8bcl2 x hMRP8bcl2 primary (n=3) and secondary (n=12) mice had increased CD47 expression. We have also found increased CD47 expression in mice that received p210bcr/abl retrovirally-transduced mouse bone marrow cells that developed leukemia.

FACS-mediated analysis of human hematopoietic progenitor populations was performed on blood and marrow derived from normal cord blood and mobilized peripheral blood (n=16) and myeloproliferative disorders (MPDs) including polycythemia vera (PV; n=16), myelofibrosis (MF; n=5), essential thrombocythemia (ET; n=7), chronic myelomonocytic leukaemia (CMML; n=11) and atypical chronic myeloid leukaemia (aCML; n=1) as well as blast crisis phase chronic myeloid leukaemia (CML; n=19), chronic phase CML (n=7) and acute myelogenous leukaemia (AML; n=13). This analysis demonstrated that granulocyte-macrophage progenitors (GMP) expanded in MPDs with myeloid skewed differentiation potential including atypical CML, proliferative phase CMML and acute leukaemia including blast crisis CML and AML (Figure 12a). AML HSC and progenitors uniformly exhibited higher levels of CD47 expression compared with normal controls (Figure 12b); every sample from BC-CML and AML had elevated levels of CD47. Moreover, progression from chronic phase CML to blast crisis was associated with a significant increase in CD47 expression (Figure 12c). Using the methods described in this study, we have found that human CD47 protein expression in CML-BC increased 2.2 fold in CD90+ CD34+ CD38- Lin-cells relative to normal (p=6.3 x 10⁻⁵), 2.3 fold in CD90- CD34+ CD38- Lin- cells relative to normal (p=4.3 x 10⁻⁵), and 2.4 fold in CD 34+ CD38+ Lin- cells (p=7.6 x 10⁻⁶) (Figures 12b-12c); however, using a newer optimized staining protocol we have observed that CD47 is increased approximately 10 fold in AML and BC-CML compared to normal human HSCs and progenitors.

It was then asked whether forced expression of mouse CD47 on human leukemic cells would confer a competitive advantage in forming tumors in mice. MOLM-13 cells, which are derived from a patient with AML 5a, were transduced with Tet-MCS-IRES-GFP (Tet) or Tet-CD47-MCS-IRES-GFP (Tet-CD47) (Figure 13a), and stable integrants were propagated on the basis of GFP expression. The cells were then transplanted intravenously in a competitive setting with untransduced MOLM-13 cells into T, B, and NK deficient recombination activating gene 2, common gamma chain deficient (RAG2-/-, Gc-/-) mice. Only cells transduced with Tet-CD47 were able to give rise to tumors in these mice, efficiently engrafting bone marrow, spleen and peripheral blood (Figures 13a-b). The tumors were also characterized by large tumor burden in the liver (Figures 13b, 13g), which is particularly significant because the liver is thought to have the highest number of macrophages of any organ, with estimates that Kupffer cells may comprise 80% of the total tissue macrophage population. These cells also make up 30% of the sinusoidal lining, thereby strategically placing them at sites of entry into the liver. Hence, significant engraftment there would have to disable a macrophage cytotoxic response. In addition to developing tumor nodules, the Tet-CD47 MOLM-13 cells exhibited patterns of hepatic involvement typically seen with human AML, with leukemic cells infiltrating the liver with a sinusoidal and perivenous pattern. (Figure 13d). Overall, Tet-CD47 MOLM-13 transplanted mice died more quickly than Tet MOLM-13 transplanted mice, which had virtually no engraftment of leukemic cells in hematopoietic tissues (Figure 13c). Tet-MOLM-13 mice still had significant mortality, most likely due to localized growth at the site of injection (retro-orbital sinus) with extension into the brain.

Since CD47 has been shown to be important for the migration of hematopoietic cells, and is known to modulate binding to extracellular matrix proteins, either by direct interaction or via its effect on integrins, one possibility for the lack of growth of Tet MOLM-13 cells in mice was their inability to migrate to niches. To test this possibility, Tet MOLM-13 or Tet-CD47 MOLM-13 cells were directly injected into the femoral cavity of immunodeficient mice. Tet-CD47 MOLM-13 cells were able to engraft all bones and other hematopoietic tissues of recipient mice, whereas Tet MOLM-13 cells had minimal, if any, engraftment only at the site of injection (Figure 13e). Mice transplanted in this manner with Tet-CD47 MOLM-13 cells died at approximately 50-60 days post-transplant (n=4), whereas mice that received Tet MOLM-13 (n=5) cells remained alive for at least 75 days without signs of disease at which point they were euthanized for analysis. These results suggest a function other than or in addition to migration or homing for CD47 in MOLM-13 engraftment.

Complete lack of CD47 has been shown to result in phagocytosis of transplanted murine erythrocytes and leukocytes, via lack of interaction with SIRPα on macrophages. Thus, we tested whether over-expression of CD47 could prevent phagocytosis of live, unopsonized MOLM-13 cells. We incubated Tet or Tet-CD47 MOLM-13 cells with bone marrow derived macrophages (BMDM) for 2-24 hours and assessed phagocytosis by counting the number of ingested GFP+ cells under a microscope or by evaluating the frequency of GFP+ macrophages using a flow cytometer. Expression of CD47 dramatically lowered macrophage clearance of these cells at all time points tested, whereas Tet-MOLM-13 were quickly phagocytosed in a manner that increased over time (Figures 14a-c). We also injected MOLM-13 cells into mice and analyzed hematopoietic organs 2 hours later for evidence of macrophage phagocytosis. Macrophages in bone marrow, spleen, and liver all had higher GFP+ fraction when injected with Tet MOLM-13 cells as compared to CD47 expressing cells. This indicates that CD47 over-expression can compensate for pro-phagocytic signals already present on leukemic cells, allowing them to survive when they would otherwise be cleared by macrophages.

Recent report indicates that lack of CD47 reactivity across species might mediate xenorejections of transplanted cells. Furthermore, a recent study has demonstrated that human CD47 is unable to interact with SIRPα from C57BI/6 mice, but is able to react with receptor from non-obese diabetic (NOD) mice, which are more permissive for human cell engraftment than C57BI/6 mice. Furthermore, we have also observed that HL-60 cells, a human promyelocytic cell line with higher levels of human CD47 expression than MOLM-13, are able to engraft mice and cause leukaemia. Jurkat cells, a human T-lymphocyte cell line, are very high for human CD47 and are phagocytosed by murine macrophages *in vitro* at a much lower rate than MOLM-13. Thus, our data indicate that the ability of cells to engraft mice *in vivo* or evade phagocytosis *in vitro* by mouse macrophages correlates with the level of human CD47 expression.

To model the tumorigenic effect of having high versus low CD47 expression, we sorted clones of murine CD47 expressing MOLM-13 cells into high and low expressers. When adjusted for cell size, CD47 density on the CD47^{lo} MOLM-13 cells was approximately equal to mouse bone marrow cells, whereas CD47^{hi} MOLM-13 cells had approximately 9 fold higher expression, an increase commensurate with the change seen in CD47 expression on primary leukemic cells compared to their normal counterparts (Figure 15a). When high or low expressing cells were transplanted into recipients, only mice transplanted with high expressing cells succumbed to disease by 75 days of age (Figure 15c). Furthermore, organomegaly was more pronounced in mice transplanted with high expressing cells (Figure 15d). Mice receiving CD47^{lo} MOLM-13 cells still had notable liver masses. However, the masses were invariably 1-2 large nodes that were well-encapsulated and physically segregated from the liver parenchyma, in marked contrast to tumor masses from CD47hi MOLM-13 cells which consisted of hundreds of small masses scattered throughout the parenchyma. Thus, these large tumor masses consist of cells which have found macrophage free-niches to grow in separate from the main organ body. As expected, the infiltration of MOLM-13 cells in bone marrow and spleen of recipient mice was much higher for mice transplanted with CD47^{hi} MOLM-13 cells as well (Figure 15e). We also examined the level of CD47 expression in two mice that received CD47^{lo} MOLM-13 cells but had significant marrow engraftment. In both cases, the persisting cells after 75 days had much higher levels of CD47 than the original line (Figure 15f), indicating that a strong selection pressure exists *in vivo* for high levels of CD47 expression on leukemic cells. In total, these data indicate that CD47 expression level is a significant factor in tumorigenic potential, and that in a heterogeneous population of leukemic cells, strong selection exists for those clones with high CD47 expression.

We then asked if higher CD47 expression level would provide added protection against macrophage phagocytosis. We performed an *in vitro* phagocytosis assay with CD47^{hi} and CD47^{lo} MOLM-13 red fluorescent protein (RFP) expressing cells. After incubation with macrophages, far greater numbers of CD47^{lo} cells were phagocytosed as compared to CD47^{hi} cells (Figure 15g). If phagocytic indices are compared for control MOLM-13 cells, bulk (un-sorted) CD47 MOLM-13 cells, CD47^{lo}, and CD47^{hi} MOLM-13 cells, then a direct correlation between CD47 expression level and ability to evade phagocytosis can be seen (Figure 14a, Figure 15f). Furthermore, when CD47^{lo} RFP MOLM-13 cells and CD47^{hi} GFP MOLM-13 cells were co-incubated with macrophages in the same wells, the low expressing cells were far more likely to be phagocytosed (Figure 15h, 15i). Thus, in a mixed population of cells with varying levels of CD47 expression, the low expressing cells are more likely to be cleared by phagocytic clearance over time.

We also titrated CD47 expression using another method. Since CD47 is expressed in MOLM-13 cells using a Tet-OFF system, we utilized the Tet-inducible promoter element to control expression of CD47 in MOLM-13 cells. Beginning two weeks after transplantation with CD47^{hi} MOLM-13 cells, a cohort of mice was given doxycycline and followed for up to 75 days post-transplant. During this time course, none of the mice given doxycycline succumbed to disease or had large infiltration of MOLM-13 cells in hematopoietic organs (Figures 15b-d). At the doses of doxycycline used in this experiment, muCD47 expression in MOLM-13 cells was reduced to levels below that of normal mouse bone marrow, but notably not completely absent (Figure 15b). Thus, a sustained high level of CD47 expression is required for robust MOLM-13 survival in hematopoietic organs.

Many examples of tumor clearance by T, B, and NK cells have been described in the literature, indicating that a healthy immune system is essential for regulating nascent tumor growth. However, to date, few examples have been produced indicating that macrophage-mediated phagocytosis can check tumor development. Collectively, our studies reveal that ectopic expression of CD47 can enable otherwise immunogenic tumor cells to grow rapidly in a T, B, and NK-cell deficient host. Furthermore, this is likely to reflect a mechanism used by human myeloid leukemias to evade the host immune system since CD47 is consistently upregulated in murine and human myeloid leukemias, including all forms of chronic and acute myeloid leukaemia tested thus far. Thus, it appears likely that tumor cells are capable of being recognized as a target by activated macrophages and cleared through phagocytosis. By upregulating CD47, cancers are able to escape this form of innate immune tumor surveillance.

This form of immune evasion is particularly important since these cancers often occupy sites of high macrophage infiltration. CD47 was first cloned as an ovarian tumor cell marker, indicating that it may play a role in preventing phagocytosis of other tissue cancers as well. Furthermore, solid tumors often metastasize to macrophage rich tissues such as liver, lung, bone marrow, and lymph nodes, indicating that they must be able to escape macrophage-mediated killing in those tissues. Finding methods to disrupt CD47-SIRPα interaction may thus prove broadly useful in developing novel therapies for cancer. Preventing CD47-SIRPα interaction is doubly effective since antigens from phagocytosed tumor cells may be presented by macrophages to activate an adaptive immune response, leading to further tumor destruction.

### Methods

*Mice.* hMRP8bcrabl, hMRP8bcl2, and Fas^{lpr/lpr} transgenic mice were created as previously described and crossed to obtain double transgenics. hMRP8bcl2 homozygotes were obtained by crossing heterozygote mice to each other. C57BI/6 Ka mice from our colony were used as a source of wild-type cells. For transplant experiments, cells were transplanted into C57BI/6 RAG2^{-/-} common gamma chain (Gc)^{-/-} mice given a radiation dose of 4 Gy using gamma rays from a cesium irradiator (Phillips). Primary mouse leukemias were transplanted into CD45.2 C57BI6/Ka mice given a radiation dose of 9.5 Gy. Mice were euthanized when moribund.

*Mouse tissues.* Long bones were flushed with PBS supplemented with 2% fetal calf serum staining media (SM) Spleens and livers were dissociated using frosted glass slides in SM, then passed through a nylon mesh. All samples were treated with ACK lysis buffer to lyse erythrocytes prior to further analysis.

*Quantitative RT-PCR Analysis.* Bone marrow was obtained from leukemic hMRP8bcr/abl x hMRP8bcl2 mice or hMRP8bcl2 control mice. Cells were c-Kit enriched using c-Kit microbeads and an autoMACS column (Miltenyi). RNA was extracted using Trizol reagent (Invitrogen) and reverse transcription performed using SuperScriptll reverse polymerase (Invitrogen). cDNA corresponding to approximately 1000 cells was used per PCR reaction. Quantitative PCR was performed with a SYBR green kit on an ABI Prism 7000 PCR (Applied Biosystems) machine at 50°C for 2 minutes, followed by 95°C for 10 minutes and then 40 cycles of 95°C for 15 minutes followed by 60°C for 1 minute. Beta-actin and 18S RNA were used as controls for cDNA quantity and results of CD47 expression were normalized. Sequences for 18S RNA forward and reverse primers were TTGACGGAAGGGCACCACCAG and GCACCACCACCCACGGAATCG, respectively, for beta-actin were TTCCTTCTTGGGTATGGAAT and GAGCAATGATCTTGATCCTC, and for CD47 were AGGCCAAGTCCAGAAGCATTC and AATCATTCTGCTGCTCGTTGC.

*Human Bone Marrow and Peripheral Blood Samples.* Normal bone marrow samples were obtained with informed consent from 20 - 25 year old paid donors who were hepatitis A, B, C and HIV negative by serology (All Cells). Blood and marrow cells were donated by patients with chronic myelomonocytic leukemia (CMML), chronic myeloid leukemia (CML), and acute myelogenous leukemia (AML) and were obtained with informed consent, from previously untreated patients.

*Cell lines.* MOLM-13 cells were obtained from DSMZ. HL-60 and Jurkat cells were obtained from ATCC. Cells were maintained in Iscove's modified Dulbecco's media (IMDM) plus 10% fetal bovine serum (FBS) (Hyclone). To fractionate MOLM-13 cells into those with high and low CD47 expression, Tet-CD47 MOLM-13 cells were stained with anti-mouse CD47 Alexa-680 antibody (mIAP301). The highest and lowest 5% of mouse CD47 expressing cells was sorted on a BD FACSAria and re-grown in IMDM+10%FCS for 2 weeks. The cells were sorted for three more rounds of selection following the same protocol to obtain the high and low expressing cells used in this study. To obtain red fluorescent protein (RFP) constructs, the mCherry RFP DNA was cloned into Lentilox 3.7 (pLL3.7) empty vector. Lentivirus obtained from this construct was then used to infect cell lines.

*Cell staining and flow cytometry.* Staining for mouse stem and progenitor cells was performed using the following monoclonal antibodies: Mac-1, Gr-1, CD3, CD4, CD8, B220, and Ter119 conjugated to Cy5-PE (eBioscience) were used in the lineage cocktail, c-Kit PE-Cy7 (eBioscience), Sca-1 Alexa680 (e13-161-7, produced in our lab), CD34 FITC(eBioscience), CD16/32(FcGRII/III) APC (Pharmingen), and CD135(Flk-2) PE (eBioscience) were used as previously described to stain mouse stem and progenitor subsets. Mouse CD47 antibody (clone mIAP301) was assessed using biotinylated antibody produced in our lab. Cells were then stained with streptavidin conjugated Quantum Dot 605 (Chemicon). Samples were analyzed using a FACSAria (Beckton Dickinson).

For human samples, mononuclear fractions were extracted following Ficoll density centrifugation according to standard methods and analyzed fresh or subsequent to rapid thawing of samples previously frozen in 90% FCS and 10% DMSO in liquid nitrogen. In some cases, CD34+ cells were enriched from mononuclear fractions with the aid of immunomagnetic beads (CD34+ Progenitor Isolation Kit, Miltenyi Biotec, Bergisch-Gladbach, Germany). Prior to FACS analysis and sorting, myeloid progenitors were stained with lineage marker specific phycoerythrin (PE)-Cy5-conjugated antibodies including CD2 RPA-2.10; CD11b, ICRF44; CD20, 2H7; CD56, B159; GPA, GA-R2 (Becton Dickinson - PharMingen, San Diego), CD3,S4.1;CD4, S3.5; CD7, CD7-6B7; CD8, 3B5; CD10, 5-1B4, CD14, TUK4; CD19, SJ25-C1 (Caltag, South San Francisco, CA) and APC-conjugated anti-CD34, HPCA-2 (Becton Dickinson-PharMingen), biotinylated anti-CD38, HIT2 (Caltag) in addition to PE-conjugated anti-IL-3Rα, 9F5 (Becton Dickinson- ParMingen) and FITC-conjugated anti-CD45RA, MEM56 (Caltag) followed by staining with Streptavidin -Texas Red to visualize CD38-BIO stained cells.

Following staining, cells were analyzed using a modified FACS Vantage (Becton Dickinson Immunocytometry Systems, Mountain View, CA) equipped with a 599 nm dye laser and a 488 nm argon laser or a FACSAria. Hematopoietic stem cells (HSC) were identified as CD34+ CD38+ CD90+ and lineage negative. Anti-human CD47 FITC (clone B6H12, Pharmingen) was used to assess CD47 expression in all human samples. Fold change for CD47 expression was determined by dividing the average mean fluorescence intensity of CD47 for all the samples of CML-BC, CML-CP, or AML by the average mean fluorescence intensity of normal cells for a given cell population. Common myeloid progenitors (CMP) were identified based on CD34+ CD38+ IL-3Rα+ CD45RA- lin- staining and their progeny including granulocyte/macrophage progenitors (GMP) were CD34+CD38+IL-3Rα+ CD45RA+ Lin- while megakaryocyte/erythrocyte progenitors (MEP) were identified based on CD34+ CD38+ IL-3Rα - CD45RA- Lin- staining.

To determine the density of mouse or human CD47, cells were stained with saturating amounts of anti-CD47 antibody and analyzed on a FACSAria. Since forward scatter is directly proportional to cell diameter, and density is equal to expression level per unit of surface area we used FloJo software to calculate geometric mean fluorescent intensity of the CD47 channel and divided by the geometric mean of the forward scatter value squared (FSC²) to obtain an approximation for density of CD47 expression on the membrane.

Engraftment of MOLM-13 cells was assessed by using anti-human CD45 PE-Cy7 (Pharmingen), anti-mouse CD45.2 APC (clone AL1-4A2), and anti-mouse CD47 Alexa-680 (mlAP301). All samples were resuspended in propidium iodide containing buffer before analysis to exclude dead cells. FACS data was analyzed using FloJo software (Treestar).

*Lentiviral preparation and transduction.* pRRL.sin-18.PPT.Tet07.IRES.GFP.pre, CMV, VSV, and tet trans-activator (tTA) plasmids were obtained from Luigi Naldini. The full length murine cDNA for CD47 form 2 was provided by Eric Brown (UCSF). The CD47 cDNA construct was ligated into the BamHI/Nhel site of Tet-MCS-IRES-GFP. Plasmid DNA was transfected into 293T cells using standard protocols. The supernatant was harvested and concentrated using a Beckman LM-8 centrifuge (Beckman). Cells were transduced with Tet or Tet-CD47-MCS-IRES-GFP and tTA lentivirus for 48 hours. GFP+ cells were sorted to purity and grown for several generations to ensure stability of the transgenes.

*Injections.* Cells were injected intravenously into the retro-orbital sinuses of recipient mice or via the tail vein as noted. For intra-femoral injections, cells were injected into the femoral cavity of anesthetized mice in a volume of 20 µl using a 27-gauge needle. An isofluorane gas chamber was used to anesthetize mice when necessary.

*MOLM-13 cell engraftment.* Animals were euthanized when moribund and bone marrow, spleen, and liver harvested. Peripheral blood was obtained by tail bleed of the animals 1 hour prior to euthanization. Engraftment of MOLM-13 cells in marrow, spleen, and peripheral blood was determined as described above. Tumor burden in the liver was determined by calculating the area of each visible tumor nodule using the formula ((length in mm + width in mm)/2) *π. Area of each nodule was then added together per liver.

Doxycycline administration. Doxycycline hydrochloride (Sigma) was added to drinking water at a final concentration of 1 mg/mL. Drinking water was replaced every 4 days and protected from light. In addition, mice received a 10 µg bolus of doxycycline by i.p. injection once a week.

*Bone marrow derived macrophages (BMDM).* Femurs and tibias were harvested from C57BI/6 Ka mice and the marrow was flushed and placed into a sterile suspension of PBS. The bone marrow suspension was grown in IMDM plus 10% FBS with 10 ng/mL of recombinant murine macrophage colony stimulating factor (MCSF, Peprotech) for 7-10 days.

*In vitro phagocytosis assays.* BMDM were harvested by incubation in trypsin/EDTA (Gibco) for 5 minutes and gentle scraping. Macrophages were plated at 5 x 10⁴ cells per well in a 24-well tissue culture plate (Falcon). After 24 hours, media was replaced with serum-free IMDM. After an additional 2 hours, 2.5 x 10⁵ Tet or Tet-CD47 MOLM-13 cells were added to the macrophage containing wells and incubated at 37 C° for the indicated times. After co-incubation, wells were washed thoroughly with IMDM 3 times and examined under an Eclipse T5100 (Nikon) using an enhanced green fluorescent protein (GFP) or Texas Red filter set (Nikon). The number of GFP+ or RFP+ cells within macrophages was counted and phagocytic index was calculated using the formula: phagocytic index=number of ingested cells/(number of macrophages/100). At least 200 macrophages were counted per well. For flow cytometry analysis of phagocytosis macrophages were harvested after incubation with MOLM-13 cells using trypsin/EDTA and gentle scraping. Cells were stained with anti-Mac-1 PE antibody and analyzed on a BD FACSAria. Fluorescent and brightfield images were taken separately using an Eclipse T5100 (Nikon), a super high pressure mercury lamp (Nikon), an endow green fluorescent protein (eGFP) bandpass filter (Nikon) a Texas Red bandpass filter (Nikon), and a RT Slider (Spot Diagnostics) camera. Images were merged with Photoshop software (Adobe).

For in vivo assays, marrow from leg long bones, spleen, and liver were harvested 2 hours after injecting target cells into RAG2^{-/-},Gc^{-/-} mice. They were prepared into single cell suspensions in PBS plus 2% FCS. Cells were labeled with anti-human CD45 Cy7-PE and anti-mouse F4/80 biotin (eBiosciences). Secondary stain was performed with Streptavidin-APC (eBiosciences). Cells that were human CD45-, F4/80+ were considered to be macrophages, and GFP+ cells in this fraction was assessed.

### EXAMPLE 3

### Hematopoietic stem and progenitor cells upregulate CD47 to facilitate mobilization and homing to hematopoietic tissues

We show here that hematopoietic stem cells (HSCs) from CD47 deficient (IAP^{-/-}) mice fail to engraft wild-type recipients. As expected, these cells are rapidly cleared by host macrophages, whereas IAP^{+/+} HSCs are not. When stem and progenitor cells are forced to divide and enter circulation using cyclophosphamide/G-CSF or lipopolysaccharide, CD47 is rapidly up-regulated on these cells. We propose that higher levels of CD47 in stem cells during stress hematopoiesis and mobilization provides added protection against phagocytosis by activated macrophages of the reticuloendothelial system. In support of this hypothesis, we show that IAP^{+/-} cells transplanted into wild-type recipients lose engraftment over time, whereas wild-type donor cells do not. We conclude that phagocytosis is a significant physiological mechanism that clears hematopoietic progenitors over time, and that CD47 over-expression is required to prevent phagocytic clearance.

HSCs have the ability to migrate to ectopic niches in fetal and adult life via the blood stream. Furthermore, HSCs can be prodded into the circulation using a combination of cytotoxic agents and cytokines that first expand HSC numbers in situ. Once in the blood stream, HSCs must navigate the vascular beds of the spleen and liver. Macrophages at these sites function to remove damaged cells and foreign particles from the blood stream. Furthermore, during inflammatory states, macrophages become more phagocytically active. Hence, additional protection against phagocytosis might be required for newly arriving stem cells at these sites.

We determined if CD47 expression on bone marrow stem and progenitor cells had a role in regulation of normal hematopoiesis. CD47 expression has been shown to be essential for preventing phagocytosis of red blood cells, T-cells, and whole bone marrow cells in a transplant setting. Thus, we asked if lack of CD47 would prevent HSCs from engrafting after being delivered intravenously. To test this, we employed the CD47 knockout mouse (IAP^{-/-}). These mice develop normally and do not display any gross abnormalities. They do, however, die very quickly after intraperitoneal bacterial challenge because neutrophils fail to migrate to the gut quickly. In addition, cells from these mice fail to transplant into wild-type recipients, but they will engraft in IAP-/- recipients.

We first examined stem and progenitor frequencies in IAP^{+/-} and IAP^{-/-} mice. When examining for cells in the stem and myeloid progenitor compartment, there was no difference between these mice and wild-type mice (Figure 18a). We then tested stem cells from these mice for their ability to form colonies in an *in vitro* assay. We sorted highly purified Flk2- CD34- KLS stem cells from these mice and plated them onto methylcellulose in the presence of a standard cytokine cocktail. We examined colony formation at day 7 and found that there was no major difference between wild-type and IAP-/- stem cells in the number and type of colonies formed (Figure 18b).

We then asked if bone marrow cells from IAP^{-/-} mice could rescue recipient mice from the effects of lethal irradiation. Typically, a dose of 2 x 10⁵ bone marrow cells will rescue 100% of wild-type recipient mice in this assay. We found that IAP^{-/-} bone marrow could not rescue these recipients (Figure 18c). However, administration of these cells did prolong lifespan; normally, mice die between day 12 and 15 after irradiation, but mice that received IAP^{-/-} bone marrow lived about 7 to 10 days longer (Figure 18c). We do not yet know the reason for the prolongation of lifespan in this case, although we have observed that multipotent progenitors and megakaryocyte erythrocyte progenitors can prolong survival after lethal irradiation, and that contribution from these cells following transplant of whole bone marrow may have contributed to the elongation of survival time.

Next, we sorted Flk-2⁻ CD34⁻ KLS stem cells from wild-type and IAP^{-/-} cells and transplanted them into wild-type recipients along with 2 x 10⁵ competitor cells. None of the mice which received IAP^{-/-} HSCs, at either a dose of 50 or 500 had any engraftment of donor cells, indicating that CD47 was indeed required for the ability of these cells to transplant (Figure 18d-e). We speculated that this was due to phagocytosis of the cells which lacked CD47, as has been shown for erythrocytes and T-cells. To test this, we enriched c-Kit⁺ cells from the bone marrow of wild-type and IAP^{-/-} mice and co-incubated them with bone marrow derived macrophages. IAP^{-/-} stem and progenitor cells were readily phagocytosed in this assay, whereas wild-type cells were only minimally phagocytosed (Figure 18f-g). Interestingly, when incubated with IAP^{-/-} macrophages, there was significantly less phagocytosis of IAP^{-/-} cells, confirming that macrophages from these mice are indeed abnormal in their phagocytic capacity.

Since mobilization of stem and progenitor cells involves several steps in which they come into contact with macrophages (egress from the marrow sinusoids, entry into the marrow and liver sinusoids, and in the splenic marginal zone), we asked if CD47 is up-regulated in the marrow of mice which have been induced to undergo mobilization. The most commonly used protocol involves administering the drug cyclophosphamide (Cy), which kills dividing (mainly myeloid progenitor) cells, followed by treatment with granulocyte colony stimulating factor (G-CSF). This involves administering cyclophosphamide on the first day, and then giving G-CSF every day thereafter. By convention, the first day after cyclophosphamide administration is called day 0. The peak numbers of stem cells in the bone marrow is achieved on day 2; from days 3-4 they egress from the bone marrow into the periphery, and their numbers in the spleen and liver reach a peak at day 5; myeloerythroid progenitors are also mobilized. There is a characteristic rise in the frequency of stem cells and myeloid progenitors during the mobilization response.

Thus, we administered this mobilization protocol to wild-type mice and sacrificed mice on days 2 through 5. We found that there was a notable increase of CD47 on c-Kit⁺ bone marrow cells on day 2 (Figure 19a). We found that there was approximately a four-fold increase in the level of CD47 on stem and progenitor cells on day 2 of mobilization (Figure 19b). The increase was seen at all levels of the myeloid progenitor hierarchy, as LT-HSCs as well as GMPs displayed this increase in CD47 expression (Figure 19b). By day 5, when egress from the marrow has largely halted, the levels of CD47 had returned to nearly normal levels. In Figure 19c, the mean fluorescence intensity of CD47 expression on GMPs is shown on days 0 to 5 of mobilization. CD47 levels are actually subnormal following myeloablation on day 0, but they quickly rise to a peak on day 2. The expression quickly lowers thereafter and the levels by day 5 are equivalent to steady state.

Endotoxins are also thought to contribute to bone marrow mobilization. This may represent a physiological response to infection, where normal marrow output of immune cells needs to be increased to clear the offending pathogens. Lipopolysaccharide (LPS) is a cell wall component of gram-negative bacteria. It binds to the lipid binding protein (LBP) in the serum, which can then form a complex with CD1411 and toll-like receptor 4 (TLR-4) 12 on monocytes, macrophages, and dendritic cells. This results in activation of macrophages and results in a pro-inflammatory response. LPS administration has also been shown to increase the phagocytic capacity of macrophages. This may be due to the fact that LBP-LPS complexes act as opsonins.

We tested if LPS administration in mice would affect CD47 expression in stem and progenitor cells. Mirroring the pattern seen in Cy/G induced mobilization, LPS caused expansion of stem and progenitor cells by 2 days post treatment, followed by migration to the spleen and liver (Figure 19d). On day 2 after LPS administration, stem and progenitor cells in the marrow had up-regulated CD47 to a similar degree as in Cy/G mobilization. By day 5, when the inflammatory response has resolved, the levels of the protein had dropped to steady-state levels (Figure 19d).

Since CD47 was consistently up-regulated in the mobilization response, we decided to test the ability of stem and progenitor cells to mobilize following Cy/G. The CD47 knockout mouse has defects in migration of neutrophils to sites of inflammation8 and of dendritic cells to secondary lymphoid organs. The exact role of CD47 in migration of these cells is unknown, but it may relate to poor integrin association in the circulation (CD47 binds to several integrins) or lack of interaction with SIRPα on endothelial cells. Hence we reasoned that if CD47 was involved in the migration capacity of these cells in the mobilization response, then IAP-/- mice would display reduced numbers of cells in the peripheral organs after Cy/G.

To test this hypothesis we administered Cy/G to both wild-type and knockout mice and sacrificed mice on days 2-5. For each mouse, we analyzed the number of stem and progenitor cells in marrow, spleen, and liver. We decided to use the crude KLS population as a surrogate for HSCs because numbers of CD34- cells drops considerably in proliferative states, making accurate calculation of LT-HSC numbers difficult. Since GMP are the most expanded of all the populations in mobilization, we decided to analyze their numbers as well. To calculate absolute progenitor count, the total cellularity of marrow, spleen, and liver was estimated by counting the mononuclear cell number in the whole organ by hemocytometer. For bone marrow, leg long bones were assumed to represent 15% of the total marrow. This number was then multiplied by the frequency of the cell population to determine an absolute count.

We found that there was little difference in mobilization of KLS or GMP between wild-type and IAP^{-/-} mice (Figure 19e). There was a modest decrease in the ability of IAP^{-/-} mice to move progenitors to the spleen by day 3, but by days 4 and 5 they had restored normal numbers of cells to the periphery. The marrow and liver compartments looked similar to wild-type mice. Hence, IAP^{-/-} mice do not have a significant mobilization defect.

Heterozygote IAP^{+/-} erythrocytes have roughly the half the amount of CD47 as wild-type erythrocytes and platelets. There is also a dose dependent increase in the amount of phagocytosis that occurs in immunoglobulin opsonized IAP^{+/-} erythrocytes and platelets relative to wild-type. Our observation that CD47 levels increase in states of stress and mobilization led us to hypothesize that cells that were genetically hemizygous for CD47 might be more prone to phagocytosis and clearance by macrophages over time. Hence, we asked if IAP^{+/-} stem cells would be disadvantaged relative to wild-type stem cells in long-term contribution to hematopoiesis.

We first analyzed the levels of CD47 expressed on IAP^{+/+}, IAP^{+/-}, and IAP^{-/-} stem cells. FACS analysis of CD34⁻Flk-2⁻ KLS stem cells revealed that the MFI of CD47 on heterozygote HSCs was indeed at roughly half the level of wild-type stem cells (Figure 20a). We then transplanted these cells and examined their ability to engraft and produce hematopoietic cells in a recipient. We gave congenic wild-type recipient mice 475 Gy, a sublethal dose of irradiation. We then transplanted one cohort of recipients with 2 x 10⁶ wild-type whole bone marrow cells, and another with the same dose of IAP^{+/-} bone marrow cells. Such a dose would be expected to contain roughly 50-100 HSCs. Since granulocyte chimerism in the peripheral blood is a good surrogate marker of stem cell fitness, we analyzed cells from the blood of these recipients at periodic intervals. When wild-type marrow was transplanted into wild-type recipients, granulocyte chimerism was maintained for up to 40 weeks. However, when IAP^{+/-} cells were transplanted, 3 out of 5 mice lost donor chimerism over time, despite having a successful engraftment initially (Figure 20b).

We have observed that CD47 is up-regulated on the surface of hematopoietic cells in the progression of leukemia. We have also found an analogous increase in the level of CD47 expression when mice were stimulated to mobilize stem and progenitor cells to the periphery using Cy/G, or when they were challenged with LPS. But why is CD47 upregulated in these states? Various studies have described a dose-dependent effect for CD47 in the prevention of phagocytosis. IAP^{+/-} erythrocytes and platelets, which have half the level of CD47 as wild-type cells, are phagocytosed more readily than their normal counterparts. Evidence also indicates that the level of CD47 expression on cells correlates well with the ability of the cell to engage the SIRPα inhibitory receptor on macrophages. Recently Danska et al reported that the ability of NOD-SCID mice to support transplantation of human hematopoietic cells correlated with a mutation in the SIRPalpha receptor in these mice. Here we show that stem and progenitor cells that express higher levels of CD47 are less likely to be cleared by phagocytosis.

These studies point to a role for CD47 up-regulation in protecting hematopoietic stem cells during states when they are more prone to being phagocytosed by macrophages, such as post-myeloablation and during mobilization. Macrophages have the function of removing aged or damaged cells that they encounter; it seems that they can eliminate damaged stem cells as well. Thus, healthy recovering stem cells might up-regulate CD47 during a mobilization response to prevent clearance, whereas damaged stem cells fail to do so and are cleared. We speculate that this is a mechanism by which the hematopoietic system self-regulates itself to ensure that only healthy, undamaged cells are permitted to survive and proliferate and utilize resources during high stress states. The mobilization of HSC and progenitors into the bloodstream and thence to hematopoietic sites following LPS induced inflammation is very interesting; HSC migrate from blood to marrow using integrin α4β1 (Wagers and Weissman, Stem Cells 24(4):1087-94, 2006) and the chemokine receptor CXCR4 (Wright DE et al., J Exp Med 195(9): 1145-54, 2002). We have shown previously that integrin α4β1 binds to VCAM1 on hematopoietic stroma (Mikaye K et al. J Exp Med 173(3):599-607, 1991); VCAM1 is also the vascular addressin on vessels that inflammatory T cells use to recognize and enter local sites of cell death and inflammation. In addition to expressing the integrin associated protein CD47, itinerant HSC express functional integrin α4β1, leading to the speculation that migrating hematopoietic stem and progenitors in states of inflammation may not only re-seed marrow hematopoiesis, but also participate in local inflammation as well.

### Materials and Methods

Mice. C57BI/6 CD45.1 and C57BI/6 CD45.2 (wild-type) mice were maintained in our colony. IAP-/- mice were obtained from Eric Brown (University of California, San Francisco). These were bred on C57BI6/J background and crossed with our wild-type colony.

Screening. IAP+/- were crossed to each other to generate IAP-/- and IAP+/- offspring. Mice were screened by PCR of tail DNA. The following primers were used: 3' Neo-GCATCGCATTGTCTGAGTAGGTGTCATTCTATTC; 5' IAP-TCACCTTGTTGTTCCTGTACTAC AAGCA; 3' IAP-TGTCACTTCGCAAGTGTAGTTCC.

Cell staining and sorting. Staining for mouse stem and progenitor cells was performed using the following monoclonal antibodies: Mac-1, Gr-1, CD3, CD4, CD8, B220, and Ter119 conjugated to Cy5-PE (eBioscience) were used in the lineage cocktail, c-Kit PE-Cy7 (eBioscience), Sca-1 Alexa680 (e13-161-7, produced in our lab), CD34 FITC(eBioscience), CD16/32(FcGRII/III) APC (Pharmingen), and CD135(Flk-2) PE (eBioscience) were used as previously described to stain mouse stem and progenitor subsets 21 22. Mouse CD47 antibody (clone mIAP301) was assessed using biotinylated antibody produced in our lab. Cells were then stained with streptavidin conjugated Quantum Dot 605 (Chemicon). Samples were analyzed using a FACSAria (Beckton Dickinson).

CD34- Flk2- KLS stem cells were double-sorted using a BD FACSAria. Peripheral blood cells were obtained from tail vein bleed and red cells were eliminated by Dextran T500 (Sigma) precipitation and ACK lysis. Cells were stained with anti-CD45.1 APC, anti-CD45.2 FITC, anti-Ter119 PE (Pharmingen), anti-B220 Cy5-PE (eBiosciences), anti-CD3 Cascade Blue, and anti-Mac-1 Cy7-PE (eBiosciences). Granulocytes were Ter119- B220- CD3-Mac-1+ SSC hi. Cells were analyzed using a BD FACSAria.

All samples were resuspended in propidium iodide containing buffer before analysis to exclude dead cells. FACS data was analyzed using FloJo software (Treestar).

*In vitro* colony forming assay. LT-HSC were directly clone sorted into a 96-well plate containing methycellulose media (Methocult 3100) that was prepared as described. The media was also supplemented with recombinant mouse stem cell factor (SCF), interleukin (IL)-3, IL-11, granulocyte-macrophage colony stimulating factor (GM-CSF), thrombopoietin (Tpo) and erythropoietin (Epo). Colonies were scored for CFU-G, CFU-M, CFU-GM, CFU-GEMM, and Meg.

Cell transfers. For whole bone marrow transfers, IAP+/+, IAP+/-, or IAP-/- cells were freshly isolated from leg long bones. Cells were counted using a hemacytometer and resuspended in PBS+2% FCS at 100uL. For some experiments, CD45.1 cells from C57BI/6 Ka CD45.1 mice were used as donors into CD45.2 wild-type mice.

For sorted cells, cells were sorted into PBS buffer at the correct dose (i.e. 50 or 500 cells per tube) and resuspended in 100uL of PBS+2% FCS. For competition experiments, 2 x 10⁵ freshly isolated whole bone marrow cells from C57BI/6 CD45.1 were added to the 100uL stem cell suspension.

C57BI/6 Ka CD45.1 or C57BI/6 J CD45.2 recipient mice were irradiated using a cesium source at the doses indicated. Sub-lethal dose was 4.75 Gray and lethal dose was a split dose of 9.5 Gray. Cells were transferred using a 27-gauge syringe into the retro-orbital sinuses of mice anesthetized with isofluorane.

Mobilization assay. Mice were mobilized with cyclophosphamide (Sigma) (200 mg/kg) and G-CSF (Neupogen) (250 µg/kg) as previously described. Bacterial LPS from E. coli 055:B5 (Sigma) was administered at a dose of 40 mg/kg into the peritoneal cavity.

For analysis of mobilized organs, whole spleen, whole liver, and leg long bones were prepared in a single cell suspension. Cell density was determined using a hemacytometer to determine overall cellularity of hematopoietic cells in these organs.

Enrichment of c-Kit⁺ cells. Whole mouse marrow was stained with CD117 microbeads (Miltenyi). c-Kit⁺ cells were selected on an AutoMACS Midi column (Miltenyi) using a magnetic separator.

*In vitro* phagocytosis assay. BMDM were prepared as previously described. c-Kit enriched bone marrow cells were stained with CFSE (Invitrogen) prior to the assay. 2.5 x 10⁵ c-Kit enriched cells were plated with 5 x 10⁴ macrophages. Macrophages and c-Kit cells were obtained from either IAP^{+/+} or IAP^{-/-} mice. Cells were incubated for 2 hours and phagocytic index was determined. Photographs were taken as described previously.

### Example 4

### CD47 is an Independent Prognostic Factor and Therapeutic Antibody Target on Human Acute Myeloid Leukemia Cells

Acute myelogenous leukemia (AML) is organized as a cellular hierarchy initiated and maintained by a subset of self-renewing leukemia stem cells (LSC). We hypothesized that increased CD47 expression on AML LSC contributes to pathogenesis by inhibiting their phagocytosis through the interaction of CD47 with an inhibitory receptor on phagocytes. We found that CD47 was more highly expressed on AML LSC than their normal counterparts, and that increased CD47 expression predicted worse overall survival in 3 independent cohorts of adult AML patients. Furthermore, blocking monoclonal antibodies against CD47 preferentially enabled phagocytosis of AML LSC by macrophages in vitro, and inhibited their engraftment in vivo. Finally, treatment of human AML-engrafted mice with anti-CD47 antibody eliminated AML in vivo. In summary, increased CD47 expression is an independent poor prognostic factor that can be targeted on human AML stem cells with monoclonal antibodies capable of stimulating phagocytosis of LSC.

### RESULTS

*CD47 is More Highly Expressed on AML LSC than Their Normal Counterparts and is Associated with the FLT3-ITD Mutation.* In our investigation of several mouse models of myeloid leukemia, we identified increased expression of CD47 on mouse leukemia cells compared to normal bone marrow. This prompted investigation of CD47 expression on human AML LSC and their normal counterparts. Using flow cytometry, CD47 was more highly expressed on multiple specimens of AML LSC than normal bone marrow HSC and MPP (Figure 6). This increased expression extended to the bulk leukemia cells, which expressed CD47 similarly to the LSC-enriched fraction.

Examination of a subset of these samples indicated that CD47 surface expression correlated with CD47 mRNA expression. To investigate CD47 expression across morphologic, cytogenetic, and molecular subgroups of AML, gene expression data from a previously described cohort of 285 adult patients were analyzed (Valk et al., 2004 N Engl J Med 350, 1617-1628). No significant difference in CD47 expression among FAB (French-American-British) subtypes was found. In most cytogenetic subgroups, CD47 was expressed at similar levels, except for cases harboring t(8;21)(q22;q22), a favorable risk group which had a statistically significant lower CD47 expression. In molecularly characterized AML subgroups, no significant association was found between CD47 expression and mutations in the tyrosine kinase domain of FLT3 (FLT3-TKD), over-expression of EVI1, or mutations in CEBPA, NRAS, or KRAS. However, higher CD47 expression was strongly correlated with the presence of FLT3-ITD (p<0.001), which is observed in nearly one third of AML with normal karyotypes and is associated with worse overall survival. This finding was separately confirmed in two independent datasets of 214 and 137 AML patients (Table 1).

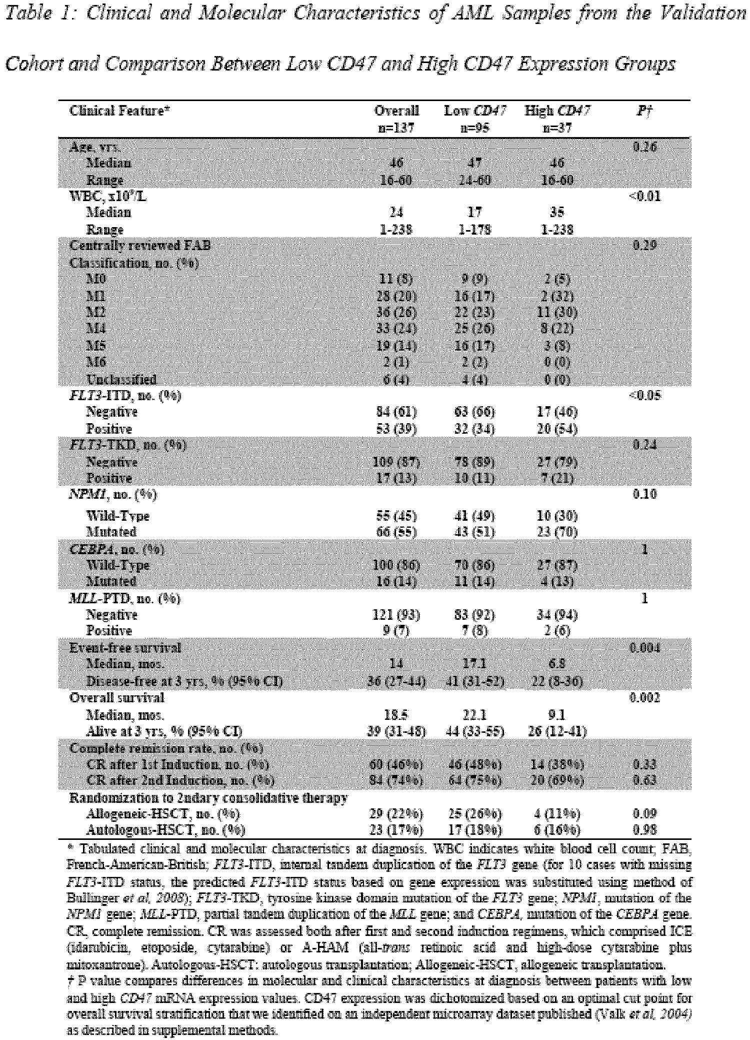

*Identification and Separation of Normal and Leukemic Progenitors From the Same Patient Based On Differential CD47 Expression.* In the LSC-enriched Lin-CD34+CD38-fraction of specimen SU008, a rare population of CD47lo-expressing cells was detected, in addition to the majority CD47^{hi}-expressing cells (Figure 21A). These populations were isolated by fluorescence-activated cell sorting (FACS) to >98% purity and either transplanted into newborn NOG mice or plated into complete methylcellulose. The CD47^{hi} cells failed to engraft in vivo or form any colonies in vitro, as can be observed with some AML specimens.

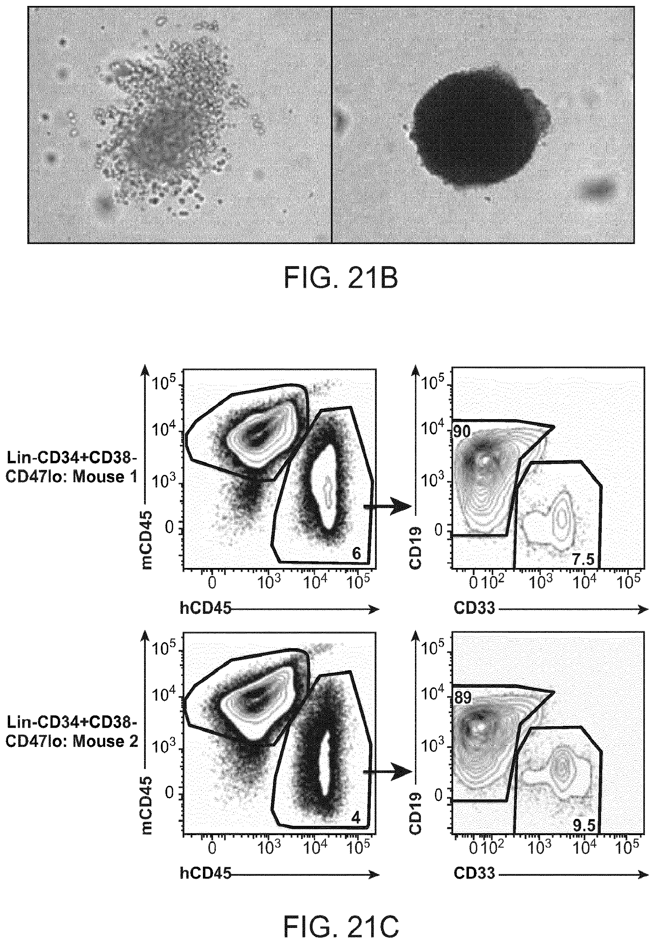

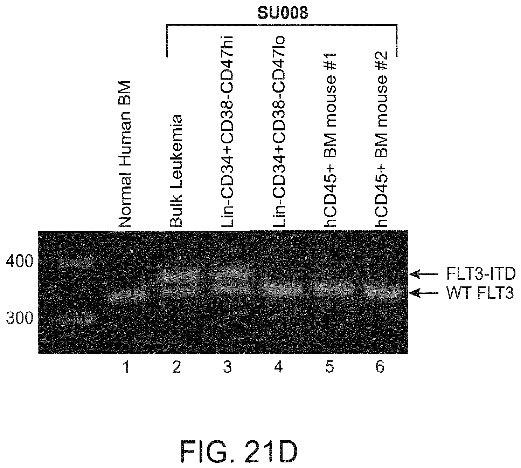

However, the CD47^{lo} cells engrafted with normal myelo-lymphoid hematopoiesis in vivo and formed numerous morphologically normal myeloid colonies in vitro (Figure 21B,C). This specimen harbored the FLT3-ITD mutation, which was detected in the bulk leukemia cells (Figure 21D). The purified CD47^{hi} cells contained the FLT3-ITD mutation, and therefore, were part of the leukemic clone, while the CD47^{lo} cells did not. Human cells isolated from mice engrafted with the CD47^{lo} cells contained only wild type FLT3, indicating that the CD47^{lo} cells contained normal hematopoietic progenitors.

*Increased CD47 Expression in Human AML is Associated with Poor Clinical Outcomes.* We hypothesized that increased CD47 expression on human AML contributes to pathogenesis. From this hypothesis, we predicted that AML with higher expression of CD47 would be associated with worse clinical outcomes. Consistent with this hypothesis, analysis of a previously described group of 285 adult AML patients with diverse cytogenetic and molecular abnormalities (Valk *et al*., 2004) revealed that a dichotomous stratification of patients into low CD47 and high CD47 expression groups was associated with a significantly increased risk of death in the high expressing group (p=0.03). The association of overall survival with this dichotomous stratification of CD47 expression was validated in a second test cohort of 242 adult patients (Metzeler *et al*., 2008 Blood) with normal karyotypes (NK-AML) (p=0.01).

Applying this stratification to a distinct validation cohort of 137 adult patients with normal karyotypes (Bullinger et al., 2008 Blood 111, 4490-4495), we confirmed the prognostic value of CD47 expression for both overall and event-free survival (Figure 22). Analysis of clinical characteristics of the low and high CD47 expression groups in this cross-validation cohort also identified statistically significant differences in white blood cell (WBC) count and FLT3-ITD status, and no differences in rates of complete remission and type of consolidative therapy including allogeneic transplantation (Table 1). Kaplan-Meier analysis demonstrated that high CD47 expression at diagnosis was significantly associated with worse event-free and overall survival (Figure 22A,B). Patients in the low CD47 expression group had a median event-free survival of 17.1 months compared to 6.8 months in the high CD47 expression group, corresponding to a hazard ratio of 1.94 (95% confidence interval 1.30 to 3.77, p=0.004). For overall survival, patients in the low CD47 expression group had a median of 22.1 months compared to 9.1 months in the high CD47 expression group, corresponding to a hazard ratio of 2.02 (95% confidence interval 1.37 to 4.03, p=0.002). When CD47 expression was considered as a continuous variable, increased expression was also associated with a worse event-free (p=0.02) and overall survival (p=0.02).

Despite the association with FLT3-ITD (Table 1), increased CD47 expression at diagnosis was significantly associated with worse event-free and overall survival in the subgroup of 74 patients without FLT3-ITD, when considered either as a binary classification (Figure 22C,D) or as a continuous variable (p=0.02 for both event-free and overall survival). In multivariable analysis considering age, FLT3-ITD status, and CD47 expression as a continuous variable, increased CD47 expression remained associated with worse event-free survival with a hazard ratio of 1.33 (95% confidence interval 1.03 to 1.73, p=0.03) and overall survival with a hazard ratio of 1.31 (95% confidence interval 1.00 to 1.71, p=0.05) (Table 2).

**Table 2**

| Outcome Measure/Variables Considered | | HR | 95% CI | *P* |
|---|---|---|---|---|
| Event-free survival | | | | |
| | CD47 expression, continuous, per 2-fold increase | 1.33 | 1.03-1.73 | 0.03 |
| | *FL7*3-ITD, positive vs. negative | 2.21 | 1.39-3.53 | <0.001 |
| | Age, per year | 1.03 | 1.00-1.06 | 0.03 |

| Overall survival | | | | |
|---|---|---|---|---|
| | CD47 expression, continuous, per 2-fold increase | 1.31 | 1.00-1.71 | 0.05 |
| | *FLT3*-ITD, positive vs. negative | 2.29 | 1.42-3.68 | <0.001 |
| | Age, per year | 1.03 | 1.01-1.06 | 0.01 |

*Monoclonal Antibodies Directed Against Human CD47 Preferentially Enable Phagocytosis of AML LSC by Human Macrophages.* We hypothesized that increased CD47 expression on human AML contributes to pathogenesis by inhibiting phagocytosis of leukemia cells, leading us to predict that disruption of the CD47-SIRPα interaction with a monoclonal antibody directed against CD47 will preferentially enable the phagocytosis of AML LSC. Several anti-human CD47 monoclonal antibodies have been generated including some capable of blocking the CD47-SIRPα interaction (B6H12.2 and BRIC126) and others unable to do so (2D3) (Subramanian et al., 2006 Blood 107, 2548-2556). The ability of these antibodies to enable phagocytosis of AML LSC, or normal human bone marrow CD34+ cells, by human macrophages in vitro was tested. Incubation of AML LSC with human macrophages in the presence of IgG1 isotype control antibody or mouse anti-human CD45 IgG1 monoclonal antibody did not result in significant phagocytosis as determined by either immunofluorescence microscopy (Figure 8A) or flow cytometry. However, addition of the blocking anti-CD47 antibodies B6H12.2 and BRIC126, but not the non-blocking 2D3, enabled phagocytosis of AML LSC (Figure 8A,C). No phagocytosis of normal CD34+ cells was observed with any of the antibodies (Figure 8C).

*Monoclonal Antibodies Directed Against Human CD47 Enable Phagocytosis of AML LSC by Mouse Macrophages.* The CD47-SIRPα interaction has been implicated as a critical regulator of xenotransplantation rejection in several cross species transplants; however, there are conflicting reports of the ability of CD47 from one species to bind and stimulate SIRPα of a different species. In order to directly assess the effect of inhibiting the interaction of human CD47 with mouse SIRPα, the in vitro phagocytosis assays described above were conducted with mouse macrophages. Incubation of AML LSC with mouse macrophages in the presence of IgG1 isotype control antibody or mouse anti-human CD45 IgG1 monoclonal antibody did not result in significant phagocytosis as determined by either immunofluorescence microscopy (Figure 8B) or flow cytometry. However, addition of the blocking anti-CD47 antibodies B6H12.2 and BRIC126, but not the non-blocking 2D3, enabled phagocytosis of AML LSC (Figure 8B,C).

*A Monoclonal Antibody Directed Against Human CD47 Inhibits AML LSC Engraftment and Eliminates AML in Vivo.* The ability of the blocking anti-CD47 antibody B6H12.2 to target AML LSC in vivo was tested. First, a pre-coating strategy was utilized in which AML LSC were purified by FACS and incubated with IgG1 isotype control, anti-human CD45, or anti-human CD47 antibody. An aliquot of the cells was analyzed for coating by staining with a secondary antibody demonstrating that both anti-CD45 and anti-CD47 antibody bound the cells (Figure 10A). The remaining cells were transplanted into newborn NOG mice that were analyzed for leukemic engraftment 13 weeks later (Figure 10B). In all but one mouse, the isotype control and anti-CD45 antibody coated cells exhibited long-term leukemic engraftment. However, most mice transplanted with cells coated with anti-CD47 antibody had no detectable leukemia engraftment.

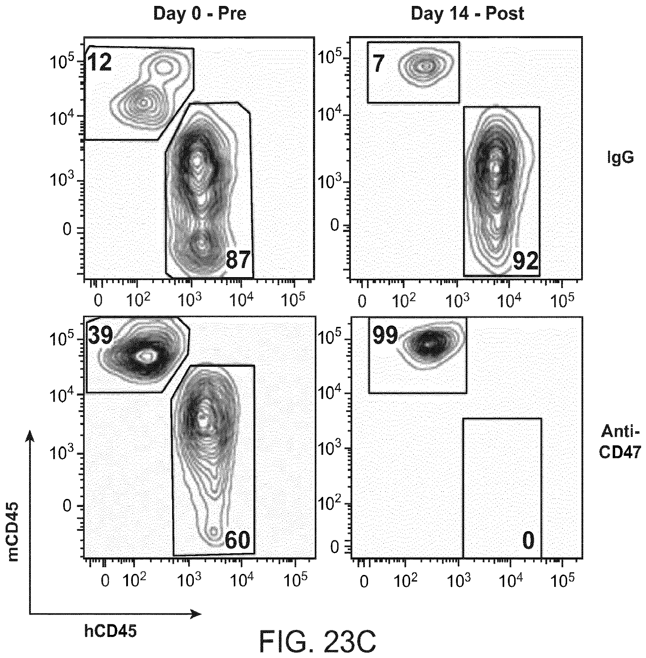

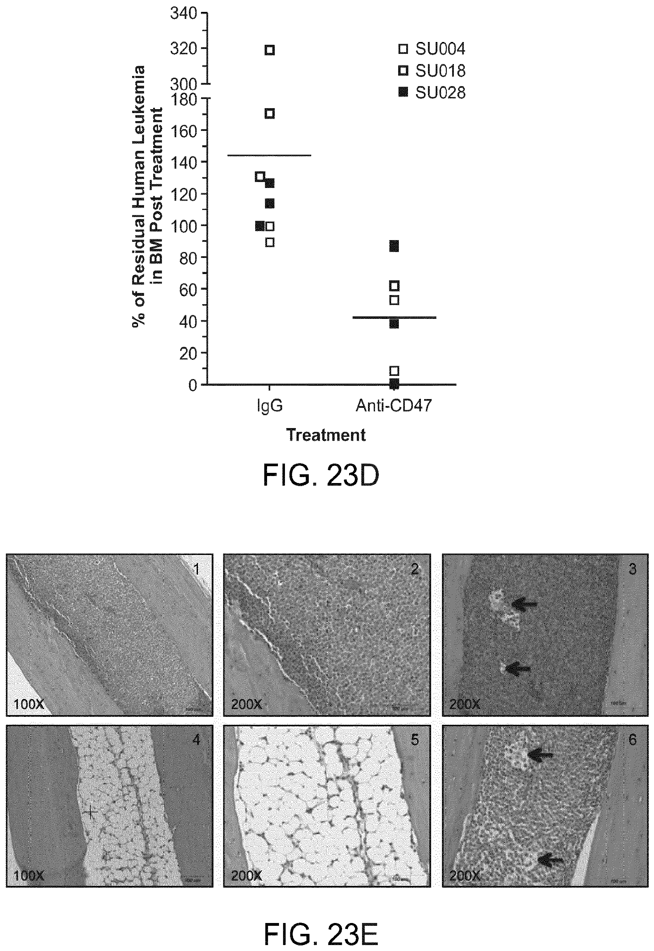

Next, a treatment strategy was utilized in which mice were first engrafted with human AML LSC and then administered daily intraperitoneal injections of 100 micrograms of either mouse IgG or anti-CD47 antibody for 14 days, with leukemic engraftment determined pre- and post-treatment. Analysis of the peripheral blood showed near complete elimination of circulating leukemia in mice treated with anti-CD47 antibody, often after a single dose, with no response in control mice (Figure 23A,B). Similarly, there was a significant reduction in leukemic engraftment in the bone marrow of mice treated with anti-CD47 antibody, while leukemic involvement increased in control IgG-treated mice (Figure 23 C,D). Histologic analysis of the bone marrow identified monomorphic leukemic blasts in control IgG-treated mice (Figure 23E, panels 1,2) and cleared hypocellular areas in anti-CD47 antibody-treated mice (Figure 23E, panels 4,5). In the bone marrow of some anti-CD47 antibody-treated mice that contained residual leukemia, macrophages were detected containing phagocytosed pyknotic cells, capturing the elimination of human leukemia (Figure 23E, panels 3,6).

We report here the identification of higher expression of CD47 on AML LSC compared to their normal counterparts and hypothesize that increased expression of CD47 on human AML contributes to pathogenesis by inhibiting phagocytosis of these cells through the interaction of CD47 with SIRPα. Consistent with this hypothesis, we demonstrate that increased expression of CD47 in human AML is associated with decreased overall survival. We also demonstrate that disruption of the CD47-SIRPα interaction with monoclonal antibodies directed against CD47 preferentially enables phagocytosis of AML LSC by macrophages in vitro, inhibits the engraftment of AML LSC, and eliminates AML in vivo. Together, these results establish the rationale for considering the use of an anti-CD47 monoclonal antibody as a novel therapy for human AML.

The pathogenic influence of CD47 appears mechanistically distinct from the two main complementing classes of mutations in a model proposed for AML pathogenesis. According to this model, class I mutations, which primarily impact proliferation and apoptosis (for example, FLT3 and NRAS), and class II mutations, which primarily impair hematopoietic cell differentiation (for example, CEBPA, MLL, and NPM1), cooperate in leukemogenesis. As demonstrated here, CD47 contributes to pathogenesis via a distinct mechanism, conferring a survival advantage to LSC and progeny blasts through evasion of phagocytosis by the innate immune system. While strategies for the evasion of immune responses have been described for many human tumors, we believe that increased CD47 expression represents the first such immune evasion mechanism with prognostic and therapeutic implications for human AML.

Higher CD47 Expression is a Marker of Leukemia Stem Cells and Prognostic for Overall Survival in AML. AML LSC are enriched in the Lin-CD34+CD38- fraction, which in normal bone marrow contains HSC and MPP. The identification of cell surface molecules that can distinguish between leukemic and normal stem cells is essential for flow cytometry-based assessment of minimal residual disease (MRD) and for the development of prospective separation strategies for use in cellular therapies. Several candidate molecules have recently been identified, including CD123, CD96, CLL-1, and now CD47. CD123 was the first molecule demonstrated to be more highly expressed on AML LSC compared to normal HSC-enriched populations. We previously identified AML LSC-specific expression of CD96 compared to normal HSC, and demonstrated that only CD96+, and not CD96-, leukemia cells were able to engraft in vivo.

CLL-1 was identified as an AML LSC-specific surface molecule expressed on most AML samples and not normal HSC; importantly, the presence of Lin-CD34+CD38-CLL-1+ cells in the marrow of several patients in hematologic remission was predictive of relapse. Here we demonstrate that not only is CD47 more highly expressed on AML LSC compared to normal HSC and MPP, but that this differential expression can be used to separate normal HSC/MPP from LSC. This is the first demonstration of the prospective separation of normal from leukemic stem cells in the same patient sample, and offers the possibility of LSC-depleted autologous HSC transplantation therapies.

We initially identified higher expression of CD47 on AML LSC, but noted that expression in bulk blasts was the same. Because of this, we decided to utilize published gene expression data on bulk AML to investigate the relationship between CD47 expression and clinical outcomes. Consistent with our hypothesis, we found that increased CD47 expression was independently predictive of a worse clinical outcome in AML patients with a normal karyotype, including the subset without the FLT3-ITD mutation, which is the largest subgroup of AML patients. As this analysis was dependent on the relative expression of CD47 mRNA, a quantitative PCR assay for AML prognosis may be based on the level of CD47 expression. Such an assay could be utilized in risk adapted therapeutic decision making, particularly in the large subgroup of AML patients with normal karyotypes who lack the FLT3-ITD mutation.

Targeting of CD47 on AML LSC with Therapeutic Monoclonal Antibodies Cell surface molecules preferentially expressed on AML LSC compared to their normal counterparts are candidates for targeting with therapeutic monoclonal antibodies. Thus far, several molecules have been targeted on AML including CD33, CD44, CD123, and now CD47. CD33 is the target of the monoclonal antibody conjugate gemtuzumab ozogamicin (Mylotarg), which is approved for the treatment of relapsed AML in older patients. Targeting of CD44 with a monoclonal antibody was shown to markedly reduce AML engraftment in mice, with evidence that it acts specifically on LSC to induce differentiation. A monoclonal antibody directed against CD123 was recently reported to have efficacy in reducing AML LSC function in vivo. Here we report that a monoclonal antibody directed against CD47 is able to stimulate phagocytosis of AML LSC in vitro and inhibit engraftment in vivo.

Several lines of evidence suggest that targeting of CD47 with a monoclonal antibody likely acts by disrupting the CD47-SIRPα interaction, thereby preventing a phagocytic inhibitory signal. First, two blocking anti-CD47 antibodies enabled AML LSC phagocytosis, while one non-blocking antibody did not, even though all three bind the cells similarly. Second, in the case of the B6H12.2 antibody used for most of our experiments, the isotype-matched anti-CD45 antibody, which also binds LSC, failed to produce the same effects. In fact, the B6H12.2 antibody is mouse isotype IgG1, which is less effective at engaging mouse Fc receptors than antibodies of isotype IgG2a or IgG2b.

For human clinical therapies, blocking CD47 on AML LSC with humanized monoclonal antibodies promotes LSC phagocytosis through a similar mechanism, as indicated by the human macrophage-mediated in vitro phagocytosis (Figure 8A,C). Higher CD47 expression is detected on AML LSC; however, CD47 is expressed on normal tissues, including bone marrow HSC. We identified a preferential effect of anti-CD47 antibodies in enabling the phagocytosis of AML LSC compared to normal bone marrow CD34+ cells by human macrophages in vitro. In fact, no increased phagocytosis of normal CD34+ cells compared to isotype control was detected, demonstrating that blocking CD47 with monoclonal antibodies is a viable therapeutic strategy for human AML.

The experimental evidence presented here provides the rationale for anti-CD47 monoclonal antibodies as monotherapy for AML. However, such antibodies may be equally, if not more effective as part of a combination strategy. The combination of an anti-CD47 antibody, able to block a strong inhibitory signal for phagocytosis, with a second antibody able to bind a LSC-specific molecule (for example CD96) and engage Fc receptors on phagocytes, thereby delivering a strong positive signal for phagocytosis, may result in a synergistic stimulus for phagocytosis and specific elimination of AML LSC. Furthermore, combinations of monoclonal antibodies to AML LSC that include blocking anti-CD47 and human IgG1 antibodies directed against two other cell surface antigens will be more likely to eliminate leukemia cells with pre-existing epitope variants or antigen loss that are likely to recur in patients treated with a single antibody.

### EXPERIMENTAL PROCEDURES

*Human Samples.* Normal human bone marrow mononuclear cells were purchased from AllCells Inc. (Emeryville, CA). Human acute myeloid leukemia samples (Figure 1A) were obtained from patients at the Stanford University Medical Center with informed consent, according to an IRB-approved protocol (Stanford IRB# 76935 and 6453). Human CD34- positive cells were enriched with magnetic beads (Miltenyi Biotech).

*Flow Cytometry Analysis and Cell Sorting.* A panel of antibodies was used for analysis and sorting of AML LSC (Lin-CD34+CD38-CD90-, where lineage included CD3, CD19, and CD20), HSC (Lin-CD34+CD38-CD90+), and MPP (Lin-CD34+CD38-CD90-CD45RA-) as previously described (Majeti et al., 2007). Analysis of CD47 expression was performed with an anti-human CD47 PE antibody (clone B6H12, BD Biosciences, San Jose CA).

*Genomic DNA Preparation and Analysis of FLT3-ITD by PCR.* Genomic DNA was isolated from cell pellets using the Gentra Puregene Kit according to the manufacturer's protocol (Gentra Systems, Minneapolis, MN). FLT3-ITD status was screened by PCR using primers that generated a wild-type product of 329 bp and ITD products of variable larger sizes.

*Anti-Human CD47 Antibodies.* Monoclonal mouse anti-human CD47 antibodies included: BRIC126, IgG2b (Abcam, Cambridge, MA), 2D3, IgG1 (Ebiosciences. San Diego, CA), and B6H12.2, IgG1. The B6H12.2 hybridoma was obtained from the American Type Culture Collection (Rockville, MD). Antibody was either purified from hybridoma supernatant using protein G affinity chromatography according to standard procedures or obtained from BioXCell (Lebanon, NH).

*Methylcellulose Colony Assay.* Methylcellulose colony formation was assayed by plating sorted cells into a 6-well plate, each well containing 1 ml of complete methylcellulose (Methocult GF+ H4435, Stem Cell Technologies). Plates were incubated for 14 days at 37°C, then scored based on morphology.

*In Vitro Phagocytosis Assays.* Human AML LSC or normal bone marrow CD34+ cells were CFSE-labeled and incubated with either mouse or human macrophages in the presence of 7 µg/ml IgG1 isotype control, anti-CD45 IgG1, or anti-CD47 (clones B6H12.2, BRIC126, or 2D3) antibody for 2 hours. Cells were then analyzed by fluorescence microscopy to determine the phagocytic index (number of cells ingested per 100 macrophages). In some cases, cells were then harvested and stained with either a mouse or human macrophage marker and phagocytosed cells were identified by flow cytometry as macrophage+CFSE+. Statistical analysis using Student's t-test was performed with GraphPad Prism (San Diego, CA).

*In Vivo Pre-Coating Engraftment Assay.* LSC isolated from AML specimens were incubated with 28 ug/mL of IgG1 isotype control, anti-CD45 IgG1, or anti-CD47 IgG1 (B6H12.2) antibody at 4°C for 30 minutes. A small aliquot of cells was then stained with donkey anti-mouse PE secondary antibody (Ebioscience) and analyzed by flow cytometry to assess coating. Approximately 10⁵ coated LSC were then transplanted into each irradiated newborn NOD.Cg-Prkdcscidll2rgtm1Wjl/SzJ (NOG) mouse. Mice were sacrificed 13 weeks post-transplantation and bone marrow was analyzed for human leukemia engraftment (hCD45+hCD33+) by flow cytometry (Majeti et al., 2007 Cell Stem Cell 1, 635-645). The presence of human leukemia was confirmed by Wright-Giemsa staining of hCD45+ cells and FLT3-ITD PCR. Statistical analysis using Student's t-test was performed with GraphPad Prism (San Diego, CA).

*In Vivo Antibody Treatment of AML Engrafted Mice.* 1-25x10⁵ FACS-purified LSC were transplanted into NOG pups. Eight to twelve weeks later, human AML engraftment (hCD45+CD33+ cells) was assessed in the peripheral blood and bone marrow by tail bleed and aspiration of the femur, respectively. Engrafted mice were then treated with daily intraperitoneal injections of 100 micrograms of anti-CD47 antibody or IgG control for 14 days. On day 15 mice were sacrificed and the peripheral blood and bone marrow were analyzed for AML.

*AML Patients, Microarray Gene Expression Data, and Statistical Analysis.* Gene expression and clinical data were analyzed for three previously described cohorts of adult AML patients: (1) a training dataset of 285 patients with diverse cytogenetic and molecular abnormalities described by Valk et al., (2) a test dataset of 242 patients with normal karyotypes described by Metzeler et al., and (3) a validation dataset of 137 patients with normal karyotypes described by Bullinger et al. The clinical end points analyzed included overall and event-free survival, with events defined as the interval between study enrollment and removal from the study owing to a lack of complete remission, relapse, or death from any cause, with data censored for patients who did not have an event at the last follow-up visit.

*FLT3-ITD PCR.* All reactions were performed in a volume of 50 µl containing 5 µl of 10x PCR buffer (50mM KCL/10nM Tris/2mM MgCl2/0.01% gelatin), 1 µl of 10mM dNTPs, 2 units of Taq polymerase (Invitrogen), 1 ul of 10µM forward primer 11F (5'-GCAATTTAGGTATGAAAGCCAGC-3') and reverse primer 12R (5'-CTTTCAGCATTTTGACGGCAACC-3'), and 10-50 ng of genomic DNA. PCR conditions for amplification of the FLT3 gene were 40 cycles of denaturation (30 sec at 95°C) annealing (30 sec at 62°C), and extension (30 sec at 72°C).

*Preparation of Mouse and Human Macrophages.* BALB/c mouse bone marrow mononuclear cells were harvested and grown in IMDM containing 10% FBS supplemented with 10 ng/mL recombinant murine macrophage colony stimulating factor (M-CSF, Peprotech, Rocky Hill, NJ) for 7-10 days to allow terminal differentiation of monocytes to macrophages. Human peripheral blood mononuclear cells were prepared from discarded normal blood from the Stanford University Medical Center. Monocytes were isolated by adhering mononuclear cells to culture plates for one hour at 37°C, after which non-adherent cells were removed by washing. The remaining cells were >95% CD14 and CD11b positive. Adherent cells were then incubated in IMDM plus 10% human serum (Valley Biomedical, Winchester, VA) for 7-10 days to allow terminal differentiation of monocytes to macrophages.

*In vitro phagocytosis assay.* BMDM or peripheral blood macrophages were harvested by incubation in trypsin/EDTA (Gibco/Invitrogen) for 5 minutes followed by gentle scraping. 5 x 10⁴ macrophages were plated in each well of a 24-well tissue culture plate in 10% IMDM containing 10% FBS. After 24 hours, media was replaced with serum-free IMDM and cells were cultured an additional 2 hours. LSC were fluorescently labeled with CFSE according to the manufacturer's protocol (Invitrogen). 2 x 10⁴ CFSE-labeled LSC were added to the macrophage-containing wells along with 7 µg/mL of IgG1 isotype (Ebiosciences), anti-CD45 (clone HI30, Ebiosciences), or anti-CD47 antibody, and incubated for 2 hours. Wells were then washed 3 times with IMDM and examined under an Eclipse T5100 immunofluorescent microscope (Nikon) using an enhanced green fluorescent protein filter able to detected CFSE fluorescence. The number of CFSE positive cells within macrophages was counted and the phagocytic index was determined as the number of ingested cells per 100 macrophages. At least 200 macrophages were counted per well. Flourescent and brightfield images were taken separately and merged with Image Pro Plus (Media Cybernetics, Bethesda, MD). In Figure 22A,B, the three left images are presented at 200x magnification, with the anti-CD47 right image at 400x magnification. For flow cytometry analysis of phagocytosis, the cells were then harvested from each well using trypsin/EDTA. Cell suspensions were then stained with a mouse macrophage antibody anti-mouse F4/80-PECy7 (Ebiosciences) or anti-human CD14-PECy7 (Ebiosciences) and analyzed on a FACSAria. Phagocytosed LSC were defined as either CFSE+F4/80+ or CFSE+CD14+ cells when incubated with murine or human macrophages, respectively.

*Microarray Gene Expression Data.* Panel A of Supplemental Figure 22 describes the main microarray datasets analyzed herein, including the training, test, and validation cohorts. Training Set: Gene expression data, cytogenetics data, and molecular data for the 285 and 465 patients with AML profiled with Affymetrix HG-U133A and HG-U133 Plus 2.0 microarrays by Valk et al. and Jongen-Lavrencic et al. respectively, were obtained from the Gene Expression Omnibus using the corresponding accession numbers (GSE1159 and GSE6891). Outcome data were only available for the former dataset, and the corresponding clinical information were kindly provided by the authors. This cohort is presented as the "training" dataset. The latter dataset was used to confirm univariate associations with karyotype and molecular mutations described in the former. However, these two datasets overlapped in that 247 of the 285 patients in the first study were included in the second, and were accordingly excluded in validation of the association of FLT3-ITD with CD47 expression in the 2nd dataset. Using NetAffx4, RefSeq5, and the UCSC Genome Browser6, we identified 211075_s_at and 213857_s_at as Affymetrix probe sets on the U133 Plus 2.0 microarray mapping exclusively to constitutively transcribed exons of CD47. The geometric mean of the base-2 logarithms of these two probe sets was employed in estimating the mRNA expression level for CD47, and corresponding statistical measures for associations with FAB classification, karyotype, and molecular mutations. Because the data provided by Valk et al. as GSE1159 were Affymetrix intensity measurements, we converted these intensities to normalized base-2 logarithms of ratios to allow comparison to the corresponding measurements from cDNA microarrays using a conventional scheme. Specifically, we first (1) normalized raw data using CEL files from all 291 microarrays within this dataset using gcRMA8, then (2) generated ratios by dividing the intensity measurement for each gene on a given array by the average intensity of the gene across all arrays, (3) log-transformed (base 2) the resulting ratios, and (4) median centered the expression data across arrays then across genes. For the assessment of the prognostic value of CD47, we employed the probe set 213857_s_at from the Affymetrix HG-U133A and HG-U133 Plus 2.0 microarrays, given its similar expression distribution (Supplemental Figure 3B), and considering its position within the mRNA transcript as compared with cDNA clones on the Stanford cDNA microarrays as annotated within the NetAffx resource.

Test Set: Gene expression and clinical data for the 242 adult patients with NKAML profiled with Affymetrix HG-U133A and HG-U133 Plus 2.0 microarrays by Metzeler et al. were obtained from the Gene Expression Omnibus using the corresponding accession numbers (GSE12417). Since raw data were not available for this dataset, for purposes of assessing the prognostic value of CD47, we employed the normalized datasets provided by the authors (base 2 logarithms) and assessed expression of CD47 using the probe set 213857_s_at on the corresponding microarrays.

Validation Set: Gene expression data for the 137 patients with normal karyotype AML profiled with cDNA microarrays by Bullinger et al. were obtained from the Stanford Microarray Database10. The corresponding clinical information including outcome data and FLT3 mutation status were kindly provided by the authors. Using the original annotations of microarray features as well as SOURCE11, RefSeq5, and the UCSC Genome Browser6, we identified IMAGE:811819 as a sequence verified cDNA clone mapping to the constitutively transcribed 3' terminal exon of CD47 on the corresponding cDNA microarrays.

Details of Treatment: AML patients described by Valk et al. (training set), were treated according to several protocols of the Dutch-Belgian Hematology-Oncology Cooperative group. The majority (90%) of the NK-AML patients described by Metzeler et al. (test set) were treated per protocol AMLCG-1999 of the German AML Cooperative Group, with all patients receiving intensive double-induction and consolidation chemotherapy. All 137 NK-AML patients described by Bullinger et al. (validation set) received standard-of-care intensified treatment regimens (protocol AML HD98A), which included 2 courses of induction therapy with idarubicin, cytarabine, and etoposide, one consolidation cycle of high-dose cytarabine and mitoxantrone (HAM), followed by random assignment to a late consolidation cycle of HAM versus autologous hematopoietic cell transplantation in case no HLA identical family donor was available for allogeneic hematopoietic cell transplantation.

Statistical Analysis. We used two tailed t-tests and analysis of variance for the estimation of significant differences in CD47 expression level across subgroups of AML based on morphologic, cytogenetic, and molecular categorizations. Associations between the high and low CD47 groups and baseline clinical, demographic, and molecular features were analyzed using Fisher's exact and Mann-Whitney rank sum tests for categorical and continuous variables, respectively. Two-sided p-values of less than 0.05 were considered to indicate statistical significance.

The prognostic value of CD47 expression was measured through comparison of the event-free and overall survival of patients with estimation of survival curves by the Kaplan-Meier product limit method and the log-rank test. Within this analysis, we first derived a binary classification of AML patients into High CD47 and Low CD47 expression groups by comparing the expression of CD47 (as measured by 213857_s_at within GSE1159) relative to an optimal threshold. This threshold was determined using X-Tile16, a method which we employed to maximize the chi-square statistic between the two groups for the expected versus observed number of deaths. This stratification segregates the 261 AML patients with available outcome data into two unequally sized groups, with 72% of patients with lowest expression considered CD47 low, and 28% with highest expression considered CD47 high. These two groups have different overall survival with a hazard ratio of 1.42 for the CD47 high group, and a corresponding uncorrected p-value of 0.033, which requires cross-validation to avoid the risk of overfitting.

Accordingly, we assessed the validity and robustness of risk stratification using CD47 expression by applying this optimal threshold to an independent test cohort of 242 NK-AML patients described by Metzeler et al. Notably, despite the presence of other variables potentially confounding associations with survival (including more advanced age, and differing therapies), derivation of an optimal cutpoint using the 242 NK-AML patients within the test dataset yielded a similar stratification, with 74% of patients with lowest expression considered CD47 low, and 26% with highest expression considered CD47 high.

Next, we assessed the validity of this stratification in a cross-validation cohort of 137 uniformly treated NK-AML patients described by Bullinger et al. Within this validation dataset, we could similarly define two groups of similar size (i.e., 72% and 28% with lowest and highest CD47 levels, respectively), and these two groups had significantly different outcomes when assessed for overall survival (Figure 22B, p=0.002, hazard ratio 2.02, 95% CI 1.37 to 4.03), and event-free survival (Figure 223A, p=0.004, hazard ratio 1.94, 95% CI 1.30 to 3.77). Of the 137 patients, 5 did not have reliable measurements for CD47 when using the data selection and normalization criteria described by the authors.

To determine the robustness of this association, we also examined the predictive value of CD47 expression when the validation cohort was divided into low and high CD47 expression groups based on expression relative to the median, or as a continuous variable. As above, higher CD47 expression was associated with worse event-free and overall survival. Of the 137 patients studied, a subset of 123 patients had available survival data, CD47 expression data, and FLT3-ITD status reported. Within this cohort, we assessed the relationship of CD47 expression level as a continuous variable with outcome using univariate Cox proportional-hazards analysis, with event-free survival or overall survival as the dependent variable. We used multivariate Cox-proportional hazards analysis with event-free survival or overall survival as the dependent variable and FLT3-ITD status, age, and continuous expression level of CD47 as directly assessed independent variables.

Associations of CD47 with other covariates (eg, NPM1, CEBPA) were limited by sample size and missing data for covariates. The Wald test was used to assess the significance of each covariate in multivariate analyses. Univariate and multivariate proportional-hazards analyses were done using the coxph function in the R statistical package.

### Example 5

### CD47 is a Prognostic Factor and Therapeutic Antibody Target on Solid Tumor Cancer Stem Cells

We have found that increased CD47 expression is associated with worse clinical outcomes in diffuse large B-cell lymphoma (DLBCL) and ovarian carcinoma (Figure 24). Additionally, we have now found that anti-CD47 antibodies enable the phagocytosis of cancer stem cells from bladder cancer, ovarian carcinoma, and medulloblastoma *in vitro* with human macrophages (Figure 25).

## Claims

1. A composition comprising a monoclonal antibody that specifically binds CD47 on the surface of cancer cells and inhibits its interaction with SIRPα receptor on the surface of phagocytic cells, for use in treating an ovarian tumor by increasing phagocytosis of cancer cells of the ovarian tumor in a human subject, wherein said antibody up-regulates phagocytosis of said cancer cells by inhibiting the binding of CD47 on the surface of the cancer cells to SIRPα receptor on the surface of phagocytic cells.

2. The composition for use of claim 1, wherein the antibody is a humanized antibody.

## Patentansprüche

1. Zusammensetzung, umfassend einen monoklonalen Antikörper, der CD47 auf der Oberfläche von Krebszellen spezifisch bindet und seine Wechselwirkung mit SIRPα-Rezeptor auf der Oberfläche von Phagozyten hemmt, zur Verwendung bei der Behandlung eines Eierstocktumors durch Erhöhen der Phagozytose von Krebszellen des Eierstocktumors bei einem menschlichen Individuum, wobei der Antikörper die Phagozytose der Krebszellen heraufreguliert, indem er die Bindung von CD47 auf der Oberfläche der Krebszellen an SIRPα-Rezeptor auf der Oberfläche von Phagozyten hemmt.

2. Zusammensetzung zur Verwendung nach Anspruch 1, wobei es sich bei dem Antikörper um einen humanisierten Antikörper handelt.

## Revendications

1. Composition comprenant un anticorps monoclonal qui se lie spécifiquement à CD47 sur la surface de cellules cancéreuses et inhibe son interaction avec le récepteur SIRPα à la surface de cellules phagocytaires, pour utilisation dans le traitement d'une tumeur ovarienne en augmentant la phagocytose de cellules cancéreuses de la tumeur ovarienne chez un sujet humain, dans laquelle ledit anticorps régule à la hausse la phagocytose desdites cellules cancéreuses en inhibant la liaison de CD47 sur la surface des cellules cancéreuses au récepteur SIRPα sur la surface de cellules phagocytaires.

2. Composition pour utilisation selon la revendication 1, dans laquelle l'anticorps est un anticorps humanisé.
